# EUROPEAN PATENT APPLICATION

(11) **EP 0 826 686 A2**
(43) Date of publication of application: **04.03.1998**
(21) Application number: 97115200.4
(22) Date of filing: 02.09.1997
(51) Int. Cl.: C07D 471/16, A61K 31/415, A61K 31/495

(54) **Tricyclic compounds, their production and use**

(30) Priority: 02.09.1996 JP 231855/96; 01.11.1996 JP 292059/96
(71) Applicant: TAKEDA CHEMICAL INDUSTRIES, LTD., Chuo-ku, Osaka 541 (JP)
(72) Inventor: Kawamoto, Tetsuji, Osaka 572 (JP); Shibouta, Yumiko, Osaka 564 (JP); Takatani,Muneo, Kyoto 616 (JP); Noda, Masakuni, Osaka 565 (JP)
(74) Representative: KUHNEN, WACKER & PARTNER

(57) **Abstract**

A pharmaceutical composition for treating or preventing proliferative glomerulonephritis, which comprises a compound of the formula: 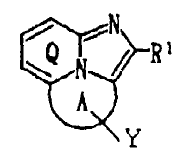
wherein ring A is a nitrogen-containing heterocyclic ring, having two nitrogen atoms as the hetero-atoms, which may be substituted with oxo or thioxo; ring Q may be substituted; Y is an optionally substituted hydrocarbon group, an optionally substituted hydroxy group or an optionally substituted mercapto group; and
R¹ is a hydrogen atom, a halogen atom, an optionally substituted hydrocarbon group or an acyl group, or a salt thereof, and a novel compound of the formula: 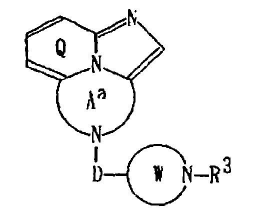
wherein ring A^{a} may have one oxo group; D is a bond or an optionally substituted divalent hydrocarbon group;
ring W is an optionally substituted nitrogen-containing heterocyclic ring; ring Q may be substituted; and R³ is an electron-withdrawing group, or a salt thereof, which have excellent PDGF-inhibiting activities, activities of ameliorating renal diseases and activities of lowering lipid level and are less toxic and are useful as a preventive and treating agent for cardisvascular diseases.

## Description

The present invention relates to therapeutic (treating) and prophylactic (preventive) agents of proliferative glomerulonephritis, especially mesangial proliferative glomerulonephritis, a novel tricyclic compound which is useful as a medicine having an excellent activity of inhibiting platelet-derived growth factor (PDGF), ameliorating activity of renal diseases and lowering the cholesterol level, a process for producing the compound, and a pharmaceutical composition comprising the compound.

### Background Art

While it has been considered that platelet-derived growth factor (PDGF) act on the process of the healing of cell proliferation in the region of injury, PDGF causes vascular hypertrophy by cell proliferation and is one of the cause of, for example, arteriosclerosis, diabetes, glomerulosclerosis (chronic renal failure) and hypertension.

As one of the common phenomena observed in these diseases mentioned above, the enhanced expression of platelet-derived growth factor (PDGF) or PDGF receptors (mRNA) has been reported by the following 1) to 11).

More specifically stating; 1) In spontaneously hypertensive rats (SHR: Spontaneous Hypertensive Rat) and renal hypertensive animals, expression of PDGF or PDGF receptors is enhanced, or the tyrosine kinase activity associated with PDGF receptors is enhanced (R. Sarzani et al., Hypertension, 18, III 93/1991; P. Pauletto et al., 15th International Meeting of Hypertension, Melbourne, Abstract 1197/1994; M. D. Sauro and B. Thomas, Life Sci., 53, PL371/1993). 2) In the essential hypertensive patients with diabetes, it has been observed that blood concentration of PDGF in blood is higher than normal subjects. (P. Bolli et al., 15th International Meeting of Hypertension, Melbourne, Abstract 767/1994). 3) In human atherosclerotic plaques, expression of PDGF mRNA is enhanced (T. Barrett and P. Benditt, Proc. Natl. Acad. Sci. USA, 85, 2810/1988; J. N. Wilcox et al., J. Clin. Invest., 82, 1134/1988), in the vascular smooth muscle cells of diabetic rats with arteriosclerosis, expression of PDGF receptors is enhanced (T. Kanzaki, Y. Saitoh, Gendai Iryo (Modern Therapeutics), 23, 2614/1991). 4) In the blood vessels of balloon injured animals and humans after PTCA, the expression of PDGF or PDGF receptor is enhanced (M. W. Majesky et al., J. Cell Biol., 111, 2149/1990; M. Ueda et al., Circulation, 86 (Suppl.), 1/1992). 5) In renal mesangial cells of 5/6 nephrectomized rats, a model of focal glomerulosclerosis (a kind of chronic renal failure, expression of PDGF is enhanced (J. Floege et al., Kidney Int., 41, 297/1992). 6) In mesangium proliferative nephritis (IgA nephropathy) and a model of nephritis in rats, enhancement of PDGF in mesangial cells is observed (R. J. Johnson et al., J. Am. Soc. Nephrol., 4, 119/1993; H. E. Abboud et al., Kidney Int., 43, 252/1993). 7) In patients with mesangium proliferative nephritis and its model in rats, PDGF receptors are activated (L. Gesaldo et al., J. Clin. Invest., 94, 50/1994; H, Iida et al., Proc. Natl. Acad. Sci., 80, 6560/1991). 8) It is demonstrated that PDGF proliferates vascular smooth muscle cells or renal glomerular mesangial cells in vitro experiments (R. Ross et al., Cell, 46, 155/1986); J. Floege et al., Clin. Exp. Immunol., 86, 334/1991) and in vivo experiments (A. Jawien et al., J. Clin. Invest., 89, 507/1992; Y. Isaka et al., J. Clin. Invest., 92 2597/1993; J. Floege et al., J. Clin. Invest., 92, 2952/1993). 9) It is also reported that the action of cytokine TGF-β (transforming growth factor β) is via the action of PDGF expressed by TGF-β (E. G. Battegay et al., Cell, 63, 515/1990). 10) Recently there have been a number of reports that hypertensive vascular hypertrophy and cardiac hypertrophy due to congestive heart failure are suppressed by administration of ACE inhibitors or angiotensin antagonistic agent. It is considered that, also in the angiotensin-mediated vascular hypertrophy and cardiac hypertrophy, PDGF plays a role (A. J. Naftilan et al., J. Clin. Invest., 83, 1419/1989; G. H. Gibbons et al., J. Clin. Invest., 90, 456/1992). 11) It has been known that, in respect of the proliferation of vascular smooth muscle cells or renal mesangial cells, LDL-cholesterol and PDGF mutually cooperate to enhance the proliferation, which has been considered as one of factors causing arteriosclerosis. Therefore, drugs capable of specifically inhibiting the action of PDGF are expected to be useful therapeutic agents of various circulatory disturbances including arteriosclerosis.

Further, various growth factors and cytokines have mitogenic activity for proliferation of mesangial cells in mesangial proliferative glomerulonephritis (Floege et al., Kidney Int. 43: S47-S54, 1993), and among them, platelet-derived growth factor (PDGF) expressing potent proliferative action in mesangial cells has been known to play the most important role (Floege et al., Clin. Exp. Immunol. 86: 334-341, 1991). Actually, it has been reported that PDGF receptors act strongly on the proliferation of mesangial cells in patients of mesangial proliferative glomerulonephritis (Gesualdo et al., J. Clin. Invest. 94: 50-58, 1994). And, in a rat model of mesangial proliferative glomerulonephritis, an increase of the expression of PDGF and its receptors, corresponding to the time of mesangial cell proliferation, is observed in glomerular mesangial region (Iida et al., Proc. Natl. Acad. Sci. USA 88: 6560-6564, 1991). It has been reported that mesangial cell proliferation is reduced by inhibiting the action of PDGF with administration of neutralizing anti-PDGF to the rat model (Johnson et al., Exp. Med. 175: 1413-1416, 1992). Furthermore, it has been reported that, by introducing PDGF gene into rat kidney, mesangial proliferative glomerulonephritis is induced (Isaka et al., J. Clin. Invest. 92: 2597-2601, 1993). On the other hand, in WO 96/02542, there is disclosed that, as the tricyclic cyclazine compounds which have PDGF-inhibiting activity, antihypertensive activity, activity of ameliorating renal diseases and activity of lowering lipid level, a compound of the formula: 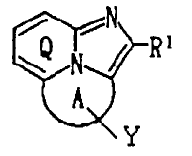 wherein
ring A is a nitrogen-containing heterocyclic ring, having two nitrogen atoms as the hetero-atoms, which is optionally substituted with oxo or thioxo; ring Q may be substituted;
Y is an optionally substituted hydrocarbon group, an optionally substituted hydroxy group or an optionally substituted mercapto group, excluding methyl group as Y; and
R¹ is a hydrogen atom, a halogen atom, an optionally substituted hydrocarbon group or an acyl group, or a salt thereof.

### Detailed Description of Invention

Circumstances being such as above, the development of low toxicity and safety therapeutic agents based on inhibiting the action of PDGF, which is clinically useful for therapy of cardiovascular diseases and excellent properties as new drugs, has been desired.

Further, new treating or preventing drugs, which are useful for therapy of proliferative glomerulonephritis such as mesangium proliferative glomerulonephritis, has been desired.

The present inventors have made extensive and intensive studies, as a result, for the first time, have found that, a compound of the formula: (I')(hereinafter called the compound (I')) 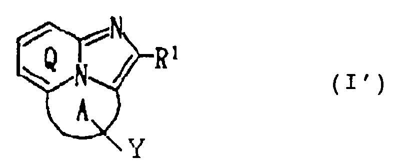
wherein ring A is a nitrogen-containing heterocyclic ring, having two nitrogen atoms as the hetero-atoms, which is optionally substituted with oxo or thioxo; ring Q may be substituted;
Y is an optionally substituted hydrocarbon group, an optionally substituted hydroxy group or an optionally substituted mercapto group; and
R¹ is a hydrogen atom, a halogen atom, an optionally substituted hydrocarbon group or an acyl group, or a salt thereof, especially a compound of the formula (I) (hereinafter called the compound (I)): 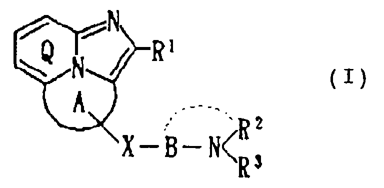
wherein ring A, ring Q and R¹ are as defined above;
B is an optionally substituted divalent hydrocarbon;
X is a bond, an oxygen atom or a sulfur atom;
R² is a hydrogen atom or an optionally substituted hydrocarbon group or, R² and B may form a ring together with the adjacent nitrogen atom; and
R³ is an electron-withdrawing group, or a salt thereof, has unexpectedly effectiveness, which is clinically useful for treating or preventing proliferative glomerulonephritis such as mesangium proliferative glomerulonephritis. Among the compound (I'), the present invention relates to a novel compound represented by the formula (A) (hereinafter called the compound (A)): 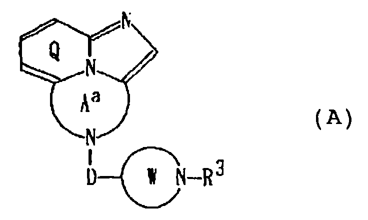
wherein ring A^{a} may have one oxo group;
D is a bond or an optionally substituted divalent hydrocarbon group;
ring W is an optionally substituted nitrogen-containing heterocyclic ring;
ring Q may be substituted; and
R³ is an electron-withdrawing group, or a salt thereof, whose characteristic feature of the chemical structure lies in the tricyclic basic structure, which does not have oxo group at all or has one oxo group, and does not have substituents on the imidazole ring of the basic structure, and further include hetero cyclic ring containing nitrogen atom in the side chain. They found that this compound has, based on the characteristic chemical structure, unexpectedly, excellent PDGF-inhibiting action, action of ameliorating nephropathy and action of lowering cholesterol level, etc. and excellent properties such as very low toxicity as drugs which is clinically useful. The present inventors have further developed studies to accomplish the present invention.

More specifically, the present invention relates to
(1) a pharmaceutical composition for treating or preventing proliferative glomerulonephritis, which comprises a compound (I') or a salt thereof, in admixture with a pharmaceutically acceptable carrier, excipient or diluent,
(2) a pharmaceutical composition as described in (1) above, wherein Y is a hydrocarbon group, a hydroxy group or a mercapto group, each of which optionally has a substituent comprising at least one nitrogen atom,
(3) a pharmaceutical composition as described in (1) above, wherein Y is a hydrocarbon group, a hydroxy group or a mercapto group, each of which optionally has a substituent comprising at least one electron-withdrawing group,
(4) a pharmaceutical composition as defined in (1) above, wherein Y is a hydrocarbon group, a hydroxy group or a mercapto group, each of which optionally has a substituent comprising an amino group which is substituted with at least one electron-withdrawing group,
(5) a pharmaceutical composition as defined in (1) above, wherein Y is group of the formula: 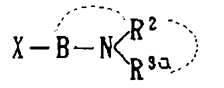
   wherein B is an optionally substituted divalent hydrocarbon group;
   X is a bond, an oxygen atom or a sulfur atom;
   R² is a hydrogen atom or an optionally substituted hydrocarbon group, or R² and B may from a ring together with the adjacent nitrogen atom; and
   R^{3a} is an electron-withdrawing group; or
   R² and R^{3a} may form a ring together with the adjacent nitrogen atom, or a salt thereof,
(6) a pharmaceutical composition as described in (1) above, which comprises a compound (I) or a salt thereof,
(7) a pharmaceutical composition as described in (1) above, wherein the nitrogen atom-containing heterocycleic ring is a 5- or 6-membered ring,
(8) a pharmaceutical composition as described in (1) above, wherein the ring Q may be substituted with 1 to 3 substituents selected from the group consisting of (i) halogen atom, (ii) a C₁₋₄ alkyl group, (iii) a C₁₋₄ alkoxy group, (iv) a C₁₋₄ alkylthio group, (v) a hydroxy group, (vi) a carboxyl group, (vii) a cyano group, (viii) a nitro group, (ix) a amino group, (x) a mono- or di- C₁₋₄ alkyl amino group, (xi) a formyl group, (xii) a mercapto group, (xiii) a C₁₋₄ alkyl-carbonyl group, (xiv) a C₁₋₄ alkoxy-carbonyl group, (xv) a sulfonyl group, (xvi) a C₁₋₄ alkyl sulfonyl group, (xvii) a carbamoyl group, (xviii) a mono- or di- C₁₋₄ alkyl-carbamoyl group and (xiv) thiocarbamoyl group,
(9) a pharmaceutical composition as described in (1) above, wherein the ring Q is unsubstituted,
(10) a pharmaceutical composition as described in (1) above, wherein R¹ is a hydrogen atom, an optionally substituted alkyl group, an optionally substituted alkenyl group, an optionally substituted aralkyl group, an optionally substituted aryl group, an alkoxy carbonyl group, an alkyl carbamoyl group or an alkanoyl group,
(11) a pharmaceutical composition as described in (1) above, wherein R¹ is a hydrogen atom, a C₁₋₆ alkyl group or a phenyl group,
(12) a pharmaceutical composition as described in (5) above, wherein R² is a hydrogen atom, an optionally substituted alkyl group or an optionally substituted alkenyl group,
(13) a pharmaceutical composition as described in (3) above, wherein the electron-withdrawing group is (i) -SO₂R⁴ (R⁴ is an optionally substituted hydrocarbon group), (ii) -CO-R⁵ (R⁵ is a hydrogen atom or an optionally substituted hydrocarbon group), (iii) -COOR⁶ (R⁶ is an optionally substituted hydrocarbon group) (iv) -CON(R⁷)R⁸ (wherein R⁷ and R⁸ respectively are a hydrogen atom or an optionally substituted hydrocarbon group, or, R⁷ and R⁸ form a ring together with the adjacent nitrogen atom), (v) a nitro group or (vi) a cyano group,
(14) a pharmaceutical composition as described in (5) above, wherein B is a C₂₋₁₀ alkylene group,
(15) a pharmaceutical composition as described in (5) above, wherein B is a group of the formula: 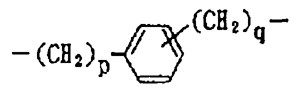 wherein p and q respectively are independently an integer of 0 to 5,
(16) a pharmaceutical composition as described in (5) above, wherein B is a C₃₋₈ alkylene group,
(17) a pharmaceutical composition as described in (6) above, which is one of the formula: 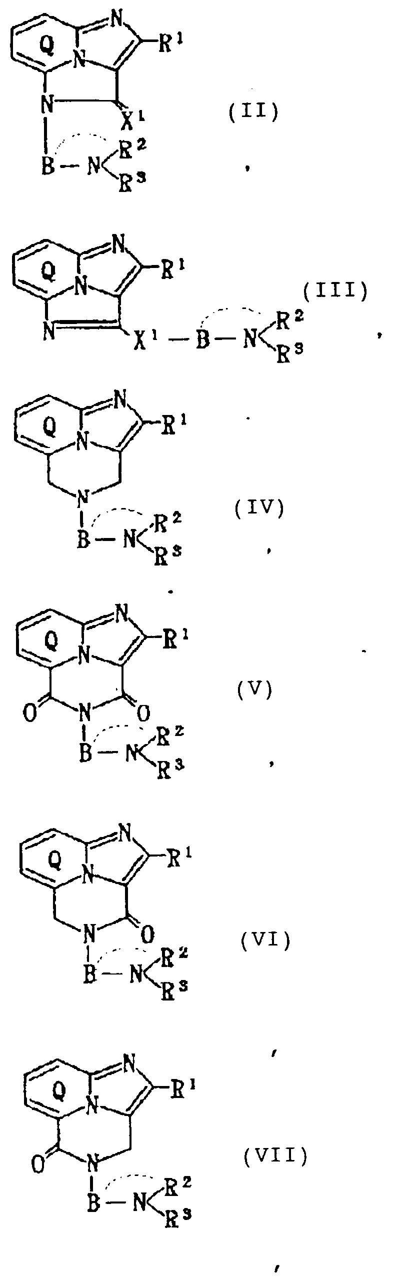 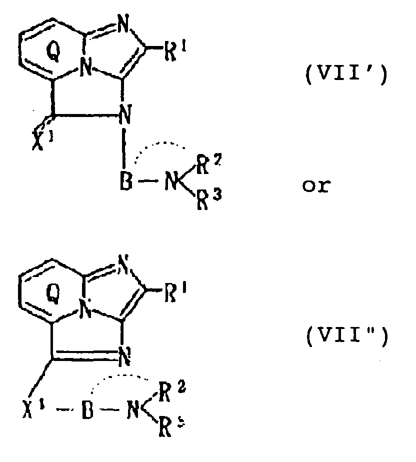 wherein X¹ is an oxygen atom or a sulfur atom, and the other symbols are of the same meanings as defined above or a salt thereof,
(18) a pharmaceutical composition as described in (6) above, which is the compound (II) or (VI) or a salt thereof,
(19) a pharmaceutical composition as described in (17) above, wherein the ring Q is unsubstituted,
(20) a pharmaceutical composition as described in (17) above, wherein R¹ is a hydrogen atom, an optionally substituted alkyl group or an optionally substituted alkenyl group,
(21) a pharmaceutical composition as described in (17) above, wherein R¹ is a hydrogen atom or a C₁₋₆ alkyl group,
(22) a pharmaceutical composition as described in (17) above, wherein R² is a hydrogen atom or C₁₋₆ alkyl group,
(23) a pharmaceutical composition as described in (17) above, wherein R² is a hydrogen atom,
(24) a pharmaceutical composition as described in (17) above, wherein X¹ is an oxygen atom,
(25) a pharmaceutical composition as described in (17) above, wherein X¹ is a sulfur atom,
(26) a pharmaceutical composition as described in (17) above, wherein B is a C₂₋₁₀ alkylene group,
(27) a pharmaceutical composition as described in (17) above, wherein B is a C₃₋₈ alkylene group,
(28) a pharmaceutical composition as described in (17) above, wherein the electron-withdrawing group represented by R³ is -SO₂R^{4a} (R^{4a} is an optionally substituted alkyl group, an optionally substituted alkenyl group, an optionally substituted aralkyl group or an optionally substituted aryl group),
(29) a pharmaceutical compoisition as described in (28) above, wherein R^{4a} is a halogeno-C₁₋₆ alkyl group,
(30) a pharmaceutical composition as described in (1) above, which comprises 4,5-dihydro-4-[4-(trifluoromethanesulfonamido)butan-1-yl]-3H-1,4,8b-triazaacenaphthylen-3-one or a salt thereof, 1,2-dihydro-3-methyl-1-[5-(trifluoromethanesulfonamido)pentan-1-yl]-1,4,7b-triazacyclopent[cd]inden-2-one or a salt thereof or 1,2-dihydro-3-methyl-1-[2-(1-trifluoromethanesulfonylpiperidin-4-yl)ethan-1-yl]-1,4,7b-triazacyclopent[cd]inden-2-one or a salt thereof,
(31) a pharmaceutical composition as described in (1) above, wherein the proliferative glomerulonephritis is mesangium proliferative glomerulonephritis,
(32) a compound (A) or a salt thereof,
(33) a compound as defined in (32) above, wherein ring A^{a} is a 5- or 6-membered nitrogen-containing heterocyclic ring which may have one oxo group,
(34) a compound as defined in (32) above, wherein 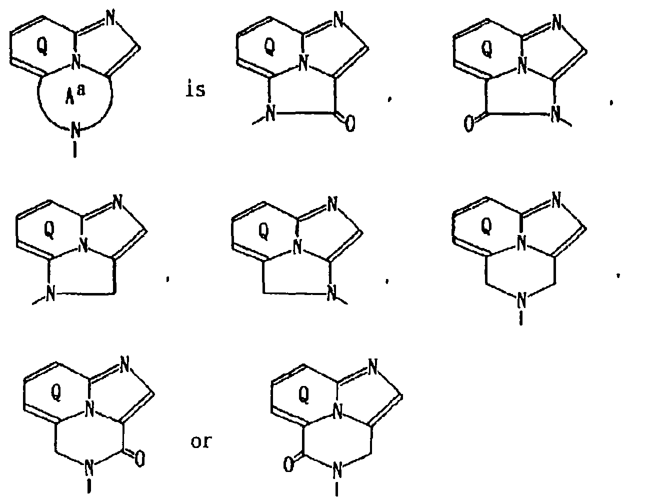 wherein ring Q is of the same meaning as defined above,
(35) a compound as defined in (32) above, wherein D is an optionally substituted divalent hydrocarbon,
(36) a compound as defined in (32) above, wherein ring W is an optionally substituted 5- or 6-membered nitrogen-containing heterocyclic ring,
(37) a compound as defined in (32) above, wherein R³ is -SO₂R⁴ (R⁴ is an optionally substituted hydrocarbon group), -COR⁵ (R⁵ is a hydrogen atom or an optionally substituted hydrocarbon group), -COOR⁶ (R⁶ is an optionally substituted hydrocarbon group) or -CON(R⁷)R⁸ (R⁷ and R⁸ respectively are a hydrogen atom or an optionally substituted hydrocarbon group, or R⁷ and R⁸ form a ring together with the adjacent nitrogen atom),
(38) a compound as defined in (32) above, wherein ring Q is unsubstituted,
(39) a compound as defined in (32) above, wherein ring W is 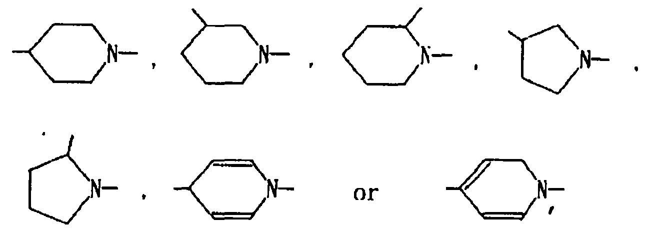
(40) a compound as defined in (32) above, wherein D is C₁₋₆ alkylene,
(41) a compound as defined in (32) above, which is a compound of the formula: 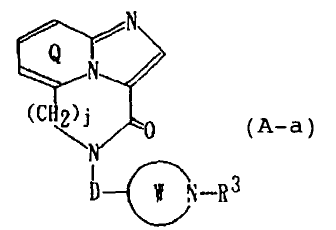 wherein j is 0 or 1, and the other symbols are of the same meanings as defined above (hereinafter called the compound (A-a)), or a salt thereof,
(42) a compound as defined in (41) above, wherein R³ is -SO₂R⁴ (R⁴ is an optionally substituted hydrocarbon group), -COR⁵ (R⁵ is a hydrogen atom or an optionally substituted hydrocarbon group) or -COOR⁶ (R⁶ is an optionally substituted hydrocarbon group),
(43) a compound as defined (42) above, wherein R⁴, R⁵ and R⁶ respectively are an optionally halogenated hydrocarbon group,
(44) a compound as defined in (41) above, wherein D is C₁₋₆ alkylene,
(45) a compound as defined in (41) above, wherein D is ethylene,
(46) a compound as defined in (41) above, wherein ring W is 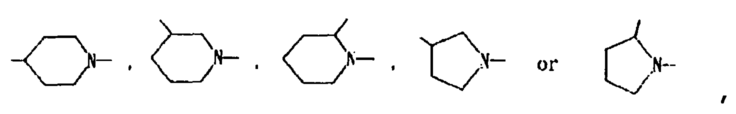
(47) a compound as defined in (41) above, wherein R³ is -SO₂R⁴ (R⁴ is an optionally substituted hydrocarbon group),
(48) a compound as defined in (47) above, wherein R⁴ is an optionally halogenated C₁₋₆ alkyl group,
(49) a compound as defined in (41) above, wherein ring W is 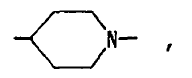
(50) a compound as defined in (41) above, wherein the group of 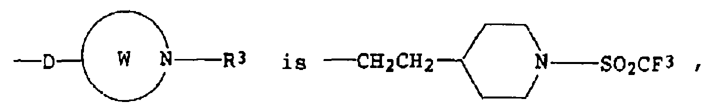
(51) a compound as defined in (41) above, wherein ring Q is unsubstituted,
(52) a compound as defined in (41) above, wherein j is 0,
(53) a compound as defined in (32) above, which is 1,2-dihydro-1-(1-trifluoromethanesulfonylpiperidin-4-ylmethyl)-1,4,7b-triazacyclopent[cd]inden-2-one, or a salt thereof,
(54) a compound as defined in (32) above, which is 1,2-dihydro-1-[2-(1-trifluoromethanesulfonylpiperidin-4-yl)ethan-1-yl]-1,4,7b-triazacyclopent[cd]inden-2-one, or a salt thereof,
(55) a compound as defined in (32) above, which is 1,2-dihydro-1-[3-(1-trifluoromethanesulfonylpiperidin-4-yl)propan-1-yl]-1,4,7b-triazacyclopent[cd]inden-2-one, or a salt thereof,
(56) a compound as defined in (32) above, which is 4,5-dihydro-4-(1-trifluoromethanesulfonylpiperidin-4-ylmethyl)-3H-1,4,8b-triazaacenaphthylen-3-one, or a salt thereof,
(57) a compound as defined in (32) above, which is 4,5-dihydro-4-[2-(1-trifluoromethanesulfonylpiperidin-4-yl)ethan-1-yl]-3H-1,4,8b-triazaacenaphthylen-3-one, or a salt thereof,
(58) a process for producing a compound (I) or a salt thereof, which comprises reacting a compound of the formula: 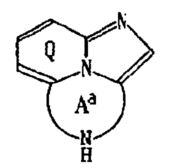 wherein the symbols are as defined above, or a salt thereof with a compound of the formula: 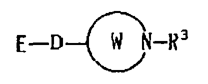 wherein E is a leaving group and the other symbols are as defined above, or a salt thereof,
(59) a pharmaceutical composition which comprises a compound (A) or a salt thereof, in admixture with a pharmaceutically acceptable carrier, excipient or diluent,
(60) a pharmaceutical composition for inhibiting platelet-derived growth factor, which comprises a compound (A) or a salt thereof, in admixture with a pharmaceutically acceptable carrier, excipient or diluent,
(61) a pharmaceutical composition for treating or preventing renal diseases, which comprises a compound (A) or a salt thereof, in admixture with a pharmaceutically acceptable carrier, excipient or diluent,
(62) a pharmaceutical composition for treating or preventing arteriosclerosis, which comprises a compound (A) or a salt thereof, in admixture with a pharmaceutically acceptable carrier, excipient or diluent,
(63) a pharmaceutical composition for treating or preventing reobstruction after percutaneous transluminal coronary angioplasty (hereinafter called PTCA), which comprises a compound (A) or a salt thereof, in admixture with a pharmaceutically acceptable carrier, excipient or diluent,
(64) a pharmaceutical composition for lowering lipid level, which comprises a compound (A) or a salt thereof, in admixture with a pharmaceutically acceptable carrier, excipient or diluent,
(65) a pharmaceutical composition for treating or preventing diabetic nephropathy, which comprises a compound (A) or a salt thereof, in admixture with a pharmaceutically acceptable carrier, excipient or diluent, and
(66) a pharmaceutical composition for treating or preventing chronic glomerulonephritis, which comprises a compound (A) or a salt thereof, in admixture with a pharmaceutically acceptable carrier, excipient or diluent.

The term "nitrogen-containing heterocyclic ring" used in the present specification means, for example, 5- to 10-membered ring containing, two nitrogen atoms as hetero-atoms. Among them, 5- or 6-membered ring is widely used. These ring may be saturated or unsaturated, and may contain 1 or 2 hetero atoms (e.g. sulfur atom, oxygen atom, nitrogen atom). More specifically, for example, the following ones 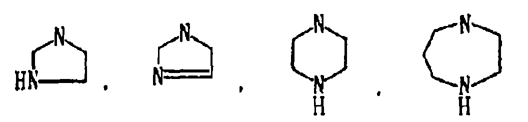 are employed. These "nitrogen-containing heterocyclic rings" may be substituted with one or two of oxo or thioxo groups.

The term "divalent hydrocarbon group" used in the present specification means, for example, divalent chain-like hydrocarbon groups including C₁₋₁₅ alkylene groups (e.g. methylene, ethylene, propylene, butylene, pentamethylene, hexamethylene, heptamethylene and octamethylene), C₂₋₁₆ alkenylene groups (e.g. vinylene, propenylene, 1-butenylene, 2-butenylene, 1-pentenylene, 2-pentenylene and 3-pentenylene), C₂₋₁₆ alkynylene groups (e.g. ethynylene, propynylene, 1-butynylene, 2-butynylene, 1-pentynylene, 2-pentynylene and 3-pentynylene), phenylene group or a combination of them.

As substituents which the said "divalent hydrocarbon group" optionally has, mention is made of, for example, optionally substituted alkyl groups, optionally substituted aralkyl groups and optionally substituted aryl groups, and optionally substituted alkyl groups are preferable. The said "divalent hydrocarbon group" may be substituted with 1 to 5 substituents at any substitutable positions.

The said "phenylene group" may be substituted.

As substituents which the said "phenylene group" optionally has, mention is made of 1 to 4 substituents selected from, for example, halogen atoms (e.g. fluorine, chlorine, bromine and iodine), C₁₋₆ alkyl groups (e.g. methyl, ethyl, propyl, isopropyl and butyl), C₁₋₆ alkoxy groups (e.g. methoxy, ethoxy, propoxy and isopropoxy), C₁₋₆ alkylthio groups (e.g. methylthio, ethylthio, propylthio and isopropylthio), hydroxy group, carboxyl group, cyano group, nitro group, amino group, mono- or di- C₁₋₆ alkylamino groups (e.g. methylamino, ethylamino, dimethylamino and diethylamino), formyl group, mercapto group, C₁₋₆ alkyl-carbonyl groups (e.g. acetyl, propionyl and butyryl), C₁₋₆ alkoxy-carbonyl groups (e.g. methoxycarbonyl, ethoxycarbonyl and propoxycarbonyl), sulfone group, C₁₋₆ alkylsulfonyl groups (e.g. methylsulfonyl, ethylsulfonyl and propylsulfonyl), carbamoyl group, mono- or di- C₁₋₆ alkyl-carbamoyl groups (e.g. N-methylcarbamoyl, N-ethylcarbamoyl, N,N-dimethylcarbamoyl and N,N-diethylcarbamoyl) and thiocarbamoyl group.

The term "halogen atom" used in the present specification means, for example, fluorine, chlorine, bromine and iodine.

The "hydrocarbon group" of the term "optionally substituted hydrocarbon group" used in the present specification means, for example, alkyl group, cycloalkyl group, alkenyl group, aralkyl group and aryl group.

Examples of the substituents, which the said "hydrocarbon group" optionally has, use is made of, the substituents which the said "alkyl group", "cycloalkyl group", "alkenyl group", "aralkyl group" and "aryl group" optionally have. The said "hydrocarbon group" optionally has one to five of these substituents at any substitutable positions.

As said "alkyl group", use is made of, for example, "straight-chain or branched C₁₋₁₅ alkyl group" such as methyl, ethyl, propyl, isopropyl, butyl, isobutyl, sec-butyl, tert-butyl, pentyl, hexyl, heptyl, octyl, nonyl, decyl, undecyl, tridecyl, tetradecyl and pentadecyl.

As said "cycloalkyl group", use is made of, for example, "C₃₋₈ cycloalkyl group" such as cyclopropyl, cyclobutyl, cyclopentyl, cyclohexyl, cycloheptyl and cyclooctyl.

Examples of the substituents, which the said "alkyl group" and "cycloalkyl group" optionally have, include (i) nitro group, (ii) hydroxy group, (iii) cyano group, (iv) carbamoyl group, (v) mono- or di- C₁₋₆ alkyl-carbamoyl groups (e.g. N-methylcarbamoyl, N-ethylcarbamoyl, N,N-dimethylcarbamoyl and N,N-diethylcarbamoyl), (vi) carboxyl group, (vii) C₁₋₆ alkoxy-carbonyl groups (e.g. methoxycarbonyl, ethoxycarbonyl, propoxycarbonyl and isopropoxycarbonyl), (viii) sulfone group, (ix) halogen atoms (e.g. fluorine, chlorine, bromine and iodine), (x) C₁₋₆ alkoxy groups (e.g. methoxy, ethoxy, propoxy and isopropoxy), (xi) phenoxy group, (xii) halogenophenoxy groups substituted with one to three halogen atoms (e.g. o-, m- or p-chlorophenoxy, and o-, m- or p-bromophenoxy), (xiii) C₁₋₆ alkylthio groups (e.g. methylthio, ethylthio, n-propylthio, isopropylthio and n-butylthio), (xiv) mercapto group, (xv) phenylthio group, (xvi) pyridylthio group, (xvii) C₁₋₆ alkylsulfinyl groups (e.g. methylsulfinyl and ethylsulfinyl), (xviii) C₁₋₆ alkylsulfonyl groups (e.g. methylsulfonyl and ethylsulfonyl), (xix) amino group, (xx) C₁₋₆ acylamino groups (e.g. acetylamino and propionylamino), (xxi) mono- or di- C₁₋₆ alkylamino groups (e.g. methylamino, ethylamino, dimethylamino and diethylamino), (xxii) 4- to 6- membered cyclic amino groups (e.g. 1-azetidinyl, 1-pyrrolidinyl, piperidino, morpholino, thiomorpholino and 1-piperazinyl), (xxiii) C₁₋₇ acyl groups (e.g. formyl and C₁₋₆ alkyl-carbonyl group such as acetyl), (xxiv) benzoyl group, (xxv) 5 to 10 membered heterocyclic groups (e.g. 5 or 6-menbered mono-aromatic heterocyclic ring containing one to four hetero atoms selected from nitrogen, sulfur and oxygen or di-aromatic heterocyclic ring which is formed by 5 or 6-membered aromatic heterocyclic ring containing one or two hetero atoms selected nitrogen, sulfur and oxygen condensed with benzene ring such as 2- or 3-thienyl, 2- or 3-furyl, 3-, 4- or 5-pyrazolyl, 2-, 4- or 5-thiazolyl, 3-, 4- or 5-isothiazolyl, 2, 4- or 5-oxazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl and isoquinolyl indolyl) and (xxvi) thiocarbamoyl group. The said "alkyl group" and "cycloalkyl group" optionally have 1 to 5 of these substituents at any substitutable positions.

Preferable examples of the said "alkyl group" include C₁₋₆ straight-chain or branched alkyl groups such as methyl, ethyl, propyl, isopropyl, butyl, isobutyl, sec-butyl, tert-butyl, pentyl and hexyl. As the substituents which the said "C₁₋₆ alkyl groups" optionally have, use is made of 1 to 3 of, for example, halogen atoms, C₁₋₄ alkoxyl group, hydroxy group, C₁₋₄ alkoxy-carbonyl groups, carboxyl group, carbamoyl group, thiocarbamoyl group, mono- or di- C₁₋₄ alkylcarbamoyl groups and pyridylthio group.

Examples of the said "alkenyl group" include "C₂₋₁₈ alkenyl groups" such as vinyl, allyl, isopropenyl, 3-butenyl, 3-octenyl and 9-octadecenyl. As the substituents which the said "alkenyl groups" optionally have, use is made of, for example, the same ones as those which the above-mentioned "alkyl group" optionally has.

Preferable examples of the said "alkenyl group" include C₂₋₆ alkenyl groups such as vinyl, allyl, 2-butenyl and 3-butenyl. As the substituents which the said "C₂₋₆ alkenyl groups" optionally has, use is made of, for example, the same ones as those which the above-mentioned "C₁₋₆ alkyl group" optionally has.

As the said "aralkyl group", use is made of, for example, C₇₋₁₆ aralkyl groups, which are specifically exemplified by phenyl- C₁₋₆ alkyl groups such as benzyl, phenethyl, 3-phenylpropyl and 4-phenylbutyl, and naphthyl- C₁₋₆ alkyl group such as (1-naphthyl)methyl, 2-(1-naphthyl)ethyl and 2-(2-naphthyl)ethyl. Preferable examples of the said "aralkyl group" include phenyl-C₁₋₆ alkyl group such as benzyl.

As the substituents which the said "aralkyl group" optionally has, use is made of, besides the substituents which the said "alkyl group" optionally has, for example, C₁₋₆ alkyl group (e.g. methyl, ethyl, propyl, isopropyl, buthyl), halogeno-C₁₋₆ alkyl groups such as C₁₋₆ alkyl group substituted with 1 to 5 halogen atoms, oxo group. Preferable examples of the substituents, which the said "aralkyl group" optionally has, include halogen atoms (e.g. fluorine, chlorine, bromine and iodine), C₁₋₄ alkyl groups (e.g. methyl, ethyl, propyl, isopropyl and butyl), halogeno-C₁₋₄ alkyl groups (e.g. trifluoromethyl, trichloromethyl), C₂₋₆ alkenyl groups (e.g. vinyl, allyl, 2-butenyl and 3-butenyl), C₁₋₃ acyl groups (e.g. formyl and acetyl), C₁₋₄ alkoxy groups (e.g. methoxy, ethoxy, propoxy and isopropoxy), nitro group, cyano group, hydroxy group, C₁₋₄ alkoxy-carbonyl groups (e.g. methoxycarbonyl, ethoxycarbonyl, propoxycarbonyl and isopropoxycarbonyl), carbamoyl group, thiocarbamoyl group, mono- or di- C₁₋₄ alkyl-carbamoyl groups (e.g. N-methylcarbamoyl, N-ethylcarbamoyl, N,N-dimethylcarbamoyl and N,N-diethylcarbamoyl) and mono- or di- C₁₋₄ alkenyl-carbamoyl groups (e.g. N-vinylcarbamoyl). The said "aralkyl group" may have 1 to 4 of these substituents at any substitutable position.

As the said "aryl group", use is made of, for example, aromatic monocyclic, dicyclic or tricyclic C₆₋₁₄ aryl groups exemplified by phenyl, 1-naphthyl, 2-naphthyl, phenanthryl and anthryl. Preferable example of the said "aryl group" include C₆₋₁₀ aryl groups such as phenyl.

As the substituents which the said "aryl group" optionally has, use is made of, for example, the same ones as those which the above-mentioned "aralkyl group" optionally has. As preferable examples of the substituents which the said "aryl group" optionally has, use is made of, besides preferable examples of the substituents which the said "aralkyl group" may have, oxo group. The said "aryl group" may have 1 to 4, preferably 1 or 2, of these substituents at any substitutable positions. Examples of the aryl group having oxo group include benzoquinonyl, naphthoquinolyl and anthraquinonyl.

The term "electron-withdrawing group" used in the present specification is exemplified by (i) -SO₂R⁴, (ii) -CO-R⁵, (iii) -COOR⁶,(iv) -CON(R⁷)R⁸, (v) a cyano group and (vi) a nitro group, preferably -SO₂R⁴, -CO-R⁵ and -COOR⁶, especially -SO₂R⁴ is commonly used. R⁴ stands for an optionally substituted hydrocarbon group; R⁵ stands for a hydrogen atom or an optionally substituted hydrocarbon group; R⁶ stands for an optionally substituted hydrocarbon group; R⁷ and R⁸ independently stand for a hydrogen atom or an optionally substituted hydrocarbon group, or R⁷ and R⁸ form, combined with the adjacent nitrogen atom, an nitrogen atom-containing heterocyclic ring.

The term "acyl group" used in the present specification is exemplified by the acyl group derived from carboxylic acid, which is exemplified by alkoxycarbonyl group, alkylcarbamoyl group and alkanoyl group.

As the said "alkoxycarbonyl group", use is made of C₁₋₆ alkoxycarbonyl groups including, for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl and tert-pentyloxycarbonyl.

As the said "alkylcarbamoyl group", use is made of mono-C₁₋₆-N-alkylcarbamoyl groups, for example, N-methylcarbamoyl, N-ethylcarbamoyl, N-propylcarbamoyl and N-butylcarbamoyl, and di-C₁₋₆-N,N-dialkylcarbamoyl groups, for example, N,N-dimethylcarbamoyl, N,N-diethylcarbamoyl, N,N-dipropylcarbamoyl, N,N-dibutylcarbamoyl and N-ethyl-methylcarbamoyl, and 4- to 6-membered cyclic carbamoyl groups formed by combination of the dialkyl portions with each other (e.g. 1-azetidinylcarbonyl, morpholinocarbonyl, 1-pyrrolidinylcarbonyl, 1-piperidinocarbonyl and 1-piperazinylcarbonyl).

As the said "alkanoyl group", use is made of formyl, C₁₋₆ alkyl-carbonyl group (e.g. acetyl, propionyl).

In the above-mentioned formula, the ring A stands for a nitrogen-containing heterocyclic ring having two nitrogen atoms containing the nitrogen atom at the head of the bridge in the condensed ring, which be further substituted with oxo or thioxo.

Preferable examples of the ring A include 5- or 6-membered nirogen-containing heterocyclic ring optionally substituted with one or two oxo groups. Especially, the following ones are commonly employed.

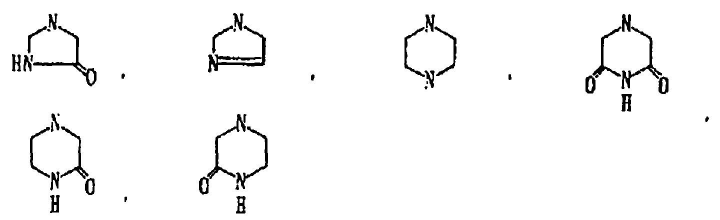

In the above-mentioned formula, the ring Q is optionally substituted.

Examples of the substituents, which the ring Q optionally has, include halogen atoms (e.g. fluorine, chlorine, bromine and iodine), C₁₋₆ alkyl groups (e.g. methyl, ethyl, propyl, isopropyl and butyl), halogeno-C₁₋₆ alkyl groups such as C₁₋₆ alkyl groups substituted with 1 to 5 halogen atoms (e.g. trifluoromethyl, trichloromethyl), C₁₋₆ alkoxy groups (e.g. methoxy, ethoxy, propoxy and isopropoxy), halogeno-C₁₋₆ alkoxy groups such as C₁₋₆ alkoxy group substituted with 1 to 5 halogen atoms (e.g. trifluoromethoxy, trichloromethoxy), C₁₋₆ alkylthio groups (e.g. methylthio, ethylthio, propylthio and isopropylthio), halogeno-C₁₋₆ alkylthio groups such as C₁₋₆ alkylthio group substituted with 1 to 5 halogen atoms (e.g. trifluoromethylthio, trichloromethylthio), hydroxy group, carboxyl group, cyano group, nitro group, amino group, mono- or di- C₁₋₆ alkyl amino groups (e.g. methylamino, ethylamino, dimethylamino and diethylamino), formyl group, mercapto group, C₁₋₆ alkyl-carbonyl groups (e.g. acetyl, propionyl and butyryl), C₁₋₆ alkoxy-carbonyl groups (e.g. methoxycarbonyl, ethoxycarbonyl and propoxycarbonyl), sulfone group, C₁₋₆ alkylsulfonyl groups (e.g. methylsulfonyl, ethylsulfonyl and propylsulfonyl), carbamoyl group, mono- or di- C₁₋₆ alkyl-carbamoyl groups (e.g. N-methylcarbamoyl, N-ethylcarbamoyl, N,N-dimethylcarbamoyl and N,N-diethylcarbamoyl) and thiocarbamoyl group. The ring Q may be substituted with 1 to 3 of these substituents at any substitutable position. The ring Q is preferably unsubstituted.

In the above-mentioned formula, Y stands for an optionally substituted hydrocarbon group, an optionally substituted hydroxy group or an optionally substituted mercapto group.

Examples of the optionally substituted hydrocarbon group shown by Y include those described in respect of the above-mentioned "optionally substituted hydrocarbon group".

Examples of the substituents, which the hydroxy group or the mercapto group shown by Y optionally has, include optionally substituted hydrocarbon group, the groups containing at least one nitrogen atom and/or the groups containing at least one electron-withdrawing groups.

Preferable examples of the substituents, which the hydroxy group or the mercapto group shown by Y optionally has, include optionally substituted hydrocarbon group. As the said "optionally substituted hydrocarbon group", use is made of the same ones as the above-mentioned "optionally substituted hydrocarbon group".

Preferable examples of the substituents, which the hydrocarbon group, the hydroxy group and the mercapto group shown by Y optionally has, include the groups containing at least one nitrogen atom and/or the groups containing at least one electron-withdrawing group, especially the groups containing an amino group substituted with at least one electron-withdrawing group.

As "the group containing at least one nitrogen atom" mentioned above, use is made of, for example, alkylaminoalkyl groups aralkylaminoalkyl groups, arylaminoalkyl groups, alkylaminoaralkyl groups, aralkylaminoaralkyl groups, arylaminoaralkyl groups, alkylaminoaryl groups, aralkylaminoaryl groups, arylaminoaryl groups, aminoalkyl groups, aminoaralkyl group and aminoaryl groups.

As "the group containing at least one electron-withdrawing group", use is made of, for example, "the hydrocarbon groups containing at least one electron-withdrawing group" as mentioned above.

As "the group containing an amino group which is substituted with at least one electron-withdrawing group", use is made of, for example, "the hydrocarbon groups containing an amino group substituted with at least one electron-withdrawing group" as mentioned above.

The most preferable examples of Y include the groups represented by the formula 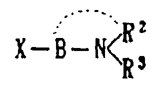 wherein the symbols are of the same meaning as defined above.

In the above-mentioned formula, B stands for an optionally substituted divalent hydrocarbon group. Specific examples of the group include those represented by (i) 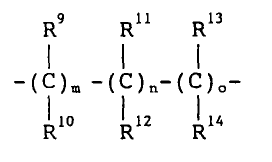 wherein m, n and o independently are integers of 0 to 5, R⁹, R¹⁰, R¹¹, R¹², R¹³ and R¹⁴ independently stand for a hydrogen atom, an optionally substituted alkyl group, an optionally substituted aralkyl group or an optionally substituted aryl group, and, R⁹ and R¹⁰; R¹¹ and R¹²;, R¹³ and R¹⁴; R⁹ or R¹⁰ and R²; R¹¹ or R¹² and R²;, or, R¹³ or R¹⁴ and R² may respectively be combined to form rings, and R⁹ or R¹¹ may be combined with R¹³ or R¹⁴ respectively to form rings, or (ii) 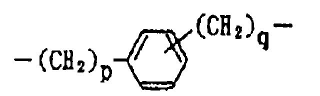 wherein phenylene group may be substituted, and, p and q are independently an integer of 0 to 5. Examples of the optionally substituted alkyl, aralkyl or aryl group shown by R⁹ to R¹⁴ include those described in respect of the above-mentioned "optionally substituted hydrocarbon groups". The rings formed by combination of R⁹ and R¹⁰, R¹¹ and R¹², and R¹³ and R¹⁴ are exemplified by C₃₋₈ cycloalkanes including cyclopropane, cyclobutane, cyclopentane and cyclohexane. The rings formed by combination of R⁹ or R¹⁰ and R²; R¹¹ or R¹² and R²; or R¹³ or R¹⁴ and R² are exemplified by azetidine, pyrrolidine or piperidine. The rings formed by combination of R⁹ or R¹¹ with R¹³ or R¹⁴, respectively are exemplified by C₃₋₈ cycloalkanes including cyclopropane, cyclobutane, cyclopentane and cyclohexane.

Preferable examples of R⁹ to R¹⁴ include a hydrogen atom or C₁₋₄ alkyl groups (e.g. methyl, ethyl, propyl and isopropyl), and, especially, a hydrogen atom or methyl group is preferably used.

Preferable examples of B include C₂₋₁₀ alkylene groups (e.g. ethylene, propylene, butylene, pentamethylene, hexamethylene, heptamethylene and octamethylene), and, among them, especially C₂₋₈ alkylene groups (e.g. ethylene, propylene, butylene, pentamethylene, hexamethylene and heptamethylene) are commonly employed.

In the above-mentioned formulae, X stands for a bond, an oxygen atom or a sulfur atom. Preferable example of X is a bond.

In the above-mentioned formulae, R¹ stands for a hydrogen atom, a halogen atom, an optionally substituted hydrocarbon group or acyl group.

Preferable examples of R¹ include a hydrogen atom, optionally substituted alkyl groups, optionally substituted alkenyl groups, optionally substituted aralkyl groups, optionally substituted aryl groups, alkoxycarbonyl groups, alkylcarbamoyl groups and alkanoyl groups; especially a hydrogen atom, C₁₋₆ alkyl groups (e.g. methyl, ethyl, propyl, isopropyl and butyl) or phenyl group are preferably used, and, a hydrogen atom is employed most preferably.

In the above-mentioned formulae, R² stands for a hydrogen atom or an optionally substituted hydrocarbon group, and, R² and B may form a ring together with the adjacent nitrogen atom.

Preferable examples of R² include a hydrogen atom, optionally substituted alkyl groups or optionally substituted alkenyl groups, especially a hydrogen atom is commonly used.

In the above-mentioned formulae, R³ and R^{3a} stand for an electron-withdrawing group, or R² and R^{3a} may form a ring together with the adjacent nitrogen atom. The said "the electron-withdrawing group" is selected from groups which is easy to withdraw an electron from combined atoms compared with hydrogen atoms. Examples of the electron-withdrawing group include (i) -SO₂R⁴ (R⁴ stands for an optionally substituted hydrocarbon group), (ii) -CO-R⁵ (R⁵ stands for a hydrogen atom or an optionally substituted hydrocarbon group), (iii) -COOR⁶ (R⁶ stands for an optionally substituted hydrocarbon group), (iv) -CON(R⁷)R⁸ (R⁷ and R⁸ each stand for a hydrogen atom or an optionally substituted hydrocarbon group, or R⁷ and R⁸ may form a ring together with the adjacent nitrogen atom), (v) a nitro group, (vi) a cyano group and (vii) halogeno-alkyl group (e.g. halogeno-C₁₋₆ alkyl group such as C₁₋₆ alkyl group which may have 1 to 5 halogen atoms such as chloromethyl, trifluoromethyl, trichloromethyl, 2,2,2-trifluoroethyl, 3,3,3-trifluoropropyl, pentafluoroethyl).

Examples of the electron-withdrawing group include -SO₂R^{4a}, -CO-R^{5a} and -COOR^{6a} (R^{4a}, R^{5a} and R^{6a} each stand for an optionally substituted alkyl group, an optionally substituted alkenyl group, an optionally substituted aralkyl group or an optionally substituted aryl group), especially -SO₂R^{4a} (R^{4a} stands for an optionally substituted alkyl group, an optionally substituted alkenyl group, an optionally substituted aralkyl group or an optionally substituted aryl group) is commonly used among others.

Preferable examples of R⁴ include an optionally substituted alkyl group, especially a halogeno- C₁₋₆ alkyl group (e.g. chloromethyl, trifluoromethyl, 2,2,2-trifluoroethyl and 3,3,3-trifluoropropyl).

Preferable examples of R⁵ include an optionally substituted alkyl group, especially a halogeno- C₁₋₆ alkyl group (e.g. chloromethyl, trifluoromethyl, 2,2,2-trifluoroethyl and 3,3,3-trifluoropropyl).

Preferable examples of R⁶ include an optionally substituted alkyl group, especially a halogeno- C₁₋₆ alkyl group (e.g. chloromethyl, trifluoromethyl, 2,2,2-trifluoroethyl and 3,3,3-trifluoropropyl).

Preferable examples of R⁷ and R⁸ include a hydrogen atom or an optionally substituted alkyl group, especially a hydrogen atom or a halogeno- C₁₋₆ alkyl group (e.g. chloromethyl, trifluoromethyl, 2,2,2-trifluoroethyl and 3,3,3-trifluoropropyl).

Examples of the ring, which R² and R^{3a} form together with the adjacent nitrogen atom, include pyrrolidin-2-one, piperidin-2-one, indolin-2-one, isoindolin-1-one, isoindoline-1,3-dione, oxazolidin-2-one, oxazolidine-2,4-dione, thiazolidin-2-one, thiazolidine-2,4-dione and 1,2-benzisothiazol-3(2H)-one. These rings optionally have substituents such as electron-withdrawing groups. As the said "electron-withdrawing group", use is made of, for example, the above-mentioned "electron-withdrawing groups".

Preferable examples of the compounds (I') are shown as follows:

Compounds of the following formula or salts thereof. 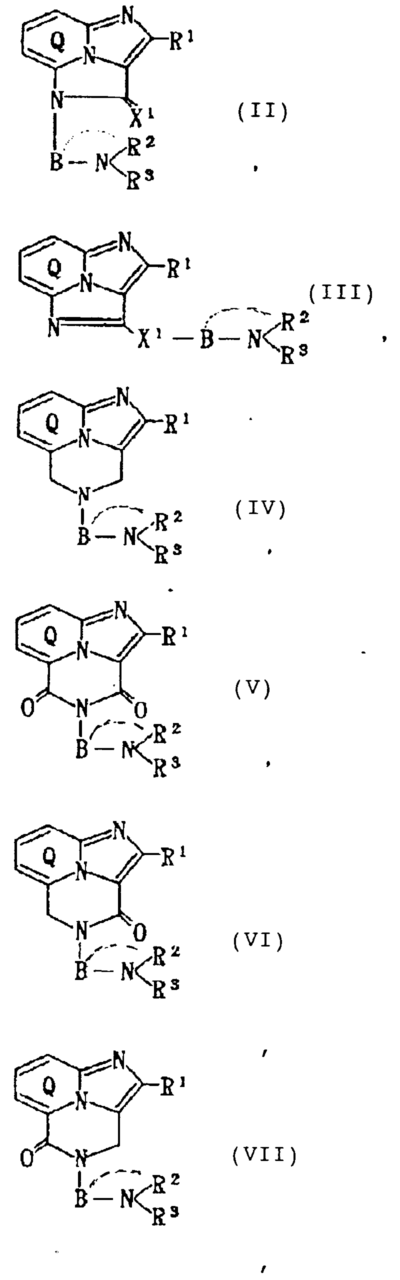 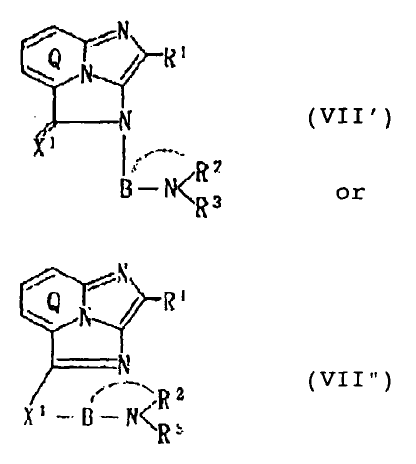 wherein X¹ stands for an oxygen atom or a sulfur atom, and the other symbols are of the same meaning as defined above, especially the compound (II) or (VI). In these compounds (II) to (VII''),
(1) a compound, wherein ring Q is unsubstituted, is preferable;
(2) a compound, wherein R¹ stands for a hydrogen atom, an optionally substituted alkyl group or an optionally substituted alkenyl group, is preferable, and, especially those, wherein R¹ stands for a hydrogen atom or a C₁₋₆ alkyl group (e.g. methyl, ethyl, propyl, isopropyl and butyl), are preferably used;
(3) a compound, wherein R² stands for a hydrogen atom or a C₁₋₆ alkyl group (e.g. methyl, ethyl, propyl, isopropyl and butyl), is preferable, and, especially those, wherein R² stands for a hydrogen atom, are preferably used;
(4) a compound, wherein X¹ stands for an oxygen atom, is preferable;
(5) a compound, wherein X¹ stands for a sulfur atom, is preferable;
(6) a compound, wherein B stands for a C₂₋₁₀ alkylene group (e.g. ethylene, propylene, butylene, pentamethylene, hexamethylene, heptamethylene and octamethylene), is preferable, and, especially those, wherein B stands for a C₃₋₈ alkylene group (e.g. propylene, butylene, pentamethylene, hexamethylene and heptamethylene), are preferable;
(7) a compound, wherein the electron-withdrawing group shown by R³ is -SO₂R^{4a} (R^{4a} stands for an optionally substituted alkyl group, an optionally substituted alkenyl group, an optionally substituted aralkyl group or an optionally substituted aryl group), is preferable; and
(8) a compound, wherein R⁴ stands for a halogeno- C₁₋₆ alkyl group (e.g. chloromethyl, trifluoromethyl, 2,2,2-trifluoroethyl and 3,3,3-trifluoropropyl), is preferable.

In these compounds (II) to (VII''), the following compounds (1) to (4) are novel compounds.
(1)In the above formula (II), a compound wherein Q is unsubstituted, X¹ is oxygen atom, R¹ is a C₁₋₆ alkyl group (preferably, methyl), the group represented by the formula: 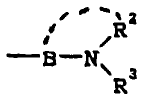 wherein the all symbols are of the same meanings as defined above is 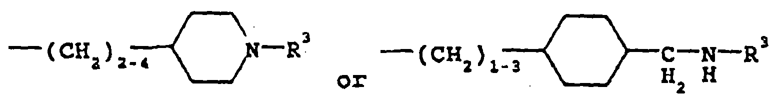 and R³ is -SO₂R⁴ or -COR⁵ wherein R⁴ and R⁵ are of all same meanings as defined above.
(2)In the above formula (III), a compound wherein Q is unsubstituted, X¹ is sulfur atom, R¹ is a hydrogen atom or a C₁₋₆ alkyl group (preferably, methyl), the group represented by the formula: 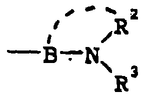 wherein the all symbols are of the same meanings as defined above is 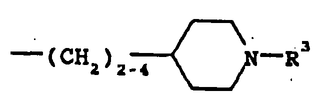 and R³ is -SO₂R⁴ or -COR⁵ wherein R⁴ and R⁵ are of all same meanings as defined above.
3)In the above formula (V), a compound wherein Q is unsubstituted, R¹ is a hydrogen atom or a C₁₋₆ alkyl group (preferably, methyl), the group represented by the formula: 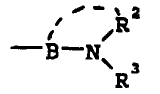 wherein the all symbols are of the same meanings as defined above is 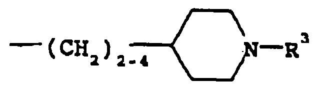 and R³ is -SO₂R⁴ or -COR⁵ wherein R⁴ and R⁵ are of all same meanings as defined above.
(4) In the above formula (IV), (VI) or (VII), a compound wherein Q is unsubstituted, R¹ is a C₁₋₆ alkyl group (preferably, methyl), the group represented by the formula: 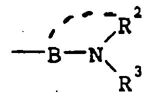 wherein the all symbols are of the same meanings as defined above is 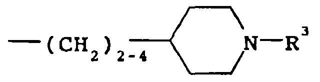 and R³ is -SO₂R⁴ or -COR⁵ wherein R⁴ and R⁵ are of all same meanings as defined above.

Practical examples are set forth as follows:
1,2-dihydro-3-methyl-1-[3-(1-tert-butoxycarbonylpiperidin-4-yl)propan-1-yl]-1,4,7b-triazacyclopent[cd]inden-2-one,
1,2-dihydro-3-methyl-1-[3-(1-trifluoroacetylpiperidin-4-yl)propan-1-yl]-1,4,7b-triazacyclopent[cd]inden-2-one,
1,2-dihydro-3-methyl-1-[3-(1-pentafluoropropionylpiperidin-4-yl)propan-1-yl]-1,4,7b-triazacyclopent[cd]inden-2-one,
1,2-dihydro-3-methyl-1-[3-(1-trifluoromethanesulfonylpiperidin-4-yl)propan-1-yl]-1,4,7b-triazacyclopent[cd]inden-2-one,
3-methyl-2-[2-(1-tert-butoxycarbonylpiperidin-4-yl)ethan-1-yl]thio-1,4,7b-triazacyclopent[cd]indene,
3-methyl-2-[2-(1-trifluoromethanesulfonylpiperidin-4-yl)ethan-1-yl]thio-1,4,7b-triazacyclopent[cd]indene,
1,2-dihydro-3-methyl-1-[2-(1-trifluoroacetylpiperidin-4-yl)ethan-1-yl]-1,4,7b-triazacyclopent[cd]inden-2-one,
1,2-dihydro-3-methyl-1-[2-(1-pentafluoropropionylpiperidin-4-yl)ethan-1-yl]-1,4,7b-triazacyclopent[cd]inden-2-one,
1,2-dihydro-3-methyl-1-[trans-4-(tert-butoxycarbonylamino)methyl-1-cyclohexymethyl]-1,4,7b-triazacyclopent[cd]inden-2-one,
1,2-dihydro-3-methyl-1-[trans-4-(trifluoroacetylamino)methyl-1-cyclohexylmethyl]-1,4,7b-triazacyclopent[cd]inden-2-one,
1,2-dihydro-3-methyl-1-[trans-4-(pentafluoropropionylamino)methyl-1-cyclohexylmethyl]-1,4,7b-triazacyclopent[cd]inden-2-one,
1,2-dihydro-3-methyl-1-[trans-4-(trifluoromethanesulfonylaminomethyl-1-cyclohexylmethyl]-1,4,7b-triazacyclopent[cd]inden-2-one,
1,2-dihydro-3-methyl-1-[trans-4-(4-methylphenylsulfonylamino)methyl-1-cyclohexylmethyl]-1,4,7b-triazacyclopent[cd]inden-2-one,
4,5-dihydro-4-[2-(1-trifluoromethanesulfonylpiperidin-4-yl)ethan-1-yl]-3H-1,4,8b-triazaacenaphthylen-3,5-dione,
2-[2-(1-trifluoromethanesulfonylpiperidin-4-yl)ethan-1-yl]thio-1,4,7b-triazacyclopent[cd]indene,
4,5-dihydro-2-methyl-4-[2-(1-trifluoromethanesulfonylpiperidin-4-yl)ethan-1-yl]-3H-1,4,8b-triazaacenaphthylen-3,5-dione,
4,5-dihydro-2-methyl-4-[2-(1-trifluoromethanesulfonylpiperidin-4-yl)ethan-1-yl]-3H-1,4,8b-triazaacenaphthylene,
4,5-dihydro-2-methyl-4-[2-(1-trifluoromethanesulfonylpiperidin-4-yl)ethan-1-yl]-3H-1,4,8b-triazaacenaphthylen-3-one.
4,5-dihydro-2-methyl-4-[2-(1-trifluoromethanesulfonylpiperidin-4-yl)ethan-1-yl]-3H-1,4,8b-triazaacenaphthylen-5-one, and their salts.

More preferable example of the compound (I) is a compound (A) represented by the formula: 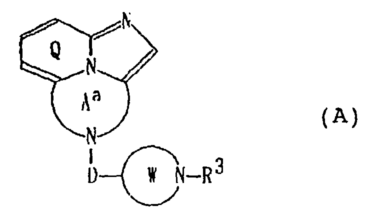 wherein the all symbols are of the same meanings as defined above.

In the compound (A), ring Q is of the same meaning as defined above, and preferably unsubstituted ring.

In the compound (A), ring A^{a} is a nitrogen-containing heterocyclic ring which may have one oxo group. The "nitrogen-containing heterocyclic ring" have two nitrogen atoms other than carbon atoms. Preferable examples of the nitrogen-containing heterocyclic ring represented by A^{a} are a 5- to 10-membered nitrogen-containing heterocyclic ring, and more preferably 5- or 6-membered nitrogen-containing heterocyclic ring.

In the compound (A), examples of the moiety: 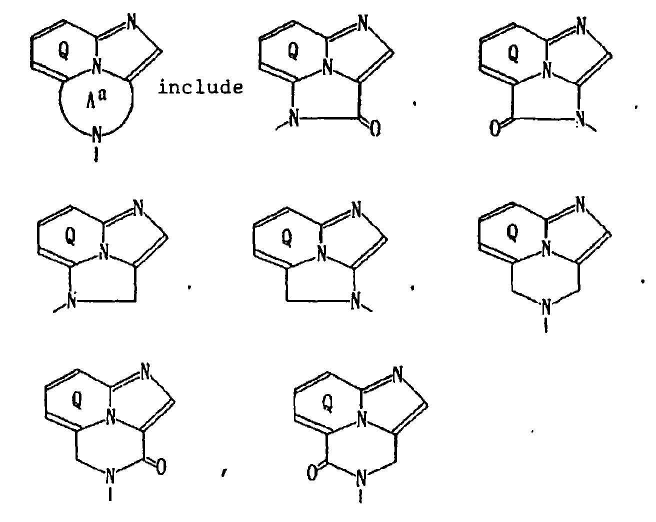 wherein ring Q is of the same meaning as defined above, and preferably the moiety: 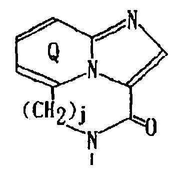 wherein j is 0 or 1, and preferably 0, and ring Q is of the same meaning as defined above.

In the compound (A), D is a bond or an optionally substituted divalent hydrocarbon. D is preferably an optionally substituted divalent hydrocarbon. The "optionally substituted divalent hydrocarbon" is of the same meaning as defined in above. The "divalent hydrocarbon" is preferably, for example, divalent liner hydrocarbon such as C₁₋₁₅ alkylene group (e.g. methylene, ethylene, propylene, butylene, pentamethylene, hexamethylene, heptamethylene, octamethylene), C₂₋₁₆ alkenylene group (e.g. vinylene, propenylene, 1-butenylene, 2-butenylene, 1-pentenylene, 2-pentenylene, 3-pentenylene), C₂₋₁₆ alkynylene group (e.g. ethynylene, propynylene, 1-butynylene, 2-butynylene, 1-pentynylene, 2-pentynylene, 3-pentynylene), phenylene group, and a combination of them. And more preferable example of the "divalent hydrocarbon group" is C₁₋₁₅ alkylene group (e.g. methylene, ethylene, propylene, butylene, pentamethylene, hexamethylene, heptamethylene, octamethylene).

The substituent of the "divalent hydrocarbon group" is exemplified by the same substituents as those of the "divalent hydrocarbon group" as mentioned above. Preferable example of the substituent of the divalent liner hydrocarbon are C₁₋₆ alkyl group (e.g. methyl, ethyl, propyl, isopropyl, buthyl) and phenyl group. The substituent of the phenylene group is exemplified by the same substituents as those of the phenylene group as mentioned above.

In the compound (A), ring W is an optionally substituted nitrogen-containing heterocyclic ring, and preferably an optionally substituted 5- or 6-membered nitrogen-containing heterocyclic ring. These ring may be saturated or unsaturated.

Preferable examples of the "nitrogen-containing heterocyclic ring" are the moiety: 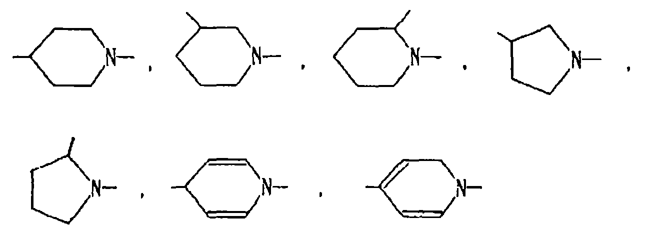 and more preferably the moiety: 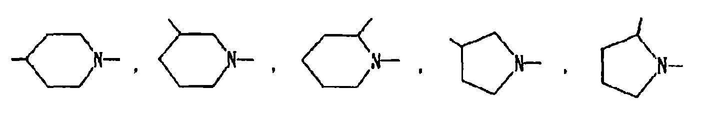 and most preferably the moiety:

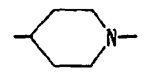

The substituent of the "nitrogen-containing heterocyclic group" is exemplified by the same one as the substituent of ring Q.

In the compound (A), R³ is of the same meaning as defined above.

Preferable example of the compound (A) is a compound (A-a) represented by the formula: 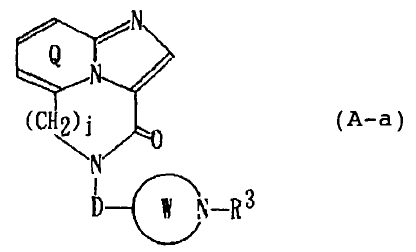 wherein all symbols are of the same meanings as defined above.

In the compound (A-a), ring Q is preferably unsubstituted pyridine ring.

In the compound (A) and (A-a), R³ is preferably -SO₂R⁴ (R⁴ is an optionally substituted hydrocarbon group), -COR⁵ (R⁵ is a hydrogen atom is an optionally substituted hydrocarbon group) or -COOR⁶ (R⁶ is an optionally substituted hydrocarbon group) and more preferably -SO₂R⁴ (R⁴ is an optionally substituted hydrocarbon group). And, preferable examples of R⁴, R⁵ and R⁶ are an optionally halogenated hydrocarbon group (e.g. C₁₋₆ alkyl group which may have 1 to 5 halogen atoms such as methyl, ethyl, propyl, isopropyl, butyl, chloromethyl, trifluoromethyl, 2,2,2-trifluoroethyl, 3,3,3-trifluoropropyl, or pentafluoroethyl).

In the compound (A-a), D is preferably a C₁₋₆ alkylene (e.g. methylene, ethylene, propylene, butylene, pentamethylene, trimethylene, tetramethylene), and more preferably ethylene.

In the compound (A-a), ring W is preferably a moiety: 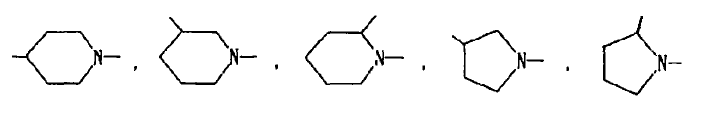 , and more preferably a moiety:

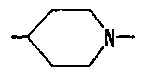

In the compound (A-a), j is preferably 0.

In the compound (A-a), R³ is more preferably -SO₂R⁴ (R⁴ is an optionally substituted hydrocarbon group). And, R⁴ is preferably a C₁₋₆ alkyl group which may have 1 to 5 halogen atoms such as methyl, ethyl, propyl, isopropyl, butyl, chloromethyl, trifluoromethyl, 2,2,2-trifluoroethyl, 3,3,3-trifluoropropyl, or pentafluoroethyl) and more preferably trifluoromethyl.

As preferable salts of the compound (I') (hereinafter referred to as "compound (I')" including the compound (I), (II), (III), (IV), (V), (VI), (VII), (VII'), (VII''), (A) and (A-a)), mention is especially made of pharmaceutically acceptable salts and physiologically acceptable acid addition salts. These salts are exemplified by those with inorganic acids (e.g. hydrochloric acid, phosphoric acid, hydrobromic acid and sulfuric acid) or with organic acids (e.g. acetic acid, formic acid, propionic acid, fumaric acid, maleic acid, succinic acid, tartaric acid, citric acid, malic acid, oxalic acid, benzoic acid, methansulfonic acid and benzenesulfonic acid). Further, when the compound (I') of the present invention has an acid group such as carboxylic group, it may optionally form a salt with, for example, an inorganic base (e.g. an alkali metal or an alkaline earth metal such as sodium, potassium, calcium and magnesium, or ammonia) or an organic base (e.g. a tri- C₁₋₃ alkylamine such as triethylamine).

As the starting compounds for producing the desired compound (I') of the present invention, similar salts to those mentioned above are employed, and they are not specifically limited unless they exert undesirable influence upon the reaction.

The compound (I') may be used as a hydrate or a non-hydrate.

The compound (I') or a salt thereof has, in some instances, asymmetric carbons in the molecule. When two kinds of stereoisomers of R-configuration and S-configurated isomers, are present, each of them and a mixture of them are all included in the scope of the present invention.

Preferable practical examples of the compound (I') and salts thereof are set forth as follows;
1,2-dihydro-3-methyl-1-[5-(trifluoromethanesulfonamido)pentan-1-yl]-1,4,7b-triazacyclopent[cd]inden-2-one,
4,5-dihydro-4-[4-(trifluoromethanesulfonamido)butan-1-yl]-3H-1,4,8b-triazaacenaphthylen-3-one,
1,2-dihydro-1-[2-(1-trifluoromethanesulfonylpiperidin-4-yl)ethan-1-yl]-1,4,7b-triazacyclopent[cd]inden-2-one and their salts (preferable example of the salts is hydrochloride).

The compound (I') or a salt thereof can be synthesized by methods disclosed in, for example, WO 96/02542, or analogous methods thereto.

The Compound 1
1,2-dihydro-3-methyl-1-[5-(trifluoromethanesulfonamido)pentan-1-yl]-1-4,7b-triazacyclopent[cd]inden-2-one hydrochloride, the Compound 2
4,5-dihydro-4-(4-(trifluoromethanesulfonamido)butan-1-yl]-3H-1,4,8b-triazaacenaphthylen-3-one hydrochloride, and the Compound 3
1,2-dihydro-3-methyl-1-[2-(1-trifluoromethanesulfonylpiperidin-4-yl)ethan-1-yl]-1,4,7b-triazacyclopent[cd]inden-2-one hydrochloride can be synthesized by the methods disclosed in Example 26, Example 6 and Example 71 of WO 96/02542.

Preferable practical examples of the compound (A) and salts thereof are set forth as follows;
1,2-dihydro-1-(1-trifluoromethanesulfonylpiperidin-4-ylmethyl)-1,4,7b-triazacyclopento[cd]inden-2-one,
1,2-dihydro-1-[2-(1-trifluoromethanesulfonylpiperidin-4-yl)ethan-1-yl]-1,4,7b-triazacyclopent[cd]inden-2-one,
1,2-dihydro-1-[3-(1-trifluoromethanesulfonylpiperidin-4-yl)propan-1-yl]-1,4,7b-triazacyclopent[cd]indene-2-one,
4,5-dihydro-4-(1-trifluoromethanesulfonylpiperidin-4-ylmethyl)-3H-1,4,8b-triazaacenaphthylen-3-one,
4,5-dihydro-4-[2-(1-trifluoromethanesulfonylpiperidin-4-yl)ethan-1-yl]-3H-1,4,8b-triazaacenaphthylen-3-one,
4,5-Dihydro-4-[2-(1-tert-butoxycarbonylpiperidin-4-yl)ethan-1-yl]-3H-1,4,8b-triazaacenaphthylene,
4,5-Dihydro-4-[2-(1-trifluoromethanesulfonylpiperidin-4-yl)ethan-1-yl]-3H-1,4,8b-triazanaphthylene dihydrochloride,
   and their salts (preferable example of the salts is hydrochloride). Among others, especially preferable practical examples of the compound (A) and salts thereof are set forth as follows;
1,2-dihydro-1-[2-(1-trifluoromethanesulfonylpiperidin-4-yl)ethan-1-yl]-1,4,7b-triazacyclopent[cd]inden-2-one and their salts (preferable example of the salts is hydrochloride).

The compound (A) or a salt thereof of the present invention can be synthesized by methods disclosed in, for example, WO 96/02542, or analogous methods thereto, or, for example, the following methods,
(1) a process for producing the compound (A) or a salt thereof which comprises reacting a compound of the formula: 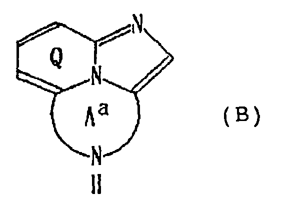 wherein all symbols are of the same meanings as defined above, or a salt thereof, with a compound of the formula: 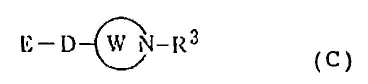 wherein all symbols are of the same meanings as defined above, or a salt thereof,
(2) a process for producing the compound (A) or a salt thereof which comprises reacting a compound of the formula: 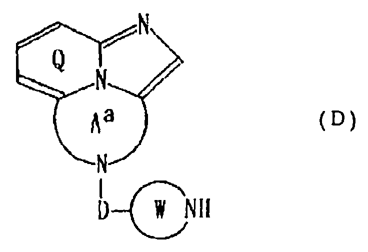 wherein all symbols are of the same meanings as defined above, or a salt thereof, with a compound of the formula: G¹ - R³
   wherein G¹ stands for a halogen atom (e.g. chlorine, bromine and iodine) or OR³ and the other symbol is of the same meanings as defined above, or a salt thereof,
(3) a process for producing the compound (A) (the compound wherein j is 0 in the formula (A-a)) or a salt thereof which comprises reacting a compound of the formula: 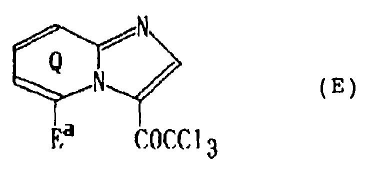 wherein E^{a} is a leaving group such as a halogen atom (e.g. chlorine, bromine, iodine), a methanesulfonyloxy group, p-toluenesulfonyloxy group and the other symbol is of the same meanings ad defined above, or a salt thereof, with a compound of the formula: 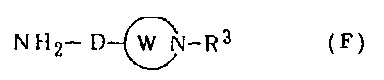 wherein all symbols are of the same meanings as defined above, or a salt thereof,
(4) a process for producing the compound (A) (the compound wherein j is 1 in the formula (A-a)) or a salt thereof which comprises reacting a compound of the formula: 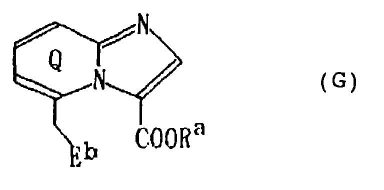 wherein E^{b} is a leaving group such as a halogen atom (e.g. chlorine, bromine, iodine), a methanesulfonyl group, a p-toluenesulfonyloxy group, and R^{a} is C₁₋₆ alkyl group (e.g. methyl, ethyl, propyl, isopropyl), and the other symbol is of the same meanings as defined above, or a salt thereof, with a compound of the formula: 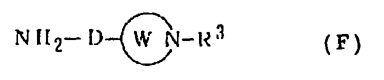 wherein all symbols are of the same meanings as defined above, or a salt thereof,
(5) a process for producing the compound (A) (the compound (A-a)) or a salt thereof which comprises subjecting a compound of the formula: 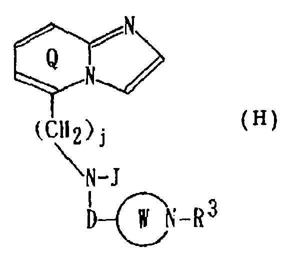 wherein J is a hydrogen atom or a protecting group (e.g. benzyloxycarbonyl, tert-butoxycarbonyl, trifluoroacetyl, trityl, benzyl) and the other symbols are of the same meanings as defined above, or a salt thereof to trichloroacetylation, and then in case of need, deprotection of the protecting group J, and further subjecting the resultant compound to ring-closure reaction,
(6) a process for producing the compound (A) (the compound (A-a)) or a salt thereof which comprises subjecting a compound of the formula: 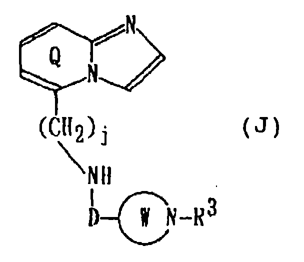 wherein all symbols are of the same meanings as defined above, or a salt thereof to ring-closure reaction by using Mannich reaction, and
(7) a process for producing the compound (A) (the compound wherein j is 0 in the formula (A-a)) or a salt thereof which comprises reacting a compound of the formula: 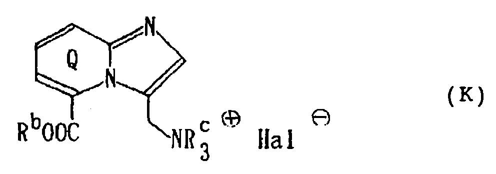 wherein R^{b} and R^{c} are respectively C₁₋₆ alkyl group (e.g. methyl, ethyl, propyl, isopropyl), Hal is a halogen atom (e.g. chlorine, bromine, iodine), ring Q is of the same meaning as defined above, or a salt thereof, with a compound of the formula: 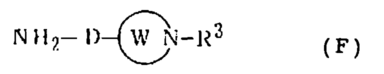 wherein all symbols are of the same meanings as defined above, or a salt thereof.

A method of producing the compound (A) or a salt thereof, or the starting compound for producing it is, more specifically, as follow.

### Method A:

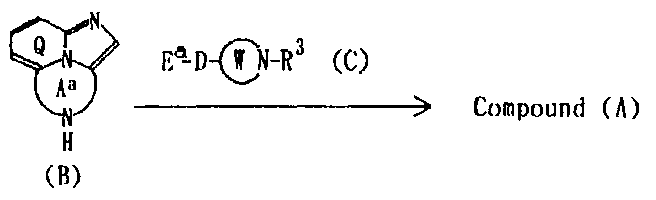

(wherein all symbols are of the same meanings as defined above)

### Method B:

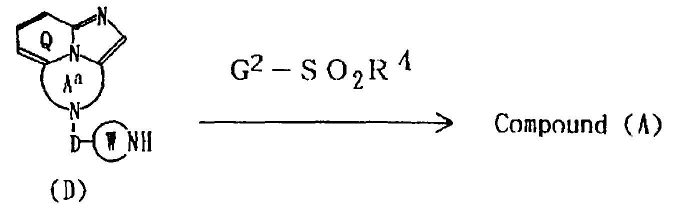

(wherein G² is a halogen atom (e.g. chlorine, bromine, iodine) or R⁴SO₂-O- (R⁴ is as defined above) and the other symbols are of the same meanings as defined above)

### Method C:

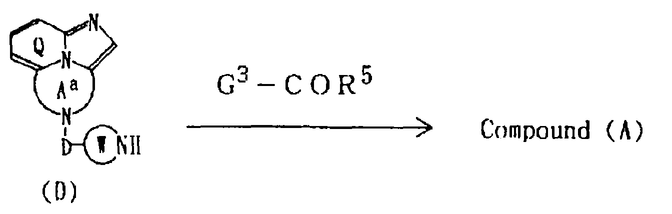

(wherein G³ is a halogen atom (e.g. chlorine, bromine) or R⁵CO-O- (R⁵ is as defined above) and the other symbols are of the same meanings as defined above)

### Method D:

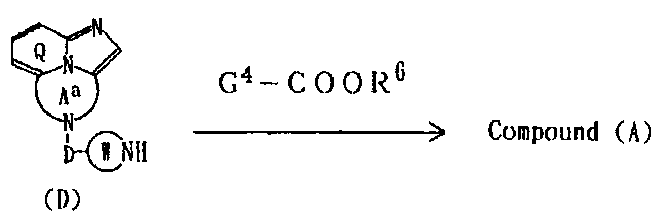

(wherein all symbols are of the same meanings as defined above)

### Method E:

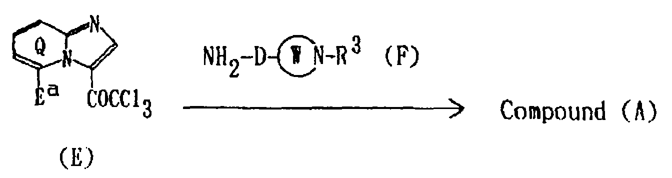

(wherein all symbols are of the same meanings as defined above)

### Method F:

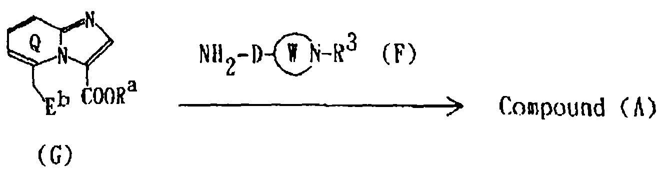

(wherein all symbols are of the same meanings as defined above)

### Method G:

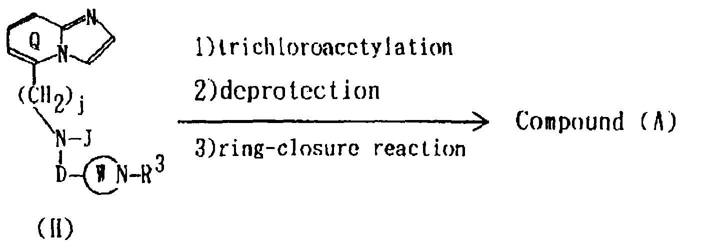

(wherein all symbols are of the same meanings as defined above)

### Method H:

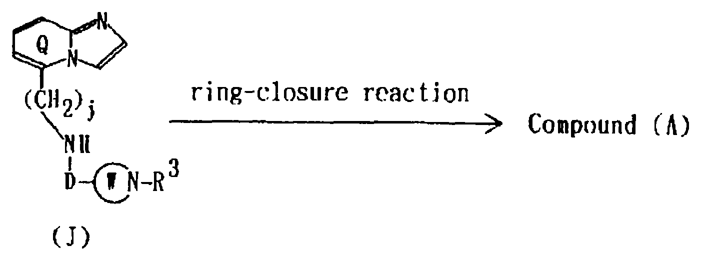

(wherein all symbols are of the same meanings as defined above)

### Method I:

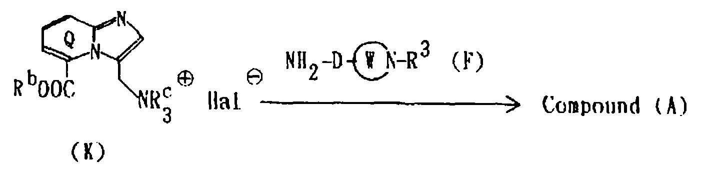

(wherein all symbols are of the same meanings as defined above)

In the reaction between the compound (B) and the compound (C) in the method A for producing the compound (A), one equivalent to a large excess amount (1 to 10 equivalents) of the compound (C) is employed relative to the compound (B). In this case, a basic compound such as sodium hydroxide, potassium hydroxide, sodium hydride, potassium carbonate, triethylamine, diisopropylethylamine 1,4-diazabicyclo[2.2.2]octane and 1,8-diazabicyclo[5.4.0]-7-undecene may be used in an amount of 1 to 10 equivalents. The reaction temperature ranges from -20 to 200°C. Examples of the solvents to be employed include water, lower alcohols (e.g. methanol, ethanol and propanol), ketones (e.g. acetone and methyl ethyl ketone), ethers (e.g. tetrahydrofuran) and aprotic polar solvents (e.g. N,N-dimethylformamide and dimethylsulfoxide). For the said reaction, as a reaction-promoting agent, sodium iodide may be added in an amount ranging from one equivalent to a large excess (1 to 10 equivalents). The reaction time ranges usually from 10 minutes to 24 hours, preferably from 0.5 to 6 hours.

In the reaction between the compound (D) and the compound: G²-SO₂-R⁴ in the method B, one equivalent to a large excess amount (1 to 10 equivalents) of the compound: G²-SO₂-R⁴ is employed relative to the compound (D). In this case, an inorganic base such as potassium carbonate and sodium hydrogencarbonate, or an organic base such as triethylamine, pyridine, dimethylaniline, 1,4-diazabicyclo[2.2.2]octane (DABCO), or 1,8-diazabicyclo[5.4.0]-7-undecene may be used in an amount of 1 to 10 equivalents. The reaction temperature ranges from -30 to 100°C. Examples of the solvents to be employed include halogenated hydrocarbons (e.g. methylene chloride, chloroform and dichloroethane), ethers (e.g. diethylether and tetrahydrofuran), esters (e.g. methyl acetate and ethyl acetate) and aprotic polar solvents (e.g. N,N-dimethylformamide, dimethyl sulfoxide and acetonitrile). The reaction time ranges usually from 10 minutes to 24 hours, preferably from 0.5 to 6 hours.

The reaction between the compound (D) and the compound: G³-CO-R⁵ in the method C is conducted, for example, under conditions similar to those of the reaction between the compound (D) and the compound: G²-SO₂-R⁴ in the method B.

The reaction between the compound (D) and the compound: G⁴-COO-R⁶ in the method D is conducted, for example, under conditions similar to those of the reaction between the compound (D) and the compound: G²-SO₂-R⁴ in the method B.

The reaction between the compound (E) and the compound (F) in the method E is conducted, for example, under conditions similar to those of the reaction between the compound (B) and the compound (C) in the method A.

The reaction between the compound (G) and the compound (F) in the method F is conducted, for example, under conditions similar to those of the reaction between the compound (B) and the compound (C) in the method A.

For the trichloroacetylation of the compound (H) in the method G, trichloroacetyl chloride or anhydrous trichloroacetate is used in an amount ranging from one equivalent to a large excess (1 to 10 equivalents) relative to the compound (H). In this case, 1 to 10 equivalents of an inorganic base (e.g. potassium carbonate and sodium hydrogencarbonate) or an organic base (e.g. 4-N,N-dimethylaminopyridine, triethylamine, pyridine, dimethylaniline and 1,4-diazabicyclo[2.2.2]octane, 1,8-diazacyclo[5.4.0]-7-undene) may optionally be employed. The reaction temperature ranges from 0 to 100°C. Examples of the solvent then employed include halogenated hydrocarbons (e.g. methylene chloride, chloroform and dichloroethane), ethers (e.g. diethyl ether and tetrahydrofuran), esters (e.g. methyl acetate and ethyl acetate) and aprotic polar solvents (e.g. N,N-dimethylformamide, dimethyl sulfoxide and acetonitrile). The reaction time ranges usually from 10 minutes to 100 hours, preferably from 3 to 24 hours. The above-mentioned deprotection reactions of the protective group of secondary amino group represented by J, are all per se known reactions, which can be conducted in according to known conditions. For example, the benzyloxycarbonyl group or the benzyl group as the amino-protecting group can be removed by catalytic reduction (reaction temperatures ranging from room temperature to 100°C) in a solvent (e.g. alcohol, acetic acid, water, tetrahydrofuran and an appropriate mixture of them) in the presence of a catalyst (e.g. palladium carbon or platinum oxide). In the case of trityl group or tert-butoxycarbonyl group, it can be removed by the reaction in a solvent (e.g. water, alcohol, tetrahydrofuran and dioxane) in the presence of an acid (e.g. a mineral acid such as hydrochloric acid, phosphoric acid and sulfuric acid or an organic acid such as toluenesulfonic acid, methansulfonic acid and acetic acid), at temperatures ranging from 0 to 150°C. And, in the case of tert-butoxycarbonyl group, it can be removed by processing with, for example, iodotrimethylsilane in a solvent such as chloroform. Further, trifluoroacetyl group can be easily removed by treating with an alkali (e.g. an aqueous solution of sodium hydroxide or sodium hydrogencarbonate). The ring-closure reaction can be conducted concurrently with the reaction of removing the protecting group. Or, after removing the protecting group, the ring-closure reaction can be conducted by using 1 to 10 equivalents of an inorganic base (e.g. potassium carbonate and sodium hydrogencarbonate) or an organic base (e.g. 4-N,N-dimethylaminopyridine, triethylamine, pyridine, dimethylaniline, 1,4-diazabicyclo[2.2.2]octane and 1,8-diazacyclo[5.4.0]-7-undene). The reaction temperatures ranges from 0 to 100°C. Examples of the solvent then employed include halogenated hydrocarbons (e.g. methylene chloride, chloroform and dichloroethane), ethers (e.g. diethyl ether and tetrahydrofuran), esters (e.g. methyl acetate and ethyl acetate) and aprotic polar solvents (e.g. N,N-dimethylformamide, dimethyl sulfoxide and acetonitrile). The reaction time ranges usually from 10 minutes to 24 hours, preferably from 10 minutes to 6 hours.

For the ring-closure reaction by Mannich reaction using the compound (J) and formalin in the method H, formalin is used in an amount of large excess (2 to 20 equivalents) relative to the compound (J). The reaction temperature ranges from -20 to 150°C. Examples of the solvent used include water, lower alcohols (e.g. methanol, ethanol, propanol and isopropanol) and lower fatty acids (e.g. acetic acid and propionic acid). The reaction time ranges usually from 10 minutes to 24 hours, preferably from 10 minutes to 3 hours.

In the reaction between the compound (K) and the compound (F) in the method I of producing the compound (A), one equivalent to a large excess amount (1 to 10 equivalents) of the compound (F) is employed relative to the compound (K). In this case, sodium hydroxide, potassium hydroxide, sodium hydride, potassium carbonate, triethylamine, diisopropylethylamine, 1,4-diazabicyclo[2.2.2]octane or 1,8-diazabicyclo[5.4.0]-7-undene may be used in an amount of 1 to 10 equivalents. The reaction temperature ranges from -20 to 200°C. Examples of the solvents to be employed include water, lower alcohols (e.g. methanol, ethanol, propanol, isopropanol), ethers (e.g. diethylether, tetrahydrofuran), estels (e.g. methyl acetate, ethyl acetate) and aprotic polar solvents (e.g. N,N-dimethylformamide, dimethylsulfoxide). The reaction time ranges usually from 0.5 to 96 hours, preferably from 6 to 24 hours.

The compound (B) can be synthesized by using the similar method disclosed in WO 96/02542.

The oxo group on ring A^{a} of the compound (B) can be reduced as follow. 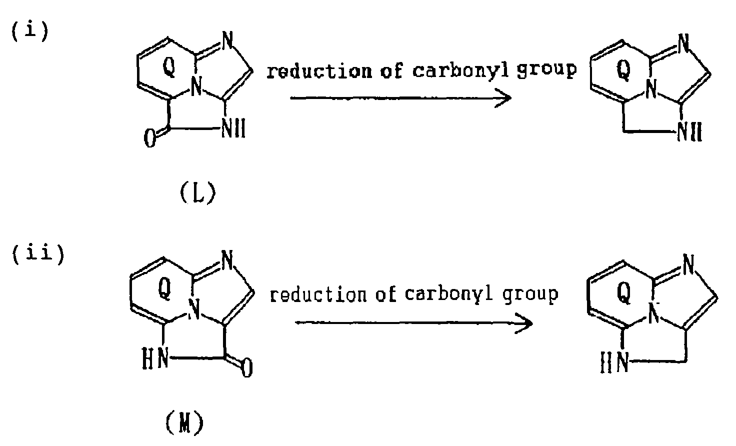 (wherein ring Q is of the same meaning as defined above)

In the reduction of the compounds (L) and (M), one equivalent to a large excess amount (1 to 10 equivalents) of metal hydride complex compound (e.g. sodium borohydride, lithium borohydride, lithium aluminum hydride) or borane complex compound is employed relative to the compounds (L) or (M). In this case, alcohols (e.g. methanol, ethanol) and ethers (e.g. ethylether, tetrahydrofuran, dioxane) may be used as the solvent.

The reaction temperature ranges from -10 to 100°C. The reaction time ranges usually from 10 minutes to 24 hours, preferably from 0.5 to 6 hours.

The compound (D) can be synthesized by following methods and so on. 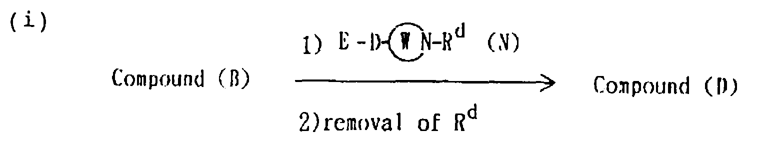 (wherein R^{d} is a hydrogen atom or a protective group of amino group (e.g. benzyloxycarbonyl, tert-butoxycarbonyl, trifluoroacetyl, trityl, benzyl), and the other symboles are of the same meanings as defined above)

The reaction between the compound (B) and the compound (N) is conducted, for example, under conditions similar to those of the reaction between the compound (B) and the compound (C) in the method A. Removal of protective group R^{d} of secondary amino group is a per se known reaction and so on. 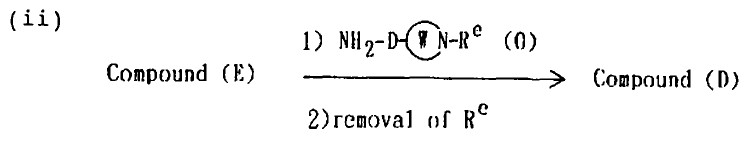 (wherein R^{e} is a hydrogen atom or a protective group of amino group (e.g. benzyloxycarbonyl, tert-butoxycarbonyl, trifluoroacetyl, trityl, benzyl), and the other symbols are of the same meanings as defined above)

The reaction between the compound (E) and the compound (O) is conducted, for example, under conditions similar to those of the reaction between the compound (B) and the compound (C) in the method A. Removal of protective group R^{e} of secondary amino group is a per se known reaction and so on. 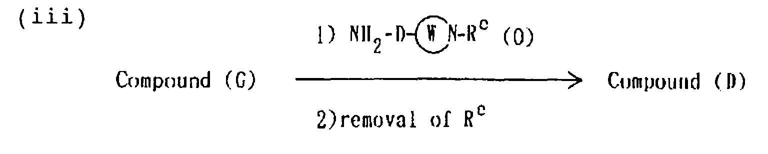 (wherein all symbols are of the same meanings as defined above)

The reaction between the compound (G) and the compound (O) is conducted, for example, under conditions similar to those of the reaction between the compound (B) and the compound (C) in the method A.

Removal of protective group R^{e} of secondary amino group is a per se known reaction and so on. 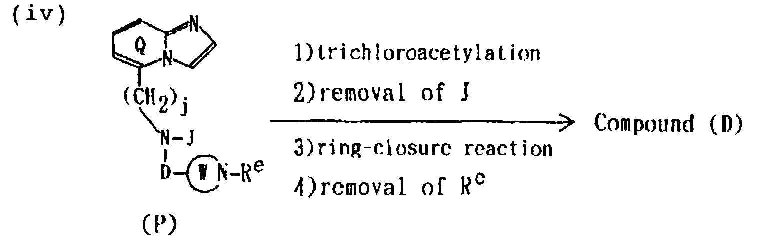 (wherein all symbols are of the same meanings as defined above)

The trichloroacetylation of the compound (P) is conducted, for example, under conditions similar to those of the trichloroacetylation of the compound (H) in the method G. Removal of protective group J of secondary amino group is conducted, for example, under conditions similar to those of the removal of protective group of amino group in the method G.

The ring-closure reaction is conducted, for example, under conditions similar to those of the ring-closure reaction in the method G. Removal of R^{e} is a per se known reaction and so on. 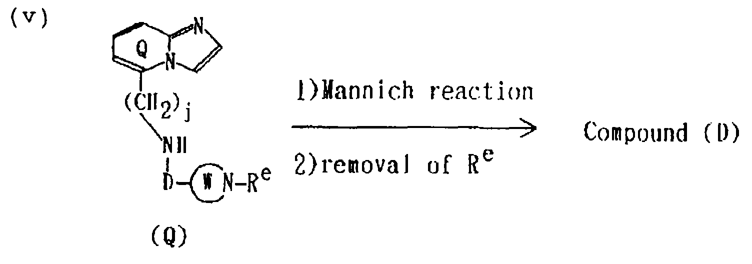 (wherein all symbols are of the same meanings as defined above)

The Mannich reaction of the compound (Q) is conducted, for example, under conditions similar to those of the Mannich reaction of the compound (J) in the method T. Removal of protective group R^{e} of secondary amino group is a per se known reaction and so on. 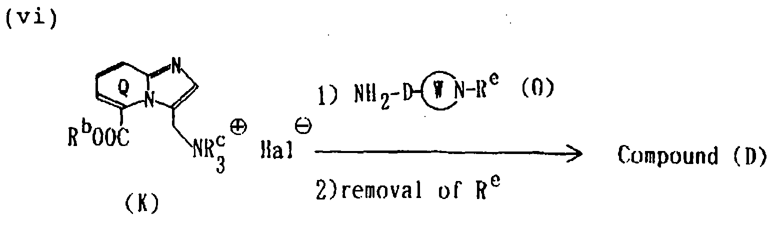 (wherein all symbols are of the same meanings as defined above)

The reaction between the compound (K) and the compound (Q) is conducted, for example, under conditions similar to those of the reaction between the compound (K) and the compound (F) in the method I. Removal of protective group R^{e} of secondary amino group is a per se known reaction and so on.

The compounds (L) and (M) can be synthesized by the similar methods described in WO 96/02542.

The compound (G) can be synthesized be the following method. 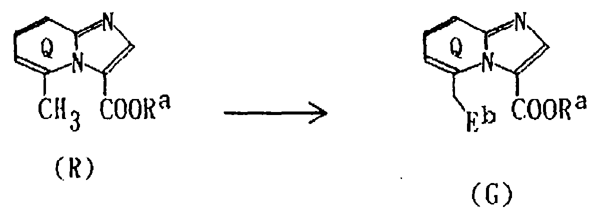 (wherein all symbols are of the same meanings as defined above)

The reaction for introducing E^{b} into the compound (R) is a per se known reaction and so on. For example, chlorine, bromine, tert-butyl hypohalogerite, N-halogenosuccinimide (e.g. N-bromosuccinimide), N-bromocaprolactam, N-bromophthalimide, 1,3-dibromo-5,5-dimethylhydantoin, trichloromethanesulfonyl halide (e.g. trichloromethanesulfonylchloride), tribromomethane and phosphorus pentachloride are used in the halogenation reaction. For the reaction, addition of a peroxide (e.g. benzoyl peroxide) or an optical irradiate for promoting the reaction promotor may be used. The reaction temperature ranges from -20 to 200°C, and the reaction time ranges usually from 0.5 to 6 hours. In this case, solvents, such as aromatic hydrocarbons (e.g. benzene), halogenated hydrocarbons (e.g. methylene chloride, chloroform, dichloroethane), saturated hydrocarbons (e.g. hexane, heptane, cyclohexane), esters (e.g. methyl acetate, ethyl acetate) may be used.

The compound (H) can be synthesized by the following method. 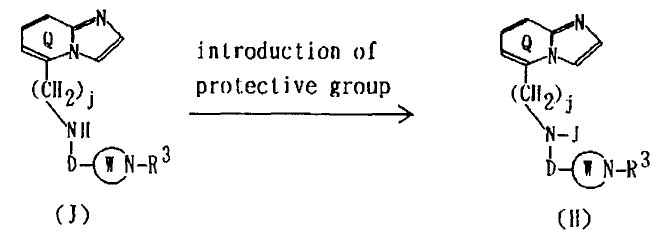 (wherein all symbols are of the same meanings as defined above)

The reaction for introducing protective group J into amino group on the compound (J) is a per se known reaction and so on.

The compound (P) can be synthesized by the following method. 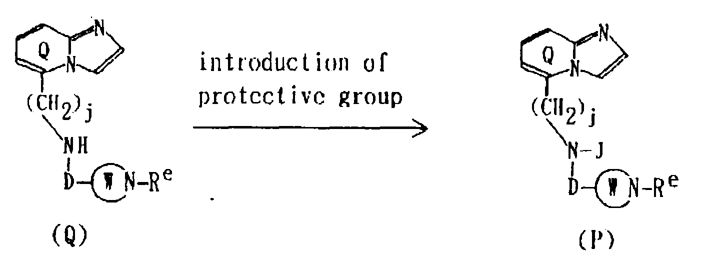 (wherein all symbols are of the same meanings as defined above). The reaction for introducing protective group J into amino group on the compound (Q) is conducted, for example, under conditions similar to those of the reaction for introducing protective group J into amino group on the compound (J). The compound (J) can be synthesized by the following method. 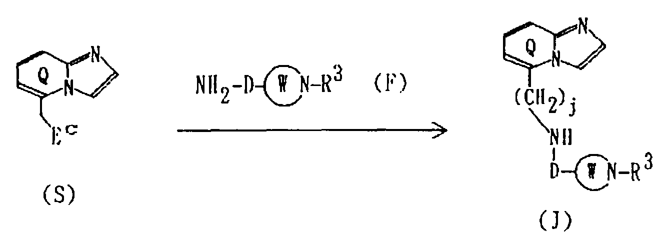 (wherein E^{c} is a leaving group such as a halogen atom (e.g. chlorine, bromine, iodine), a methanesulfonyl group, a p-toluenesulfonyloxy group and the other symbols are of the same meanings as defined above)

The reaction between the compound (S) and the compound (F) is conducted, for example, under conditions similar to those of the reaction between the compound (B) and the compound (C) in the method A.

The compound (Q) can be synthesized by the following method. 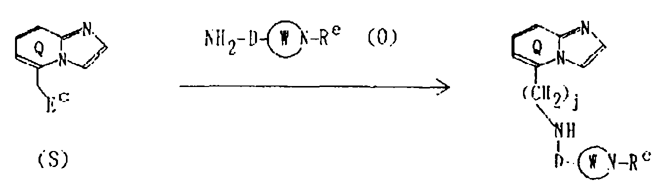 (wherein all symbols are of the same meanings as defined above)

The reaction betwen the compound (S) and the compound (O) is conducted, for example, under conditions similar to those of the reaction betwen the compound (B) and the compound (C) in the method A.

The compound (K) can be synthesized by the following method.

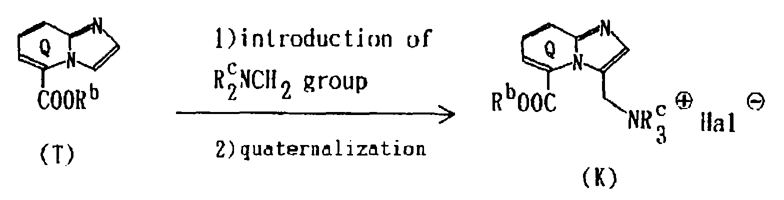

In the dialkylaminomethylation of the compound (T), one equivalent to a large excess amount (1 to 10 equivalents) of N,N-dimethyleneammonium iodide is employed relative to the compound (T). The reaction temperature ranges from 0 to 100°C, and the reaction time ranges usually from 0.5 hour to 24 hours, preferably from 1 to 6 hours. Examples of the solvents to be employed include, for example, halogenated hydrocarbons (e.g. methylene chloride, chloroform, carbon tetrachloride, dichloroethane), ethers (e.g. diethylether, tetrahydrofuran), esters (e.g. methyl acetate, ethyl acetate) and aprotic polar solvents (e.g. N,N-dimethylformamide, dimethylsulfoxide, acetonitrile).

In the quaternalization, one equivalent to a large excess amount (1 to 10 equivalents) of C₁₋₆ alkyl halide such as methyl iodide is employed relative to the dialkylaminomethyl compound. The reaction temperature ranges from 0 to 100°C, and the reaction time ranges usually from 1 to 100 hours, preferably 6 to 24 hours. Examples of the solvents to be employed include, for example, halogenated hydrocarbons (e.g. methylene chloride, chloroform, carbon tetrachloride, dichloroethane), ethers (e.g. diethylether, tetrahydrofuran), estes (e.g. methyl acetate, ethyl acetate) and aprotic polar solvents (e.g. N,N-dimethylformamide, dimethylsulfoxide, acetonitrile).

The compound (S) can be synthesized by the following method. 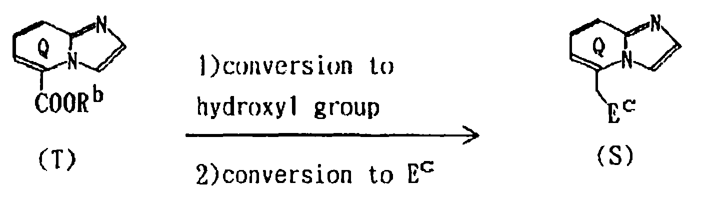 (wherein all symbols are of the same meanings as defined above)

In the reduction of the compound (T), one equivalent to a large excess amount (1 to 10 equivalents) of metal hydride complex compound (e.g. sodium borohydride, lithium borohydride, lithium alminum hydride) or borane complex compound is employed relative to the compound (T). The reaction temperature ranges from -20 to 100°C, and the reaction time ranges usually from 10 minutes to 24 hours, preferably 0.5 to 6 hours. Examples of the solvents to be employed include, for example, alcohols (e.g. methanol, ethanol), ethers (e.g. diethylether, tetrahydrofuran, dioxane).

In the conversion from hydroxyl group to E^{c} (e.g. halogen), 1 to 5 equivalents of halogenated phosphorus (e.g. phosphorus trichloride, phosphorus oxychloride, phosphorus pentachloride, phosphorus tribromide), halogenation agent (e.g. red phosphorus and halogen, thionyl chloride) are employed relative to the alcohol compound.

In the conversion from hydroxyl group to E^{c} (e.g. p-toluenesulfonyloxy, methanesulfonyloxy), 1 to 5 equivalents of p-toluensulfonylchloride or methanesulfonyl chloride are employed relative to the alcohol compound. In this case, an inorganic base such as potassium carbonate, sodium hydrogencarbonate, an organic base such as 4-N,N-dimethylaminopyridine, triethylamine, pyridine, dimethylamiline, 1,4-diazabicyclo[2.2.2]octane, 1,8-diazabicyclo[5.4.0]-7-undecene may be used in an amount of 1 to 10 equivalents. The reaction temperature ranges from 0 to 100°C, and the reaction time ranges usually from 10 minutes to 100 hours, preferably from 3 to 24 hours. Examples of the solvents to be employed include, for example, halogenated hydrocarbons (e.g. methylene chloride, chloroform, dichloroethane), water, ethers (e.g. diethylether, tetrahydrofuran), esters (e.g. methyl acetate, ethyl acetate) and aprotic polar solvents (e.g. N,N-dimethylformamide, dimethylsulfoxide, acetonitrile).

The compound (T) can be synthesized by the following method. 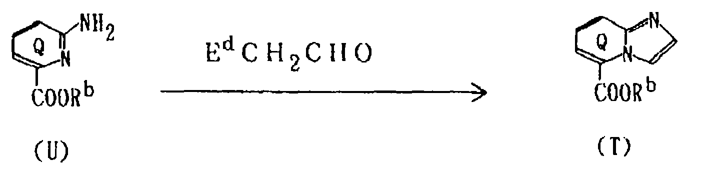 (wherein E^{d} is a leaving group such as a halogen atom (e.g. chlorine, bromine, iodine), the other symbols are of the same meanings as defined above)

In the reaction between the compound (U) and the compound: E^{d}CH₂CHO, one equivalent to a large excess amount (1 to 10 equivalents) of the compound of the compound E^{d}CH₂CHO is employed relative to the compound (U). The reaction temperature ranges from 0 to 200°C, and the reaction time ranges usually from 10 minutes t 24 hours, preferably 0.5 to 6 hours. Examples of the solvents to be employed include, for example, halogenated hydrocarbons (e.g. methylene chloride, chloroform, dichloroethane), water, alcohols (e.g. methanol, ethanol), ethers (e.g. diethylether, tetrahydrofuran, dioxane), esters (e.g. methyl acetate, ethyl acetate) and aprotic polar solvents (e.g. N,N-dimethylformamide, dimethylsulfoxide, acetonitrile).

The compound (E) can be synthesized by the following method. 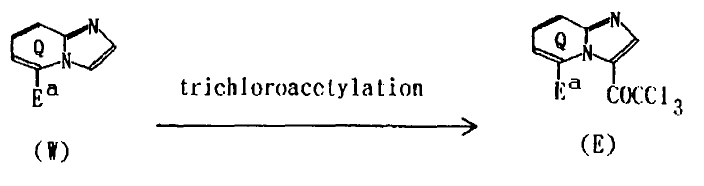 (wherein all symbols are of the same meanings as defined above)

The trichloroacetylation of the compound (W) is conducted, for example, under conditions similar to those of the trichloroacetylation of the compound (H).

The compound (W) can be synthesized by the following method. 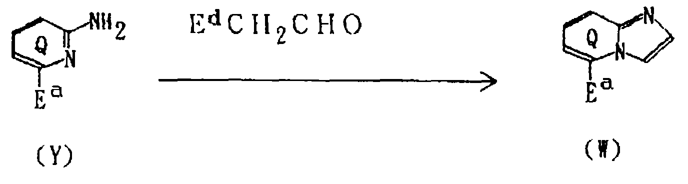 (wherein all symbols are of the same meanings as defined above)

The reaction between the compound (Y) and the compound: E^{d}CH₂CHO is conducted, for example, under conditions similar to those of the reaction between the compound (U) and the compound: E^{d}CH₂CHO.

The starting materials for producing the compound (I') may form a salt. The salt may be employed as similar as the salt of the compound (I').

The intermediate compounds for synthesizing the desired compound (I') or a salt obtained by the above-mentioned methods can be isolated by the following conventional separation means, or reaction mixture per se may optionally be used, as the starting materials for the subsequent step without isolation.

The isolation and purification of the compound (I') from the reaction mixture is conducted according to conventional separation means (for example, extraction, concentration, filtration, recrystallization, column chromatography and thin-layer chromatography).

And, in each of the above-mentioned reactions, when the starting compounds and intermediate compounds has amino group, carboxyl group or hydroxyl group as the substituent, they may have a protective group generally used in the peptide chemistry. After completion of the reaction, the desired compound can be obtained by removing the protective group upon necessity.

Examples of the amino-protecting group include optionally substituted C₁₋₆ alkyl carbonyl (e.g. formyl, methyl carbonyl and ethyl carbonyl), phenyl carbonyl, C₁₋₆ alkyl-oxycarbonyl (e.g. methoxycarbonyl and ethoxycarbonyl), phenyloxycarbonyl (e.g. benzoxycarbonyl), C₇₋₁₀ aralkyloxy-carbonyl (e.g. benzyloxycarbonyl), trityl and phthaloyl. Examples of substituents of them include halogen atoms (e.g. fluoro, chloro, bromo and iodo), C₁₋₆ alkyl-carbonyl (e.g. methylcarbonyl, ethylcarbonyl and butylcarbonyl) and nitro group, and the number of the substituents ranges from about 1 to 3.

Examples of the carboxyl-protecting group include C₁₋₆ alkyl (e.g. methyl, ethyl, n-propyl, i-propyl, n-butyl and tert-butyl), phenyl, trityl and silyl. Examples of substituents of them include halogen atoms (e.g. fluorine, chlorine, bromine and iodine), C₁₋₆ alkylcarbonyl (formyl, methylcarbonyl, ethylcarbonyl and butylcarbonyl) and nitro group, and the number of the substituents ranges from about 1 to 3.

Examples of the hydroxyl-protecting group include for example, optionally substituted C₁₋₆ alkyl (e.g. methyl, ethyl, n-propyl, i-propyl, n-butyl and tert-butyl), phenyl, C₇₋₁₀ aralkyl (e.g. benzyl), C₁₋₆ alkylcarbonyl (e.g. formyl, methylcarbonyl and ethylcarbonyl), phenyloxycarbonyl, C₇₋₁₀ aralkyloxycarbonyl (e.g. benzyloxycarbonyl), pyranyl, furanyl and silyl. As the substituents mentioned above, halogen atoms (e.g. fluoro, chloro, bromo and iodo), C₁₋₆ alkyl, phenyl, C₇₋₁₀ aralkyl and nitro group were used. The number of substituents ranges from about 1 to 4.

And, the protecting groups can be introduced and removed by per se known means or those analogous thereto (for example, I.F.W. McOmie et al., PROTECTIVE GROUPS IN ORGANIC CHEMISTRY, Plenum Press). More specifically, those protecting groups are removed by, for example, acid, base, reduction, ultraviolet ray, hydrazine, phenylhydrazine, sodium N-methyldithiocarbamate, tetrabutylammonium fluoride or palladium acetate.

The compound (I') produced by the above-mentioned methods can be isolated and purified by a conventional separating means such as recrystallization, distillation and chromatography. When the compound (I') thus obtained is in the free form, it can be converted to a salt by per se known means or analogous means thereto (e.g. neutralization). Conversely, when the compound (I') is obtained in the form of a salt, it can be converted to the free form or any other salt by per se known means or analogous means thereto.

Further, when the compound (I') is an optically active compound, it can be resolved into d-isomer and l-isomer by a conventional means for optical resolution.

The compound (I') and the pharmaceutically acceptable salt of the present invention have an excellent inhibiting activity of PDGF action, ameliorating activity of renal diseases and activity of lowering lipid level, and are relatively less toxic. Therefore, these compounds or their salts can be safely used, in mammals (e.g. mouse, rat, hamster, rabbit, cat, dog, cow, horse, sheep, monkey and human), as therapeutic agents for renal diseases [e.g. acute renal failure, diabetic nephropathy, nephritis (e.g. chronic or acute glomerulonephritis, for example, proliferative glomerulonephritis such as mesangial proliferative glomerulonephritis, endocapillary proliferative glomerulonephritis, membranoproliferative glomerulonephritis type I, II, III, crescentic glomerulonephritis, diffuse sclerosing glomerulonephritis)], arteriosclerotic diseases, the other cardiovasular diseases, chronic rheumatoid arthritis, restenosis after PTCA, cancers and hyperlipemia.

While the compound (I') or a salt thereof can be administered as it is, it is usually administered in the form of preparation formulated by a conventional method using carriers or diluents for pharmaceutical preparations adequately selected from excipients (e.g. calcium carbonate, kaolin, sodium hydrogencarbonate, lactose, starch, crystalline cellulose, talc, fine granulated sugar and porous substance), binders (e.g. dextrin, gum, alcoholated starch, gelatin, hydroxypropyl cellulose, hydroxypropyl methyl cellulose and furfuran), disintegrants (e.g. carboxymethyl cellulose calcium, closcarmellose sodium, clospovidone, low-substituted hydroxypropyl cellulose and partial α-starch), lubricants (e.g. magnesium stearate, calcium stearate, talc, starch and sodium benzoate), colorants (e.g. tar pigment, caramel, iron sesquioxide, titanium oxide and riboflavins), flavoring agents (e.g. sweeteners and perfume), stabilizers (e.g. sodium sulfite) and preservatives (e.g. parabens and sorbic acid) in adequate amounts respectively. The therapeutic agent of the present invention containing the above-mentioned pharmaceutical preparation contains the compound (I') or a salt thereof in a amount effective for the therapy and prophylaxis. The content of the compound (I') or a salt thereof in the pharmaceutical preparation of the present invention ranges usually from 0.1 to 100 weight % relative to the whole weight of the pharmaceutical preparation. And, the pharmaceutical preparation of the present invention may contain, as active components, medicinal components other than the compound (I') or a salt thereof. These medicinal components are not specifically restricted so long as the object of this invention is attained, and can be used in adequate ratios. As the said "medicinal components", use is made of, for example, diuretic, angiotensin II receptor antagonist, calcium blocker, ACE inhibitor, Chymase inhibitor, antiphlogistic, HMG-CoA (hydroxymethylglutaryl CoA) reductase inhibitor and squalene synthetase inhibitor. Specific examples of the formulation include tablets (including sugar-coated tablets and film-coated tablets), pills, capsules, granules, powdery preparations, syrups, emulsions, suspensions, injections, inhalants and ointments. These formulations are prepared by a conventional method (e.g. the method described in the Japanese Pharmacopeia).

More specifically, tablets can be prepared by, for example, the following processes:
1) the pharmaceutical preparation as it is, or a homogeneous mixture of the pharmaceutical preparation with an excipient, a binder, a disintegrant or any other suitable additive, is granulated by an adequate means, to which is added, for example, a lubricant, and the whole mixture is subjected to compression molding;
2) the pharmaceutical preparation as it is, or a homogeneous mixture of the pharmaceutical preparation with an excipient, a binder, a disintegrant or any other suitable additive, is directly subjected to compression molding; or
3) the granules prepared in advance as they are, or a homogeneous mixture of the granules with a suitable additive, is subjected to compression molding. And, to this pharmaceutical preparation, a colorant or a flavoring agent may optionally be supplemented upon necessity. Furthermore, this pharmaceutical preparation may optionally be coated with a coating agent.
Capsule preparations can be prepared by, for example filling a homogenous mixture of the pharmaceutical preparation with an excipient or any suitable additive, the granules by an adequate means, or the granules coated with an adequate coating agent, into a capsule.

Injectable preparations can be provided by dissolving, suspending or emulsifying a given amount of the pharmaceutical preparation in, when using an aqueous solvent, e.g. water for injection, physiological saline or Ringer's solution, and, when using a water-insoluble solvent, usually e.g. vegetable oil, or by filling a given amount of the pharmaceutical preparation into a vessel, followed by sealing the vessel.

As the carriers for orally administrable preparations, use is made of substances commonly employed in the field of pharmaceutical preparations, for example, starch, mannitol, crystalline cellulose and sodium carboxymethyl cellulose. As the carriers for injectable preparations, use is made of, for example, distilled water, physiological saline solution, glucose solution and an agent of infusion. Besides, additives generally employed for pharmaceutical preparations can be adequately supplemented.

The pharmaceutical preparations of this invention are relatively less toxic and useful as medicinal preparations, which have PDGF-inhibiting activity, ameliorating activity of renal diseases and lipid lowering activity. Therefore, the pharmaceutical preparations of this invention are useful as medicines for diseases due to these pharmacological actions. The pharmaceutical preparations of this invention can thus be used as the therapy or prophylaxis of, among others, hypertension, acute renal failure, diabetic nephropathy, nephritis, arteriosclerosis, chronic rheumatoid arthritis, cancers and hyperlipemia.

The dose of the pharmaceutical preparations of this invention varies with administration routes, symptoms, the age and body weight of patients and it is preferable that for treating diabetic nephropathy a daily dose of 0.05 to 500 mg/kg, preferably 0.1 to 30 mg/kg, more preferably 5 to 30 mg/kg for oral administration to an adult patient (about weight 60 kg) is given once or divided into several times, for example, 1 to 3 times. The administration route may be either oral or non-oral. Examples of the non-oral include intravenously, intramuscularly, subcutaneously, intrarectally, intravaginally, intraperitoneally, intranasally and sublingually.

Furthermore, the dose of the pharmaceutical preparations of this invention varies with administration routes, symptoms, the age and body weight of patients, it is desirable that, in the case of oral administration to an adult patient (about weight 60 kg) for treating mesangial proliferative glomerulonephritis, a daily dose of 0.01 to 300 mg/kg, preferably 0.5 to 50 mg/kg, more preferably 5 to 30 mg/kg, in terms of the compound (I') or a salt thereof is given once or divided into several times, for example, 1 to 3 times.

The compound (I') or a salt thereof has an activity of inhibiting PDGF, which can be used as a prophylactic and therapeutic agent of diseases including, for example, acute bacterial meningitis, acute myocardial infarction, acute pancreatitis, acute viral encephalitis, adult respiratory distress syndrome, ankylosing spondylitis, bacterial pneumonia, bladder carcinoma, breast cancer, cancer of the uterine cervix, chronic lymphocytic leukemia, chronic mylogenous leukemia, chronic pancreatitis, cancer of the large intestine (carcinoma of the colon/rectum), gastritis, virus A hepatitis, virus B hepatitis, virus C hepatitis H, herpes simplex virus infection, varicellazoster virus infection, Hodgkin's disease, HIV infection, human papilloma virus infection, influenza infection, invasive staphylococcal infection, malignant melanoma, metastasis, multiple myeloma, non-Hodgkin's lymphoma, non-small cell lung cancer, bone arthritis, osteopenia (prophylaxis of osteoporosis), osteoporosis, ovarian cancer, bone Behcet's disease, pain, peptic ulcer, peripheral vascular diseases, prostatic cancer, rheumatoid arthritis, septic shock, systemic fungal infection, small cell lung cancer, gastric cancer, valvular heart disease, restenosis after percutaneous transluminal coronary angiloplasty and arteriosclerosis.

The present invention will be explained in more detail by the following working examples, reference examples, preparation examples and test examples. These are mere examples and are not intended to restrict the present invention in any manner, and may be modified within the range of not deviating the scope of this invention.

In Examples and Reference Examples, abbreviations mean as follows.
NMR : Nuclear magnetic resonance spectrum
DMF : dimethylformamide, DMSO : dimethyl sulfoxide,
DBU: 1,8-diazabicyclo[5,4,0]-7-undecene, Hz : herz, J : coupling constant, m : multiplet, q : quartet, dd : double doublet, t : triplet, d : doublet, s : singlet, br : broad, like : approximate
Room temperature means 10 to 30°C.

### Reference Example 1

### 1,2-Dihydro-1,4,7b-triazacyclopent[cd]inden-2-one·2NaCl

### i) Synthesis of 5-amino-3-carbethoxyimidazo[1,2-a]pyridine

In 1250 ml of ether was suspended 56.0 g (500 mM) of potassium tert-butoxide and while the suspension was stirred vigorously, a solution of 37.0 g (500 mM) of ethyl formate and 61.3 g (500 mM) of ethyl chloroacetate in 100 ml of ether was added dropwise at room temperature over 15 minutes. The reaction mixture was stirred at room temperature for 30 minutes and the precipitate that formed was recovered by filtration. The precipitate was rinsed with 50 ml of ether and dried in vacuo to give 93.13 g of ethyl 2-chloro-2-formylacetate potassium salt as light-yellow solid. In 40 ml of ethanol was suspended 7.59 g (40.0 mM) of this ethyl 2-chloro-2-formylacetate potassium salt as well as 2.18 g (20.0 mM) of 2,6-diaminopyridine and following addition of 2.3 ml (40 mM) of acetic acid, the mixture was refluxed for 3 hours. After completion of the reaction, the precipitate that formed was recovered by filtration and rinsed with 10 ml of ethanol. The filtrate and the washes were pooled and neutralized with aqueous NaHCO₃ solution, and the solvent was distilled off under reduced pressure. The residue was extracted with 100 ml of ethyl acetate and the organic layer was washed with 100 ml of saturated aqueous NaCl solution and dried over MgSO₄. The solvent was distilled off under reduced pressure and the residue was washed with ether to provide 1.65 g of the title compound as light-yellow solid. The washes were concentrated and the residue was purified by silica gel column chromatography (eluent: ethyl acetate) to provide 0.61 g of the title compound (total yield 2.26 g, percent yield 55.1%).
¹H-NMR(200MHz,CDCl₃)δ: 1.42(3H,t,J=7.2Hz), 4.37(2H,q, J=7.2Hz), 6.10(1H,dd,J=7.2, 1.0Hz), 6.84(2H,br s,NH₂), 7.07(1H,dd,J=8.6, 1.2Hz), 7.32(1H,t,J=8.6Hz), 8.39(1H,s).
IR(KBr): 3225, 1689, 1647 cm⁻¹.

### ii) Synthesis of 1,2-dihydro-1,4,7b-triazacyclopent[cd]inden-2-one·2NaCl

To a solution prepared by dissolving 1.44 g (7.0 mM) of 3-carbethoxy-5-aminoimidazo[1,2-a]pyridine in 10 ml of acetonitrile followed by addition of 3.2 ml (14.0 mM) of 25% sodium methoxide-methanol and the mixture was refluxed for 1 hour. After completion of the reaction, 1.15 ml (14.0 mM) of 12N-hydrochloric acid was added to the reaction mixture under ice-cooling and the solvent was thoroughly distilled off under reduced pressure to provide 2.01 g (yield 100%) of a crude product as tan-colored solid.
¹H-NMR(200MHz,DMSO-d₆)δ: 7.05 (1H,d,J=7.4Hz), 7.66 (1H,d,J=8.6Hz), 7.85(1H,dd,J=8.6, 7.4Hz), 8.41(1H,s).
IR(KBr): 3452, 1699, 1668 cm⁻¹.

### Reference Example 2

### 1,2-Dihydro-1,4,7b-triazacyclopent[cd]inden-2-one sodium salt

To a solution prepared by dissolving 51.3 g (25.0 mM) of 5-amino-3-carbethoxyimidazo[1,2-a]pyridine in 500 ml of ethanol was added 114 ml (50.0 mM) of 25% sodium methoxide-methanol and the mixture was refluxed for 3 hours. After completion of the reaction, the precipitate that formed was recovered by filtration, rinsed with 50 ml of ethanol, and dried in vacuo to provide 32.66 g (yield 72.1%) of the title compound as gray powders.
¹H-NMR(200MHz,DMSO-d₆)δ: 6.47(1H,d,J=7.4Hz), 7.02(1H,d, J=8.4Hz), 7.45(1H,dd,J=8.4, 7.4Hz), 7.78(1H,s).

### Reference Example 3

### 3-Carbethoxy-5-chloromethylimidazo[1,2-a]pyridine sulfate

### i) Synthesis of 3-carbethoxy-5-methylimidazo[1,2-a]pyridine

In 10 ml of ether was suspended 560 mg (5.0 mM) of potassium tert-butoxide and while the suspension was vigorously stirred, a solution of ethyl formate:370 mg (5.00 mM) and ethyl chloroformate: 613 mg (5.00 mM) in 10 ml of ether was added dropwise over 3 minutes at room temperature. This mixture was stirred at room temperature for 30 minutes and the precipitate that formed was recovered by filtration and rinsed with a small amount of ether. This precipitate was dried in vacuo to give 700 mg (yield 73.8%) of ethyl 2-chloro-2-formylacetate potassium salt as light-yellow solid. Then, 700 mg (3.69 mM) of ethyl 2-chloro-2-formylacetate potassium salt and 399 mg (3.69 mM) of 6-amino-2-methylpyridine were mixed with 20 ml of ethanol and after addition of 0.53 ml (9.23 mM) of acetic acid, the whole mixture was refluxed for 3 hours. After completion of the reaction, the solvent was distilled off under reduced pressure and the residue was diluted with 20 ml of ethyl acetate and 20 ml of purified water. Then, saturated aqueous NaHCO₃ solution was added until the water layer became pH 8. This mixture was extracted with 40 ml of ethyl acetate and the organic layer was washed with 30 ml of saturated aqueous NaCl solution and dried over MgSO₄. The solvent was then distilled off under reduced pressure and the residue was purified by silica gel column chromatography (eluent: ethyl acetate) to provide 430 mg (yield 57.1%) of the title compound as light-yellow liquid.
¹H-NMR(200MHz,CDCl₃)δ: 1.42(3H,t,J=7.0Hz), 2.82(3H,s), 4.38(2H,q,J=7.0Hz), 6.81(1H,d,J=7.0Hz), 7.36(1H,dd, J=8.8, 7.0Hz), 7.62(1H,d,J=8.8Hz), 8.30(1H,s).

### ii) Synthesis of 3-carboethoxy-5-chloromethylimidazo[1,2-a]pyridine

To a solution prepared by dissolving 430 mg (2.06 mM) of 3-carbethoxy-5-methylimidazo[1,2-a]pyridine in 10 ml of ethyl acetate was added 330 mg (2.67 mM) of N-chlorosuccinimide. Then, 1.03 ml (1.03 mM) of 1N-trifluoroacetic acid-ethyl acetate was added dropwise at room temperature. The mixture was stirred under argon gas at room temperature for 14 hours. After the reaction, the reaction mixture was poured in 30 ml of saturated aqueous NaHCO₃ solution with ice-cooling and the mixture was extracted with 20 ml of ethyl acetate. The organic layer was washed with 30 ml of saturated aqueous NaCl solution and dried over MgSO₄. The solvent was then distilled off under reduced pressure and the residue was purified by silica gel column chromatography (eluent: ethyl acetate) to provide 350 mg (yield 71.2%) of the title compound as yellow liquid.
¹H-NMR(200MHz,CDCl₃)δ: 1.45(3H,t,J=7.2Hz), 4.44(2H,q,J=7.2Hz), 5.44(2H,s), 7.08(1H,d,J=7.0Hz), 7.42(1H,dd,J=8.8, 7.0Hz), 7.80(1H,d,J=8.8Hz), 8.35(1H,s).

### iii) Synthesis of 3-carbethoxy-5-chloromethylimidazo[1,2-a]pyridine sulfate

To a solution prepared by dissolving 43.91 g (215.03 mM) of 3-carbethoxy-5-methylimidazo[1,2-a]pyridine in 200 ml of ethyl acetate was added 31.58 g (236.53 mM) of N-chlorosuccinimide. Then, 1.66 ml (21.50 mM) of trifluoroacetic acid was added dropwise at room temperature. This mixture was stirred under argon gas at room temperature for 14 hours. After completion of the reaction, the reaction mixture was poured in 300 ml of saturated aqueous NaHCO₃ solution with ice-cooling and extracted with 200 ml of ethyl acetate. The organic layer was washed with 300 ml of saturated aqueous NaCl solution and dried over MgSO₄ and the solvent was distilled off under reduced pressure to recover 22.15 g of crude 3-carbethoxy-5-chloromethylimidazo[1,2-a]pyridine as tan-colored liquid. To a solution prepared by dissolving 22.15 g of this crude 3-carbethoxy-5-chloromethylimidazo[1,2-a]pyridine in 200 ml of acetonitrile was added 4.95 ml (92.80 mM) of sulfuric acid with ice-cooling and the mixture was stirred. The purified precipitate was recovered by filtration, rinsed with a small amount of acetonitrile, and dried in vacuo to provide 16.20 g (yield 22.4%) of the title compound as ochre powders.
¹H-NMR(200MHz,DMSO-d₆)δ: 1.38(3H,t,J=7.0Hz), 4.41(2H,q, J=7.0Hz), 5.60(2H,s), 7.62(1H,d,J=7.0Hz), 7.83(1H,dd,J=7.6, 7.0Hz), 8.00(1H,d,7.6Hz), 8.61(1H,s).

### Reference Example 4

### 5-[N-[2-(1-tert-butoxycarbonylpiperidin-4-yl)ethan-1-yl]aminomethyl]imidazo[1,2-a]pyridine

A mixture containing 4.82 g (22.2 mM) of 5-chloromethylimidazo[1,2-a]pyridine hydrochloride, 5.07 g (22.2 mM) of 2-(1-tert-butoxycarbonylpiperidin-4-yl)ethylamine and 9.3 ml (66.6 mM) of triethylamine in 50 ml of ethanol was refluxed for 2 hours. After completion of the reaction, the solvent was distilled off under reduced pressure and the residue was extracted with 100 ml of purified water and 100 ml of chloroform. The organic layer was washed with 100 mM of saturated aqueous NaCl solution and dried over MgSO₄ and the solvent was distilled off under reduced pressure. The residue was purified by silica gel column chromatography (eluent: chloroform-methanol = 10:1) to provide 5.74 g (yield 72.1%) of the title compound as light-yellow liquid.
¹H-NMR(200MHz,CDCl₃)δ: 0.95-1.31(2H,m), 1.31-1.68(5H,m), 1.45(9H,s), 2.55-2.82(4H,m), 3.95-4.18(2H,m), 4.06(2H,s), 6.80(1H,d,J=7.0Hz), 7.18(1H,dd,J=7.0, 9.2Hz), 7.60(1H,d,J=9.2Hz), 7.68(1H,s), 7.75(1H,s).
IR(Neat): 1686, 1425, 1165 cm⁻¹.

### Reference Example 5

### 2-(1-Trifluoromethanesulfonylpiperidin-4-yl)ethylamine hydrochloride

### i) Synthesis of N-2-(1-trifluoromethanesulfonylpiperidin-4-yl)ethylphthalimide

A mixture containing 14.0 g (50.0 mM) of 2-(1-trifluoromethanesulfonylpiperidin-4-yl)ethan-1-ylchloride, 9.26 g (50.0 mM) of phthalimide potassium and 7.49 g (50.0 mM) of sodium iodide was added 100 ml of DMF and the mixture was heated at 100°C for 1 hour. After completion of the reaction, the reaction mixture was poured in iced water and the mixture was extracted with 200 ml of ethyl acetate. The organic layer was washed with 200 ml of purified water 3 times and further with 200 ml of saturated aqueous NaCl solution and dried over MgSO₄. The solvent was then distilled off under reduced pressure to provide 17.01 g (yield 87.1%) of the title compound as light yellow solid.
¹H-NMR(200MHz,CDCl₃)δ: 1.20-1.55(2H,m), 1.55-1.76(3H,m), 1.87-2.02(2H,m), 2.90-3.12(2H,m), 3.75(2H,t,J=7.0Hz), 3.88-4.03(2H,m), 7.68-7.80(2H,m), 7.80-7.92(2H,m).
IR(KBr): 1705, 1385, 1182 cm⁻¹.

### ii) Synthesis of 2-(1-trifluoromethanesulfonylpiperidin-4-yl)ethylamine hydrochloride

A solution prepared by dissolving 17.0 g (43.55 mM) of N-2-(1-trifluoromethanesulfonylpiperidin-4-yl)ethylphthalimide and 6.34 ml (130.64 mM) of hydrazine monohydrate in 200 ml of ethanol was refluxed for 1 hour. After completion of the reaction, the precipitate of phthalhydrazide that formed was collected by filtration and the filtrate was distilled off under reduced pressure. The residue was added 200 ml of purified water and was extracted with 200 ml of chloroform. The organic layer was washed with 200 ml of saturated aqueous NaCl solution and dried over MgSO₄ and the solvent was distilled off under reduced pressure to provide 11.1 g (yield 98.7%) of crude 2-(1-trifluoromethanesulfonylpiperidin-4-yl)ethylamine as light-yellow liquid. A solution prepared by dissolving 11.1 g (42.98 mM) of crude 2-(1-trifluoromethanesulfonylpiperidin-4-yl)ethylamine in 100 ml of ethanol was added 12N hydrochloric acid and the mixture was stirred. The mixture was concentrated under reduced pressure and the precipitate that formed was recovered by filtration and was rinsed with small amounts of ethanol and ether to provide 8.7 g (yield 68.1%) of the title compound as white powders.
¹H-NMR(200MHz,DMSO-d₆)δ: 1.18-1.44(2H,m), 1.68-1.92(5H,m), 2.94-3.17(2H,m), 3.60(2H,t,J=6.4Hz), 3.89-4.05(2H,m).
IR(KBR): 3405, 1385, 1181 cm⁻¹.

### Reference Example 6

### Trans-4-(tert-butoxycarbonylmethyl)amino-1-cyclohexanemethanol

### i) Synthesis of trans-4-(tert-butoxycarbonylmethyl)amino-1-cyclohexanecarboxylic acid

To a suspension of 23.6 g (150 mM) of trans-4-aminomethyl-cyclohexane-1-carboxylic acid in 150 ml of water and 100 ml of THF, was added 20.8 ml (150 mM) of triethylamine and then 32.7 g (150 mM) of di-tert-butyl carbonate dropwise at room temperature. The reaction mixture was stirred at room temperature for two hours. After the reaction, the reaction solution was acidified at pH=1 with 12N hydrochloric acid under cooling and was extracted with 500 ml of ethyl acetate. The organic layer was washed with brine and was dried over MgSO₄. The solvent was distilled off under reduced pressure to give 29.6 g (yield 76.7%) of title compound as white solid.
¹H-NMR(200MHz;DMSO) δ: 0.74-0.98(m,2H), 1.09-1.47(m,12H), 1.61-1.79(m,2H), 1.79-1.98(m,2H), 1.98-2.19(m,1H), 2.76(t,2H,J=6.2Hz), 6.78(t,1H,J=5.4Hz).
IR(KBr): 3375, 1694, 1529 cm⁻¹.

### ii) Synthesis of trans-4-(tert-butoxycarbonylmethyl)amino-1-cyclohexane-methanol

To a solution of 200 ml of 1.0M borane-THF complex solution in THF, was added 25.7 g (100 mM) of trans-4-(tert-butoxycarbonylmethyl)-amino-1-cyclohexanecarboxylic acid portionwise at room temperature. After completion of the addition, the reaction solution was stirred at room temperature for an hour. After the reaction, the reaction solution was poured onto 500 ml of iced-water. The mixture was extracted with 200 ml of ethyl acetate. The organic layer was washed with brine and was dried over MgSO₄. The solvent was distilled off under reduced pressure to afford 21.9 g (yield 90.0%) of title compound as white solid.
¹H-NMR(200MHz;DMSO-d₆) δ: 0.74-0.96(m,4H), 1.14-1.45(m,11H), 1.62-1.81(m,4H), 2.76(t,2H,J-6.2Hz), 3.20(t,2H,J-6.0Hz), 4.31(t,1H,OH,J=6.0Hz), 6.72(t,1H,J=5.4Hz).
IE(KBe): 3376, 1698, 1533 cm⁻¹.

### Example 1

### 1,2-Dihydro-1-(1-tert-butoxycarbonylpiperidin-4-ylmethyl)-1,4,7b-triazacyclopent[cd]inden-2-one

### i) Synthesis of (1-tert-butoxycarbonylpiperidin-4-ylmethyl)methanesulfonate

To a solution prepared by dissolving 4.31 g (20.0 mM) of 1-tert-butoxycarbonyl-4-hydroxymethylpiperidine and 5.54 ml (40.0 mM) of triethylamine in 50 ml of THF was added 1.86 ml (24.0 mM) of methanesulfonyl chloride dropwise at 0°C. After completion of dropwise addition, the reaction mixture was stirred at room temperature for 30 minutes. After completion of the reaction, the reaction mixture was poured in 50 ml of iced water and extracted with 50 ml of ethyl acetate. The organic layer was washed with 50 ml of saturated aqueous NaCl solution and dried over anhydrous sodium sulfate (Na₂SO₄). The solvent was then distilled off under reduced pressure and the residue was dried in vacuo to provide 5.86 g (yield 100%) of the title compound as light-yellow liquid.
¹H-NMR(200MHz,CDCl₃)δ: 1.18-2.32(2H,m), 1.46(9H,s), 1.68-1.82(2H,m), 1.82-2.10(1H,m), 2.61-2.82(2H,m), 3.02(3H,s), 4.07(2H,d,J=6.2Hz), 4.09-4.24(2H,m).

### ii) Synthesis of 1,2-dihydro-1-(1-tert-butoxycarbonylpiperidin-4-ylmethyl)-1,4,7b-triazacyclopent[cd]inden-2-one

A mixture containing 5.68 g (20.0 mM) of (1-tert-butoxycarbonylpiperidin-4-ylmethyl) methanesulfonate, 4.35 g (20.0 mM) of 1,2-dihydro-1,4,7b-triazacyclopent[cd]inden-2-one·2NaCl, and 3.58 ml (24.0 mM) of DBU in 50 ml of DMF was heated at 80°C for 2 hours. After completion of the reaction, the reaction mixture was poured in 50 ml of iced water and extracted with 100 ml of ethyl acetate. The organic layer was washed with 50 ml of purified water twice and, then, with 50 ml of saturated aqueous NaCl solution and dried over Na₂SO₄. The solvent was then distilled off under reduced pressure and the residue was purified by silica gel column chromatography (eluent: ethyl acetateethanol = 10:1) to provide 3.00 g (yield 42.1%) of the title compound as a brown amorphous substance.
¹H-NMR(200MHz,CDCl₃)δ: 1.16-2.40(2H,m), 1.45(9H,s), 1.62-1.81(2H,m), 2.01-2.24(1H,m), 2.57-2.79(2H,m), 3.97(2H,d,J=7.4Hz), 4.05-4.29(2H,m), 6.86(1H,d,J=7.4Hz), 7.66(1H,d,J=8.8Hz), 7.88(1H,dd,J=8.8, 7.4Hz), 8.37(1H,s).

### Example 2

### 1,2-Dihydro-1-[2-(1-tert-butoxycarbonylpiperidin-4-yl)ethan-1-yl]-1,4,7b-triazacyclopent[cd]inden-2-one

To a solution prepared by dissolving 8.26 g (36.0 mM) of 2-(1-tert-butoxycarbonylpiperidin-4-yl)ethanol and 10.0 ml (72.0 mM) of triethylamine in 50 ml of ether was added 3.34 ml (43.2 mM) of methanesulfonyl chloride dropwise at 0°C. After completion of dropwise addition, the reaction mixture was stirred at room temperature for 30 minutes. After completion of the reaction, the reaction mixture was poured in 100 ml of iced water and the mixture was extracted with 100 ml of ethyl acetate. The organic layer was washed with 100 ml of saturated aqueous NaCl solution and dried over anhydrous magnesium sulfate (MgSO₄) and the solvent was distilled off under reduced pressure to recover the mesylate as solid. To a solution prepared by dissolving this mesylate and 10.37 g (36.0 mM) of 1,2-dihydro-1,4,7b-triazacyclopent[cd]inden-2-one·2NaCl in 25 ml of DMF was added 6.46 ml (43.2 mM) of DBU and the mixture was heated at 80°C for 2 hours. After completion of the reaction, the reaction mixture was poured in iced water and the mixture was extracted with 200 ml of ethyl acetate. The organic layer was washed with 200 ml of purified water 3 times and further with 200 ml of saturated aqueous NaCl solution and dried over MgSO₄. The solvent was then distilled off under reduced pressure and the residue was purified by silica gel column chromatography (eluent: ethyl acetate) to provide 9.10 g (yield 68.2%) of the title compound as light-yellow solid.
¹H-NMR(200MHz,CDCl₃)δ: 1.08-1.34(2H,m), 1.46(9H,s), 1.42-1.63(1H,m), 1.72-1.91(4H,m), 2.57-2.83(2H,m), 3.98-4.24(4H,m), 6.87(1H,d,J=7.4Hz), 7.66(1H,d,J=8.6Hz), 7.79(1H,dd,J=8.6, 7.4Hz), 8.36(1H,s).
IR(KBr): 1704, 1685, 1624, 1431 cm⁻¹.

### Example 3

### 1,2-Dihydro-1-[2-(1-(tert-butoxycarbonyl)piperidin-4-yl)ethan-1-yl]-1,4,7b-triazacyclopent[cd]inden-2-one

To a solution prepared by dissolving 5.73 g (25.0 mM) of 2-[(1-tert-butoxycarbonyl)piperidin-4-yl]ethanol and 7.0 ml (50.0 mM) of triethylamine in 50 ml of ether was added 2.3 ml (30.0 mM) of methanesulfonyl chloride dropwise at 0°C. After completion of dropwise addition, the reaction mixture was stirred at room temperature for 30 minutes. After completion of the reaction, the reaction mixture was poured in 100 ml of iced water and the mixture was extracted with 100 ml of ethyl acetate. The organic layer was washed with 100 ml of saturated aqueous NaCl solution and dried over MgSO₄ and the solvent was distilled off under reduced pressure to recover 6.88 g of the mesylate as solid. To 50 ml of DMF was added 6.88 g (22.38 mM) of this mesylate as well as 4.46 g (24.62 mM) of 1,2-dihydro-1,4,7b-triazacyclopent[cd]inden-2-one sodium salt and the mixture was heated at 100°C for 1 hour. After completion of this reaction, the reaction mixture was poured in iced water and the mixture was extracted with 200 ml of ethyl acetate. The organic layer was washed with 200 ml of purified water 3 times and further with 200 ml of saturated aqueous NaCl solution and dried over MgSO₄. The solvent was then distilled off under reduced pressure and the residue was rinsed with ether to provide 5.10 g (yield 55.9%) of the title compound as white solid.
¹H-NMR(200MHz,CDCl₃)δ: 1.08-1.34(2H,m), 1.46(9H,s), 1.42-1.63(1H,m), 1.72-1.91(4H,m), 2.57-2.83(2H,m), 3.98-4.24(4H,m), 6.87(1H,d,J=7.4Hz), 7.66(1H,d,J=8.6Hz), 7.79(1H,d,J=8.6, 7.4Hz), 8.36(1H,s).
IR(KBr): 1704, 1685, 1624, 1431 cm⁻¹.

### Example 4

### 1,2-Dihydro-1-[3-(1-tert-butoxycarbonylpiperidin-4-yl)propan-1-yl]-1,4,7b-triazacyclopent[cd]inden-2-one

To a solution prepared by dissolving 4.87 g (20.0 mM) of 1-tert-butoxycarbonyl-4-(3-hydroxypropan-1-yl)piperidine and 5.54 ml (40.0 mM) of triethylamine in 50 ml of THF was added 1.86 ml (24.0 mM) of methanesulfonyl chloride at 0°C. After completion of dropwise addition, the reaction mixture was stirred at room temperature for 30 minutes. After completion of this reaction, the reaction mixture was poured in 50 ml of iced water and extracted with 50 ml of ethyl acetate. The organic layer was washed with 50 ml of saturated NaCl solution and dried over Na₂SO₄. The solvent was then distilled off under reduced pressure and the residue was dried in vacuo to provide 6.42 g of yellow liquid. To 50 ml of DMF was added 6.42 g (20.0 mM) of 3-(1-tert-butoxycarbonylpiperidin-4-yl)propan-1-yl methanesulfonate thus obtained as well as 4.35 g (20.0 mM) of 1,2-dihydro-1,4,7b-triazacyclopent[cd]inden-2-one·2NaCl and 3.58 ml (24.0 mM) of DBU and the mixture was heated at 80°C for 2 hours. After completion of the reaction, the reaction mixture was poured in 50 ml of iced water and extracted with 100 ml of ethyl acetate. The organic layer was washed with 50 ml of purified water twice and further with 50 ml of saturated aqueous NaCl solution and dried over Na₂SO₄. The solvent was then distilled off under reduced pressure and the residue was purified by silica gel column chromatography (eluent: ethyl acetateethanol = 10:1) to provide 3.82 g (yield 49.7%) of the title compound as brown solid.
¹H-NMR(200MHz,CDCl₃)δ: 0.98-1.20(2H,m), 1.44(9H,s), 1.32-1.53(2H,m), 1.57-1.72(2H,m), 1.79-1.98(3H,m), 2.56-2.75(2H,m), 3.98-4.17(4H,m), 6.87(1H,d,J=7.2Hz), 7.65(1H,d,J=8.6Hz), 7.78(1H,dd,J=8.6, 7.2Hz), 8.36(1H,s).

### Example 5

### 4,5-Dihydro-4-(1-tert-butoxycarbonylpiperidin-4-ylmethyl)-3H-1,4,8b-triazaacenaphthylen-3-one

A solution prepared by dissolving 5.78 g (24.22 mM) of 3-carbethoxy-5-chloromethylimidazo[1,2-a]pyridine, 7.78 g (36.33 mM) of 1-tert-butoxycarbonyl-4-aminomethylpiperidine, and 6.75 ml (48.44 mM) of triethylamine in 100 ml of ethanol was refluxed under argon gas for 3 hours. After completion of the reaction, the solvent was distilled off under reduced pressure and the residue was extracted with 100 ml of chloroform. The organic layer was washed with saturated aqueous NaCl solution and dried over MgSO₄ and the solvent was distilled off under reduced pressure. The residue was purified by silica gel column chromatography (eluent: chloroform-methanol = 20:1) to provide 6.50 g (yield 72.4%) of the title compound as yellow liquid.
¹H-NMR(200MHz,CDCl₃)δ: 1.30-1.56(2H,m), 1.43(9H,s), 1.79-1.95(2H,m), 1.96-2.15(1H,m), 2.58-2.82(2H,m), 3.53-3.71(2H,m), 4.00-4.22(2H,m), 5.01(2H,s), 6.72(1H,d,J=6.7Hz), 7.36(1H,dd,J=9.2, 6.7Hz), 7.54(1H,d,J=9.2Hz), 8.18(1H,s).

### Example 6

### 4,5-Dihydro-4-[2-(1-tert-butoxycarbonylpiperidin-4-yl)ethan-1-yl]-3H-1,4,8b-triazaacenaphthylen-3-one

In 50 ml of acetonitrile was suspended 6.73 g (20.0 mM) of 3-carbethoxy-5-chloromethylimidazo[1,2-a]pyridine sulfate followed by addition of 5.97 ml (40.0 mM) of DBU at room temperature with stirring. To the resulting solution was added 4.57 g (20.0 mM) of 2-(1-tert-butoxycarbonylpiperidin-4-yl)-1-ethylamine as well as 5.54 ml (40.0 mM) of triethylamine and 3.00 g (20.0 mM) of sodium iodide and the mixture was stirred at room temperature for 16 hours. After completion of the reaction, the solvent was distilled off under reduced pressure and the residue was extracted with 100 ml of 2-butanone. The organic layer was washed with 100 ml of saturated aqueous NaCl solution and dried over MgSO₄ and the solvent was distilled off under reduced pressure. The residue was purified by silica gel column chromatography (eluent: ethyl acetateethanol = 5:1) to provide 2.88 g (yield 37.5%) of the title compound as yellow oil.
¹H-NMR(200MHz,CDCl₃)δ: 1.04-1.29(2H,m), 1.45(9H,s), 1.52-1.78(3H,m), 1.78-1.84(2H,m), 2.58-2.82(2H,m), 3.54-3.72(2H,m), 3.98-4.20(2H,m), 5.00(2H,s), 6.74(1H,d,J=6.6Hz), 7.35(1H,dd,J=9.2, 6.6Hz), 7.53(1H,d,J=9.2Hz), 8.17(1H,s).

### Example 7

### 4,5-Dihydro-4-[3-(1-tert-butoxycarbonylpiperidin-4-yl)propan-1-yl]-3H-1,4,8b-triazaacenaphthylen-3-one

A solution prepared by dissolving 3.59 g (10.66 mM) of 3-carbethoxy-5-chloromethylimidazo[1,2-a]pyridine sulfate, 3.10 g (12.79 mM) of 3-(1-tert-butoxycarbonylpiperidin-4-yl)-1-propylamine, and 5.94 ml (42.64 mM) of triethylamine in 30 ml of ethanol was refluxed for 5 hours. After completion of the reaction, the solvent was distilled off under reduced pressure and the residue was extracted with 100 ml of chloroform. The organic layer was washed with 100 ml of saturated aqueous NaCl solution and dried over MgSO₄ and the solvent was distilled off under reduced pressure. The residue was purified by silica gel column chromatography (eluent: chloroform-methanol = 50:1) to provide 2.55 g (yield 62.1%) of the title compound as yellow oil.
¹H-NMR(200MHz,CDCl₃)δ: 0.97-1.24(2H,m), 1.25-1.39(3H,m), 1.45(9H,s), 1.57-1.81(4H,m), 2.52-2.78(2H,m), 3.57(2H,t,J=7.4Hz), 3.96-4.18(2H,m), 5.01(2H,s), 6.74(1H,d,J=6.0Hz), 7.33(1H,dd,J=9.2, 7.0Hz), 7.59(1H,d,J=9.2Hz), 8.18(1H,s).
IR(Neat): 1678, 1533, 1161 cm⁻¹.

### Example 8

### 1,2-Dihydro-1-(1-trifluoromethanesulfonylpiperidin-4-ylmethyl)-1,4,7b-triazacyclopent[cd]inden-2-one

### i) Synthesis of 1,2-dihydro-1-(piperidin-4-ylmethyl)-1,4,7b-triazacyclopent[cd]inden-2-one dihydrochloride

To a solution of 3.00 g (8.42 mM) of 1,2-dihydro-1-(1-tert-butoxycarbonylpiperidin-4-ylmethyl)-1,4,7b-triazacyclopent[cd]inden-2-one in 50 ml of ethanol was added 10 ml (122 mM) of 12N-hydrochloric acid and the mixture was stirred at room temperature for 1 hour. After completion of the reaction, the reaction mixture was concentrated under reduced pressure and the precipitate that formed was recovered by filtration. This precipitate was rinsed with small amounts of ethanol and ether to provide 2.30 g (yield 83.0%) of the title compound as brown solid.
¹H-NMR(200MHz,DMSO-d₆)δ: 1.42-1.67(2H,m), 1.76-1.95(2H,m), 2.05-2.29(1H,m), 2.66-2.93(2H,m), 3.14-3.34(2H,m), 4.04(2H,d,J=7.2Hz), 7.63(1H,d,J=7.6Hz), 7.87(1H,d,J=8.8Hz), 8.20(1H,dd,J=8.8, 7.6Hz), 8.92(1H,s).

### ii) Synthesis of 1,2-dihydro-1-(1-trifluoromethanesulfonylpiperidin-4-ylmethyl)-1,4,7b-triazacyclopent[cd]inden-2-one

In 20 ml of acetonitrile was suspended 988 mg (3.0 mM) of 1,2-dihydro-1-(piperidin-4-ylmethyl)-1,4,7b-triazacyclopent[cd]inden-2-one dihydrochloride followed by addition of 0.9 ml (6.0 mM) of DBU at room temperature and thorough mixing. To the resulting solution was added 0.83 ml (6.0 mM) of triethylamine, followed by addition of 1.29 g (3.6 mM) of N-phenyltrifluoromethanesulfonimide, and the mixture was stirred at room temperature for 16 hours. After completion of the reaction, the solvent was distilled off under reduced pressure and the residue was extracted with 100 ml of ethyl acetate. The organic layer was washed with 100 ml of saturated aqueous NaCl solution and dried over MgSO₄ and the solvent was then distilled off under reduced pressure. The residue was purified by silica gel column chromatography (eluent: ethyl acetate-ethanol = 10:1) to provide 890 mg (yield 76.4%) of the title compound as light-yellow crystals.
¹H-NMR(200MHz,CDCl₃)δ: 1.39-1.64(2H,m), 1.80-1.96(2H,m), 2.08-2.35(1H,m), 2.92-3.13(2H,m), 3.91-4.04(2H,m), 4.01(2H,d,J=7.0Hz), 6.86(1H,d,J=8.6Hz), 7.80(1H,dd,J=8.6, 6.8Hz), 8.38(1H,s).
IR(KBr): 1698, 1507, 1383, 1227 cm⁻¹.

| Elemental Analysis for C₁₅H₁₅N₄O₃SF₃: | | | |
|---|---|---|---|
| Calcd.: | C, 46.39; | H, 3.89; | N, 14.43. |
| Found : | C, 46.37; | H, 3.66; | N, 14.19. |

### Example 9

### 1,2-Dihydro-1-[2-(1-trifluoromethanesulfonylpiperidin-4-yl)ethan-1-yl]-1,4,7b-triazacyclopent[cd]inden-2-one hydrochloride

### i) Synthesis of 1,2-dihydro-1-[2-(piperidin-4-yl)ethan-1-yl]-1,4,7b-triazacyclopent[cd]inden-2-one dihydrochloride

To a solution prepared by dissolving 5.30 g (14.13 mM) of 1,2-dihydro-1-[2-(1-tert-butoxycarbonylpiperidin-4-yl)ethan-1-yl]-1,4,7b-triazacyclopent[cd]inden-2-one in 50 ml of ethanol was added 5.88 ml (71.53 mM) of 12N-hydrochloric acid and the mixture was stirred at room temperature for 1 hour. After completion of the reaction, the reaction mixture was concentrated under reduced pressure and the crystals that formed were collected by filtration and rinsed with small amounts of ethanol and ether to provide 3.51 g (yield of 71.5%) of the title compound as white crystals.
¹H-NMR(200MHz,DMSO-d₆)δ: 1.32-1.68(3H,m), 1.68-1.84(2H,m), 1.84-2.02(2H,m), 2.64-2.93(2H,m), 3.15-3.33(2H,m), 4.14(2H,t,J=7.6Hz), 7.62(1H,d,J=7.6Hz), 7.86(1H,d,J=8.6Hz), 8.18(1H,dd,J=8.6, 7.6Hz), 8.90(1H,m), 8.82-9.22(2H,br s,NH₂).
IR(KBr): 1714, 1645, 1549 cm⁻¹.

### ii) Synthesis of 1,2-dihydro-1-[2-(1-trifluoromethanesulfonylpiperidin-4-yl)ethan-1-yl]-1,4,7b-triazacyclopent[cd]inden-2-one

In 30 ml of acetonitrile was suspended 1.72 g (5.0 mM) of 1,2-dihydro-1-[2-(piperidin-4-yl)ethan-1-yl]-1,4,7b-triazacyclopent[cd]inden-2-one dihydrochloride, followed by addition of 1.5 ml (10.0 mM) of DBU and 1.4 ml (10.0 mM) of triethylamine. Then, 8.93 g (25.0 mM) of N-phenyltrifluoromethanesulfonimide was added and the mixture was stirred at room temperature for 16 hours. After completion of the reaction, the solvent was distilled off under reduced pressure and the residue was extracted with 100 ml of chloroform. The organic layer was washed with 100 ml of saturated aqueous NaCl solution and dried over MgSO₄ and the solvent was distilled off under reduced pressure. The residue was purified by silica gel column chromatography (eluent: ethyl acetate-ethanol = 10:1) to provide 1.54 g (yield 76.3%) of the title compound as white solid. Recrystallization of the solid (1.54 g) from 10 ml of ethanol gave 1.23 g (recovery rate 80.0%) of prisms.
¹H-NMR(200MHz,CDCl₃)δ: 1.24-1.67(3H,m), 1.78-2.06(4H,m), 2.92-3.13(2H,m), 3.88-4.04(2H,m), 4.16(2H,t,J=7.0Hz), 6.87(1H,d,J=7.0Hz), 7.68(1H,d,J=8.6Hz), 7.80(1H,dd,J=8.6, 7.0Hz), 8.37(1H,s).
IR(KBr): 1689, 1624, 1504 cm⁻¹.

| Elemental Analysis for C₁₆H₁₇N₄O₃SF₃: | | | |
|---|---|---|---|
| Calcd.: | C, 47.76; | H, 4.26; | N, 13.92. |
| Found : | C, 47.57; | H, 4.23; | N, 13.96. |

### iii) Synthesis of 1,2-dihydro-1-[2-(1-trifluoromethanesulfonyl)piperidin-4-yl)ethan-1-yl]-1,4,7b-triazacyclopent[cd]inden-2-one hydrochloride

To a solution prepared by dissolving 402 mg (1.0 mM) of 1,2-dihydro-1-[2-(1-trifluoromethanesulfonylpiperidin-4-yl)ethan-1-yl]-1,4,7b-triazacyclopent[cd]inden-2-one in 10 ml of 2-propanol was added 0.16 ml (2.0 mM) of 12N-hydrochloric acid and the mixture was stirred and concentrated under reduced pressure. The resulting precipitate was recovered by filtration and rinsed with a small amount of ether to provide 378 mg (yield 86.2%) of the title compound as white crystals.
¹H-NMR(200MHz,DMSO-d₆)δ: 1.12-1.38(2H,m), 1.48-1.79(1H,m), 1.79-1.84(2H,m), 1.84-2.02(2H,m), 2.98-3.23(2H,m), 3.69-3.88(2H,m), 4.06-4.22(2H,m), 7.61(1H,d,J=7.6Hz), 7.87(1H,d,J=8.6Hz), 8.18(1H,dd,J=8.6, 7.6Hz), 8.90(2H,s).

### Example 10

### 1,2-Dihydro-1-[3-(1-trifluoromethanesulfonylpiperidin-4-yl)propan-1-yl]-1,4,7b-triazacyclopent[cd]inden-2-one

### i) Synthesis of 1,2-dihydro-1-[3-(piperidin-4-ylmethyl)propan-1-yl]-1,4,7b-triazacyclopent[cd]inden-2-one dihydrochloride

To a solution prepared by dissolving 3.82 g (9.94 mM) of 1,2-dihydro-1-[3-(1-tert-butoxycarbonylpiperidin-4-yl)propan-1-yl]-1,4,7b-triazacyclopent[cd]inden-2-one in 50 ml of ethanol was added 10 ml (122 mM) of 12N-hydrochloric acid and the mixture was stirred at room temperature for 1 hour. After completion of the reaction, the reaction mixture was concentrated under reduced pressure and the resulting precipitate was recovered by filtration. The precipitate was rinsed with small amounts of ethanol and ether to provide 2.99 g (yield 84.5%) of the title compound as brown solid.
¹H-NMR(200MHz,DMSO-d₆)δ: 1.18-1.44(4H,m), 1.44-1.66(1H,m), 1.66-1.91(4H,m), 2.67-2.92(2H,m), 3.09-3.29(2H,m), 4.10(2H,t,J=7.0Hz), 7.68(1H,d,J=7.8Hz), 7.89(1H,d,J=8.8Hz), 8.24(1H,dd,J=8.8, 7.8Hz), 9.00(1H,s), 8.87-9.26(2H,m).

### ii) Synthesis of 1,2-dihydro-1-[3-(1-trifluoromethanesulfonylpiperidin-4-yl)propan-1-yl]-1,4,7b-triazacyclopent[cd]inden-2-one

In 20 ml of acetonitrile was suspended 1072 mg (3.0 mM) of 1,2-dihydro-1-[3-(piperidin-4-yl)propan-1-yl]-1,4,7b-triazacyclopent[cd]inden-2-one dihydrochloride followed by addition of 0.9 ml (6.0 mM) of DBU at room temperature and thorough mixing. To the resulting solution was added 0.83 ml (6.0 mM) of triethylamine. Then, 1.29 g (3.6 mM) of N-phenyltrifluoromethanesulfonimide was added and the mixture was stirred at room temperature for 16 hours. After completion of the reaction, the solvent was distilled off under reduced pressure and the residue was extracted with 100 ml of ethyl acetate. The organic layer was washed with 100 ml of saturated aqueous NaCl solution and dried over MgSO₄ and the solvent was distilled off under reduced pressure. The residue was purified by silica gel column chromatography (eluent: ethyl acetate-ethanol = 10:1) to provide 870 mg (yield 69.7%) of the title compound as light-brown crystals.
¹H-NMR(200MHz,CDCl₃)δ: 1.25-1.64(5H,m), 1.64-2.02(4H,m), 2.90-3.11(2H,m), 3.84-4.02(2H,m), 4.09(t, 2H, J=7.4 Hz), 6.86 (d, 1H, J=7.0 Hz), 7.66 (1H,d,J=8.8Hz), 7.78(1H,dd,J=8.8, 7.0Hz), 8.36(1H,s).
IR(KBr): 1701, 1507, 1383, 1228 cm⁻¹.

| Elemental Analysis for C₁₈H₁₉N₄O₃SF₃: | | | |
|---|---|---|---|
| Calcd.: | C, 49.03; | H, 4.60; | N, 13.45. |
| Found : | C, 49.12; | H, 4.49; | N, 13.43. |

### Example 11

### 4,5-Dihydro-4-(1-trifluoromethanesulfonylpiperidin-4-ylmethyl)-3H-1,4,8b-triazaacenaphthylen-3-one hydrochloride

### i) Synthesis of 4,5-dihydro-4-(piperidin-4-ylmethyl)-3H-1,4,8b-triazaacenaphthylen-3-one dihydrochloride

To a solution prepared by dissolving 3.71 g (10.0 mM) of 4,5-dihydro-4-(1-tert-butoxycarbonylpiperidin-4-ylmethyl)-3H-1,4,8b-triazaacenaphthylen-3-one in 50 ml of ethanol was added 4.11 ml of 12N-hydrochloric acid at room temperature. This mixture was stirred at room temperature for 1 hour and the resulting precipitate was recovered by filtration. The precipitate was rinsed with a small amount of ether and dried in vacuo to provide 3.24 g (yield 94.2%) of the title compound as white powders.
¹H-NMR(200MHz,DMSO-d₆)δ: 1.24-1.42(2H,m), 1.76-1.92(2H,m), 1.94-2.20(1H,m), 2.64-2.90(2H,m), 3.11-3.30(2H,m), 3.46(2H,d,J=7.2Hz), 5.25(2H,s), 7.42(1H,d,J=7.0Hz), 7.88(1H,d,J=8.7Hz), 8.01(1H,dd,J=8.7, 7.0Hz), 8.65(1H,s), 8.95-9.33(2H,m).

### ii) Synthesis of 4,5-dihydro-4-(1-trifluoromethanesulfonylpiperidin-4-ylmethyl)-3H-1,4,8b-triazaacenaphthylen-3-one

In 20 ml of THF was suspended 2.06 g (6.0 mM) of 4,5-dihydro-4-(piperidin-4-ylmethyl)-3H-1,4,8b-triazaacenaphthylen-3-one dihydrochloride and 3.35 ml (24.0 mM) of triethylamine and 1.51 ml (9.0 mM) of trifluoromethanesulfonic anhydride were added in that order at 0°C. The mixture was stirred at room temperature for 1 hour and the solvent was then distilled off under reduced pressure. The residue was extracted with 100 ml of chloroform and the organic layer was washed with 100 ml of saturated aqueous NaCl solution and dried over MgSO₄. The solvent was then distilled off under reduced pressure and the residue was purified by silica gel column chromatography (eluent: ethyl acetate-ethanol = 10:1) to provide 1.71 g (yield 71.0%) of the title compound as light-yellow amorphous substance.
¹H-NMR(200MHz,CDCl₃)δ: 1.32-1.58(2H,m), 1.78-1.96(2H,m), 1.96-2.17(1H,m), 2.93-3.17(2H,m), 3.51(2H,d,J=7.4Hz), 3.88-4.07(2H,m), 5.03(2H,s), 6.77(1H,d,J=7.0Hz), 7.36(1H,dd,J=8.8, 7.0Hz), 7.56(1H,d,J=8.8Hz), 8.19(1H,s).

### iii) Synthesis of 4,5-dihydro-4-(1-trifluoromethanesulfonylpiperidin-4-ylmethyl)-3H-1,4,8b-triazaacenaphthylen-3-one hydrochloride

To a solution prepared by dissolving 580 mg (1.44 mM) of 4,5-dihydro-4-(1-trifluoromethanesulfonylpiperidin-4-ylmethyl)-3H-1,4,8b-triazaacenaphthylen-3-one in 10 ml of ethanol was added 0.24 ml (2.88 mM) of 12N-hydrochloric acid with stirring. The resulting solution was concentrated under reduced pressure and the precipitate was recovered by filtration. The precipitate was rinsed with a small amount of ether and dried in vacuo to provide 480 mg (yield 80.0%) of the title compound as white solid.
¹H-NMR(200MHz,DMSO-d₆)δ: 1.24-1.41(2H,m), 1.75-1.94(2H,m), 1.96-2.22(2H,m), 3.04-3.26(2H,m), 3.48(2H,d,J=7.4Hz), 3.73-3.91(2H,m), 5.25(2H,s), 7.38(1H,d,J=7.0Hz), 7.83(1H,d,J=8.6Hz), 7.95(1H,dd,J=8.6, 7.0Hz), 8.59(1H,s).

### Example 12

### 4,5-Dihydro-4-[2-(1-trifluoromethanesulfonylpiperidin-4-yl)ethan-1-yl]-3H-1,4,8b-triazaacenaphthylen-3-one

### i) Synthesis of 4,5-dihydro-4-[2-(piperidin-4-yl)ethan-1-yl]-3H-1,4,8b-triazaacenaphthylen-3-one dihydrochloride

To a solution prepared by dissolving 2.88 g (7.49 mM) of 4,5-dihydro-4-[2-(1-tert-butoxycarbonylpiperidin-4-yl)ethan-1-yl]-3H-1,4,8b-triazaacenaphthylen-3-one in 30 ml of ethanol was added 6.25 ml (74.9 mM) of 12N-hydrochloric acid and the mixture was stirred at room temperature for 1 hour. After completion of the reaction, the reaction mixture was concentrated under reduced pressure and the precipitate was recovered by filtration. The precipitate was rinsed with small amounts of ethanol and ether to provide 2.13 g (yield 79.8%) of the title compound as light-yellow crystals.
¹H-NMR(200MHz,DMSO-d₆)δ: 1.29-1.76(5H,m), 1.78-1.98(2H,m), 2.64-2.91(2H,m), 3.12-3.31(2H,m), 3.47-3.66(2H,m), 5.27(2H,s), 7.43(1H,d,J=7.0Hz), 7.86(1H,d,J=8.8Hz), 8.00(1H,dd,J=8.8, 7.0Hz), 8.64(1H,s), 8.93-9.31(2H,m).

### ii) Synthesis of 4,5-dihydro-4-[2-(1-trifluoromethanesulfonylpiperidin-4-yl)ethan-1-yl]-3H-1,4,8b-triazaacenaphthylen-3-one

In 15 ml of acetonitrile was suspended 713 mg (2.0 mM) of 4,5-dihydro-4-[2-(piperidin-4-yl)ethan-1-yl]-3H-1,4,8b-triazaacenaphthylen-3-one hydrochloride followed by addition of 0.6 ml (4.0 mM) of DBU at room temperature with stirring. To the resulting solution was added 0.83 ml (6.0 mM) of triethylamine as well as 0.37 ml (2.2 mM) of trifluoromethanesulfonic anhydride at 0°C and the mixture was stirred at room temperature for 1 hour. After completion of the reaction, the solvent was distilled off under reduced pressure and the residue was extracted with 50 ml of 2-butanone. The organic layer was washed with 50 ml of saturated aqueous NaCl solution and dried over MgSO₄ and the solvent was distilled off under reduced pressure. The residue was purified by silica gel column chromatography (eluent: ethyl acetate-ethanol = 9:1) to provide 434 mg (yield 35.0%) of the title compound as light-yellow amorphous substance.
¹H-NMR(200MHz,CDCl₃)δ: 1.26-1.48(2H,m), 1.48-1.75(3H,m), 1.88-2.04(2H,m), 2.84-3.02(2H,m), 3.65(2H,t,J=6.8Hz), 3.88-4.04(2H,m), 5.04(2H,s), 6.81(1H,d,J=6.2Hz), 7.38(1H,dd,J=9.2, 6.2Hz), 7.54(1H,d,J=9.2Hz), 8.14(1H,s).
IR(KBr): 1732, 1709, 1633, 1227 cm⁻¹.

### Example 13

### 4,5-Dihydro-4-[3-(1-trifluoromethanesulfonylpiperidin-4-yl)propan-1-yl]-3H-1,4,8b-triazaacenaphthylen-3-one hydrochloride

### i) Synthesis of 4,5-dihydro-4-[3-(piperidin-4-yl)propan-1-yl]-3H-1,4,8b-triazaacenaphthylen-3-one dihydrochloride

To a solution prepared by dissolving 2.12 g (5.51 mM) of 4,5-dihydro-4-[3-(1-tert-butoxycarbonylpiperidin-4-yl)propan-1-yl]-3H-1,4,8b-triazaacenaphthylen-3-one in 30 ml of ethanol was added 1.40 ml of 12N-hydrochloric acid and the mixture was stirred at room temperature for 1 hour. After completion of the reaction, the solvent was distilled off under reduced pressure. To the residue was added 5 ml of ethanol and 5 ml of ether and the resulting precipitate was recovered by filtration. The precipitate was rinsed with small amounts of ethanol and ether to provide 1.87 g (yield 91.2%) of the title compound as light-yellow crystals.
¹H-NMR(200MHz,DMSO-d₆)δ: 1.17-1.51(4H,m), 1.51-1.72(3H,m), 1.71-1.89(2H,m), 2.67-2.94(2H,m), 3.09-3.31(2H,m), 3.36-3.61(2H,m), 5.29(2H,s), 7.47(1H,d,J=6.4Hz), 7.89(1H,d,J=9.0Hz), 8.02(1H,d,J=7.4Hz), 8.67(1H,s), 8.82-9.28(2H,m,NH). IR(KBr): 1653, 1599, 1443 cm⁻¹.

### ii) Synthesis of 4,5-dihydro-4-[3-(1-trifluoromethanesulfonylpiperidin-4-yl)propan-1-yl]-3H-1,4,8b-triazaacenaphthylen-3-one

In 20 ml of acetonitrile was suspended 1.37 g (3.69 mM) of 4,5-dihydro-4-[3-(piperidin-4-yl)propan-1-yl]-3H-1,4,8b-triazaacenaphthylen-3-one hydrochloride followed by addition of 1.1 ml (7.38 mM) of DBU at room temperature with stirring. To the resulting solution was added 1.0 ml (7.38 mM) of triethylamine as well as 6.59 g (18.45 mM) of N-phenyltrifluoromethanesulfonimide and the mixture was stirred at room temperature for 16 hour. After completion of the reaction, the solvent was distilled off under reduced pressure and the residue was extracted with 100 ml of chloroform. The organic layer was washed with 100 ml of saturated aqueous NaCl solution and dried over MgSO₄ and the solvent was distilled off under reduced pressure. The residue was purified by silica gel column chromatography (eluent: chloroform-methanol = 50:1) to provide 1.08 g (yield 68.3%) of the title compound as light-yellow solid.
¹H-NMR(200MHz,CDCl₃)δ: 1.16-1.61(4H,m), 1.61-1.91(5H,m), 2.92-3.11(2H,m), 3.58(2H,t,J=7.6Hz), 3.87-4.03(2H,m), 5.01(2H,s), 6.75(1H,d,J=7.0Hz), 7.36(1H,dd,J=9.0, 7.0Hz), 7.54(1H,d,J=9.0Hz), 8.18(1H,s).
IR(KBr): 1647, 1543, 1387, 1182 cm⁻¹.

### iii) Synthesis of 4,5-dihydro-4-[3-(1-trifluoromethanesulfonylpiperidin-4-yl)propan-1-yl]-3H-1,4,8b-triazaacenaphthylene-3-one hydrochloride

To a solution prepared by dissolving 300 mg (0.70 mM) of 4,5-dihydro-4-[3-(1-trifluoromethanesulfonylpiperidin-4-yl)propan-1-yl]-3H-1,4,8b-triazaacenaphthylen-3-one in 10 ml of ethanol was added 0.12 ml (1.4 mM) of 12N hydrochloric acid at room temperature. After stirring, the solvent was distilled off under reduced pressure and 10 ml of 2-propanol was added to the residue. The precipitate that formed was recovered by filtration and rinsed with a small amount of ether to provide 265 mg (yield 81.1%) of the title compound as light-yellow solid.
¹H-NMR(200MHz,DMSO-d₆)δ: 1.07-1.41(4H,m), 1.43-1.74(3H,m), 1.74-1.89(2H,m), 3.04-3.25(2H,m), 3.40-3.91(4H,m), 5.25(2H,s), 7.40(1H,d,J=7.2Hz), 7.83(1H,d,J=9.0Hz), 7.96(1H,dd,J=9.0, 7.2Hz), 8.60(1H,s).
IR(KBr): 1666, 1552, 1375, 1184 cm⁻¹.
m.p. 172-174°C

| Elemental Analysis for C₁₈H₂₂N₄O₃SClF₃·H₂O: | | | |
|---|---|---|---|
| Calcd.: | C, 44.58; | H, 4.99; | N, 11.55. |
| Found : | C, 44.31; | H, 4.65; | N, 11.17. |

### Example 14

### 4,5-Dihydro-4-(1-trifluoroacetylpiperidin-4-ylmethyl)-3H-1,4,8b-triazaacenaphthylen-3-one hydrochloride

### i) Synthesis of 4,5-dihydro-4-(1-trifluoroacetylpiperidin-4-ylmethyl)-3H-1,4,8b-triazaacenaphthylen-3-one

In 20 ml of acetonitrile was suspended 687 mg (2.0 mM) of 4,5-dihydro-4-(piperidin-4-ylmethyl)-3H-1,4,8b-triazaacenaphthylen-3-one dihydrochloride followed by addition of 2.23 ml (16.0 mM) of triethylamine and 1.41 ml (10.0 mM) of trifluoroacetic anhydride in the order mentioned. This mixture was stirred at room temperature for 1 hour and the solvent was then distilled off under reduced pressure. The residue was extracted with 50 ml of chloroform and the organic layer was washed with 50 ml of saturated aqueous NaCl solution and dried over MgSO₄. The solvent was then distilled off under reduced pressure and the residue was purified by silica gel column chromatography (eluent: ethyl acetate-ethanol = 10:1) to provide 540 mg (yield 73.7%) of the title compound as light-yellow amorphous substance.
¹H-NMR(200MHz,CDCl₃)δ: 1.13-1.40(2H,m), 1.82-2.00(2H,m), 2.05-2.26(2H,m), 2.66-2.85(1H,m), 3.02-3.24(1H,m), 3.30-3.42(1H,m), 3.54-3.68(1H,m), 4.00-4.11(1H,m), 4.45-4.60(1H,m), 5.00(1H,s), 6.75(1H,d,J=6.8Hz), 7.35(1H,dd,J=8.8, 6.8Hz), 7.57(1H,d,J=8.8Hz), 8.20(1H,s).

### ii) Synthesis of 4,5-dihydro-4-(1-trifluoroacetylpiperidin-4-ylmethyl)-3H-1,4,8b-triazaacenaphthylen-3-one hydrochloride

To a solution prepared by dissolving 540 mg (1.47 mM) of 4,5-dihydro-4-(1-trifluoroacetylpiperidin-4-yl-methyl)-3H-1,4,8b-triazaacenaphthylen-3-one in 10 ml of ethanol was added 0.24 ml (2.94 mM) of 12N-hydrochloric acid with stirring. The resulting solution was concentrated under reduced pressure and the precipitate that formed was recovered by filtration. The precipitate was rinsed with a small amount of ether and dried in vacuo to provide 330 mg (yield 55.7%) of the title compound as white solid.
¹H-NMR(200MHz,DMSO-d₆)δ: 1.26-1.42(2H,m), 1.72-1.91(2H,m), 1.93-2.20(1H,m), 3.03-3.26(2H,m), 3.46(2H,d,J=7.0Hz), 3.73-3.88(2H,m), 5.22(2H,s), 7.38(1H,d,J=6.8Hz), 7.86(1H,d,J=8.8Hz), 7.95(1H,dd,J=8.8, 6.8Hz), 8.61(1H,s).

### Example 15

### 4,5-Dihydro-4-[2-(1-trifluoroacetylpiperidin-4-yl)ethan-1-yl]-3H-1,4,8b-triazaacenaphthylen-3-one

In 15 ml of acetonitrile was suspended 713 mg (2.0 mM) of 4-[2-(piperidin-4-yl)ethan-1-yl]-4,5-dihydro-3H-1,4,8b-triazaacenaphthylen-3-one hydrochloride followed by addition of 0.6 ml (4.0 mM) of DBU at room temperature with stirring. To the resulting solution was added 0.83 ml (6.0 mM) of triethylamine as well as 0.31 ml (2.2 mM) of trifluoroacetic anhydride at 0°C and the mixture was stirred at room temperature for 1 hour. After completion of the reaction, the solvent was distilled off under reduced pressure and the residue was extracted with 50 ml of 2-butanone. The organic layer was washed with 50 ml of saturated aqueous NaCl solution and dried over MgSO₄ and the solvent was distilled off under reduced pressure. The residue was purified by silica gel column chromatography (eluent: ethyl acetate-ethanol = 9:1) to provide 455 mg (yield 59.8%) of the title compound as light yellow solid.
¹H-NMR(200MHz,CDCl₃)δ: 1.13-1.41(2H,m), 1.56-1.71(3H,m), 1.85-2.07(2H,m), 2.66-2.85(1H,m), 3.01-3.22(1H,m), 3.65(2H,t,J=7.0Hz), 3.93-4.12(1H,m), 4.46-4.62(1H,m), 5.01(2H,s), 6.76(1H,d,J=7.0Hz), 7.34(1H,dd,J=7.0, 9.2Hz), 7.56(1H,d,J=9.2Hz), 8.19(1H,s).
IR(KBr): 1683, 1657, 1180, 1146 cm⁻¹.

### Example 16

### 4,5-Dihydro-4-(1-pentafluoropropionylpiperidin-4-ylmethyl)-3H-1,4,8b-triazaacenaphthylen-3-one hydrochloride

### i) Synthesis of 4,5-dihydro-4-(1-pentafluoropropionylpiperidin-4-ylmethyl)-3H-1,4,8b-triazaacenaphthylen-3-one

In 10 ml of acetonitrile was suspended 687 mg (2.0 mM) of 4,5-dihydro-4-(piperidin-4-ylmethyl)-3H-1,4,8b-triazaacenaphthylen-3-one dihydrochloride, followed by addition of 2.23 ml (16.0 mM) of triethylamine and 1.97 ml (10.0 mM) of pentafluoropropionic anhydride in the order mentioned. This mixture was stirred at room temperature for 1 hour and the solvent was then distilled off under reduced pressure. The residue was extracted with 50 ml of chloroform and the organic layer was washed with 50 ml of saturated aqueous NaCl solution and dried over MgSO₄. The solvent was distilled off under reduced pressure and the residue was purified by silica gel column chromatography (eluent: ethyl acetate-ethanol = 10:1) to provide 526 mg (yield 63.2%) of the title compound as light-yellow amorphous substance.
¹H-NMR(200MHz,CDCl₃)δ: 1.22-1.53(2H,m), 1.80-1.97(2H,m), 2.05-2.27(1H,m), 2.71-2.89(1H,m), 3.06-3.23(1H,m), 3.31-3.46(1H,m), 3.52-3.68(1H,m), 4.05-4.23(1H,m), 4.46-4.62(1H,m), 5.04(2H,s), 6.77(1H,d,J=6.8Hz), 7.36(1H,dd,J=9.2, 6.8Hz), 7.57(1H,d,J=9.2Hz), 8.20(1H,s).

### ii) Synthesis of 4,5-dihydro-4-(1-pentafluoropropionylpiperidin-4-ylmethyl)-3H-1,4,8b-triazaacenaphthylen-3-one hydrochloride

To a solution of 526 mg (1.26 mM) of 4,5-dihydro-4-(1-pentafluoropropionylpiperidin-3-4-ylmethyl)-3H-1,4,8b-triazaacenaphthylen-3-one in 10 ml of ethanol was added 0.20 ml (2.52 mM) of 12N-hydrochloric acid with stirring. The resulting solution was concentrated under reduced pressure and the precipitate that formed was recovered by filtration. This precipitate was rinsed with a small amount of ether and dried in vacuo to provide 430 mg (yield 75.4%) of the title compound as white solid.
¹H-NMR(200MHz,DMSO-d₆)δ: 1.23-1.40(2H,m), 1.73-1.93(2H,m), 1.95-2.22(1H,m), 3.02-3.25(2H,m), 3.46(2H,d,J=7.2Hz), 3.72-3.89(2H,m), 5.24(2H,s), 7.36(1H,d,J=7.0Hz), 7.85(1H,d,J=8.8Hz), 7.96(1H,dd,J=8.8, 7.0Hz), 8.60(1H,s).

### Example 17

### 4,5-Dihydro-4-[2-(1-tert-butoxycarbonylpiperidin-4-yl)ethan-1-yl]-3H-1,4,8b-triazaacenaphthylene

A solution prepared by dissolving 4.85 g (13.5 mM) of 5-[N-[2-(1-tert-butoxycarbonylpiperidin-4-yl)ethan-1-yl]aminomethyl]imidazo[1,2-a]pyridine in 20 ml of acetic acid was added 20.27 ml (270.6 mM) of 37% formaldehyde solution and the reaction mixture was heated at 60°C for 30 minutes. After completion of the reaction, the solvent and the excess of formaldehyde solution was distilled off under reduced pressure and the residue was dissolved in 100 ml of purified water. The solution was 2N aqueous NaOH solution until the solution became pH10 with ice-cooling and was extracted with 100 ml of ethyl acetate. The organic layer was washed 100 ml of saturated aqueous NaCl solution and dried over MgSO₄ and the solvent was distilled off under reduced pressure. The residue was purified by silica gel column chromatography (chloroform: methanol = 10:1) to provide 4.00 g (yield 79.8%) of the title compound as light-yellow liquid.
¹H-NMR(200MHz,CDCl₃)δ: 0.99-1.28(2H,m), 1.45(9H,s), 1.47-1.74(5H,m), 2.46-2.78(4H,m), 3.96(2H,s), 3.98-4.10(2H,m), 4.12(2H,s), 6.53(1H,d,J=7.0Hz), 7.10(1H,dd,J=9.2, 6.6Hz), 7.39(1H,s), 7.44(1H,d,J=8.8Hz).
IR(Neat): 1687, 1460, 1169 cm⁻¹.

### Example 18

### 4,5-Dihydro-4-[2-(1-trifluoromethanesulfonylpiperidin-4-yl)ethan-1-yl]-3H-1,4,8b-triazanaphthylene dihydrochloride

### i) Synthesis of 4,5-dihydro-4-[2-(piperidin-4-yl)ethan-1-yl]-3H-1,4,8-b-triazanaphthylene trihydrochloride

A solution prepared by dissolving 3.85 g(10.4 mM) of 4,5-dihydro-4-[2-(1-tert-butoxycarbonylpiperidin-4-yl)ethan-1-yl]-3H-1,4,8b-triazaacenaphthylene in 50 ml of ethanol was added 17.1 ml (207.8 mM) of 12N hydrochloric acid and the mixture was stirred at room temperature for 1 hour. After completion of the reaction, the reaction mixture was distilled off under reduced pressure and the crystals that found were collected by filtration. The crystal was rinsed with small amounts of ethanol and ether to provide 3.63 g (yield 92.1%) of the title compound as white crystal.
¹H-NMR(200MHz,D₂O)δ: 1.28-1.55(2H,m), 1.55-1.76(3H,m), 1.87-2.03(2H,m), 2.62-2.77(2H,m), 2.87-3.07(2H,m), 3.32-3.49(2H,m), 4.30(4H,s), 7.29(1H,d,J=7.6Hz), 7.73(1H,s), 7.79-7.93(2H,m).
IR(KBR): 1637, 1512, 1443 cm⁻¹.

### ii) Synthesis of 4,5-dihydro-4-[2-(trifluoromethanesulfonylpiperidin-4-yl)ethan-1-yl]-3H-1,4,8b-triazaacenaphthylene

In 20 ml of acetonitrile was suspended 1.52 g (4.0 mM) of 4,5-dihydro-4-[2-(piperidin-4-yl)ethan-1-yl]-3H-1,4,8b-triazaacenaphthylene trihydrochloride followed by addition of 1.8 ml (12.0 mM) of DBU and 1.2 ml (8.0 mM) of triethylamine at room temperature with stirring to provide uniform solution. The solution was added 2.86 g (8.0 mM) of N-phenyltrifluoromethanesulfonimide and the reaction mixture was stirred at room temperature for 16 hours. After completion of the reaction, the solvent was distilled off under reduced pressure and the residue was added 100 ml of purified water and, further, was extracted with 100 ml of ethyl acetate. The organic layer was washed with 100 ml of saturated aqueous NaCl solution and dried over MgSO₄ and the solvent was distilled off under reduced pressure. The residue was purified by silica gel column chromatography (eluent: chloroform-methanol = 10:1) to provide 1.40 g (yield 86.8%) of the title compound as brown liquid.
¹H-NMR(200MHz,CDCl₃)δ: 1.18-1.47(2H,m), 1.47-1.72(3H,m), 1.72-1.97(2H,m), 2.54(2H,t,J=7.0Hz), 2.89-3.13(2H,m), 3.84-4.92(2H,m), 3.96(2H,s), 4.12(2H,s), 6.53(1H,d,J=6.6Hz), 7.11(1H,dd,J=9.2, 6.8Hz), 7.39(1H,s), 7.45(1H,d,J=9.2Hz).
IR(Neat): 1387, 1227, 1186 cm⁻¹.

### iii) Synthesis of 4,5-dihydro-4-[2-(trifluoromethanesulfonylpiperidin-4-yl)ethan-1-yl]-3H-1,4,8b-triazaacenaphthylene dihydrochloride

A solution prepared by dissolving 1.35 g (3.35 mM) of 4,5-dihydro-4-[2-(trifluoromethanesulfonylpiperidin-4-yl)ethan-1-yl]-3H-1,4,8b-triazaacenaphthylene in 10 ml of ethanol was added 0.83 ml (10.1 mM) of 12N hydrochloric acid and the mixture was stirred. The solution was concentrated under reduced pressure and the crystals that formed were collected by filtration. The crystals were rinsed with small amounts of ethanol and ether to provide 1.24 g (yield 77.9%) of the title compound as white crystal.
¹H-NMR(200MHz,D₂O)δ: 1.23-1.52(2H,m), 1.56-2.03(5H,m), 3.08-3.41(4H,m), 3.82-4.02(2H,m), 4.78(2H,s), 4.79(2H,s), 7.76(1H,d,J=6.6Hz), 7.88-8.12(3H,m).
IR(KBR): 1660, 1375, 1172 cm⁻¹.
m.p. 173-175°C

| Elemental Analysis for C₁₇H₂₃ N₄O₂SCl₂F₃·0.5H₂O: | | | |
|---|---|---|---|
| Calcd.: | C, 42.16; | H, 4.99; | N, 11.57. |
| Found : | C, 42.39; | H, 4.80; | N, 11.85. |

### Example 19

### 4,5-Dihydro-4-[2-(1-trifluoromethanesulfonylpiperidin-4-yl)ethan-1-yl]-3H-1,4,8b-triazaacenaphthylen-5-one

In 20 ml of acetonitrile was added 389 mg (1.0 mM) of 5-ethoxycarbonyl-3-trimethylammoniomethylimidazo[1,2-a]pyridine iodide, 295 mg (1.0 mM) of 2-(1-trifluoromethanesulfonylpiperidin-4-yl)ethylamine hydrochloride, 0.15 ml (1.0 mM) of DBU and 0.28 ml (2.0 mM) of triethylamine and the mixture was refluxed for 5 hours. After completion of the reaction, the solvent was distilled off under reduced pressure and the residue was added 100 ml of purified water and, further, was extracted with 100 ml of chloroform. The organic layer was washed 100 ml of saturated aqueous NaCl solution and dried over MgSO₄ and the solvent was distilled off under reduced pressure. The residue was purified by silica gel chromatography (eluent: chloroform-methanol = 10:1) and was recrystallised from ethanol to provide 2.84 mg (yield 68.1%) of the title compound as light-yellow crystal.
¹H-NMR(200MHz,CDCl₃)δ: 1.17-1.33(2H,m), 1.48-1.78(3H,m), 1.78-1.98(2H,m), 3.04-3.24(2H,m), 3.50-3.62(2H,t,J=7.0Hz), 3.70-3.88(2H,m), 5.13(2H,s), 7.22(1H,dd,J=9.0, 7.0Hz), 7.46(1H,s), 7.51(1H,dd,J=7.0, 1.0Hz), 7.62(1H,dd,J=9.0, 1.0Hz).
IR(KBR): 1670, 1631, 1383, 1180 cm⁻¹.
m.p. 152-153°C

| Elemental Analysis for C₁₇H₁₉N₄O₃SF₃·H₂O: | | | |
|---|---|---|---|
| Calcd.: | C, 48.00; | H, 4.74; | N, 13.17. |
| Found : | C, 48.08; | H, 4.47; | N, 13.25. |

### Example 20

### 1,2-Dihydro-3-methyl-1-[3-(1-tert-butoxycarbonylpiperidin-4-yl)propan-1-yl]-1,4,7b-triazacyclopent[cd]inden-2-one

A solution prepared by dissolving 4.87 g (20,0 mM) of 3-(1-tert-butoxycarbonylpiperidin-4-yl)-1-propanol and 5.54 ml (40.0 mM) of triethylamine in 100 ml of ethanol was added 1.86 ml (24.0 mM) of methanesulfonyl chloride dropwise at 0°C. After completion of dropwise addition, the reaction mixture was stirred at room temperature for 30 minutes and was poured in 200 ml of iced water. The reaction mixture was extracted with 100 ml of ethyl acetate and the organic layer was washed with 100 ml of saturated aqueous NaCl solution and dried over MgSO₄. The solvent was distilled off under reduced pressure to provide 6.66 g (yield 100%) of [3-(1-tert-butoxycarbonylpiperidin-4-yl)propan-1-yl]methanesulfonate as brown oil. Further, a solution prepared by dissolving 3.46 g (20.0 mM) of 1,2-dihydro-3-methyl-1,4,7b-triazacyclopent[cd]inden-2-one, 6.66 g (20.0 mM) of [3-(1-tert-butoxycarbonylpiperidin-1-yl]methanesulfonate and 3.58 ml (24.0 mM) of DBU in 50 ml of DMF was heated at 80°C for 5 hours. After completion of the reaction, the reaction mixture was poured in 100 ml of iced water and the mixture was extracted with 200 ml of ethyl acetate. The organic layer was washed with 200 ml of purified water at 3 times and further with 200 ml of saturated NaCl solution and dried over MgSO₄. The solvent was distilled off under reduced pressure and the residue was purified by silica gel column chromatography (eluent: ethyl acetate) to privide 7.0 g (yield 87.8%) of the title compound as brown oil.
¹H-NMR(200MHz,CDCl₃)δ: 0.93-1.42(5H,m), 1.44(9H,s), 1.57-1.73(2H,m), 1.75-2.01(2H,m), 2.56-2.77(2H,m), 6.79(1H,d,J=7.4Hz), 7.49(1H,d,J=8.6Hz), 7.72(1H,dd,J=8.6, 7.6Hz).
IR(KBR): 1700, 1680, 1623, 1436 cm⁻¹.

### Example 21

### 1,2-Dihydro-3-methyl-1-[3-(1-trifluoroacetylpiperidin-4-yl)propan-1-yl]-1,4,7b-triazacyclopent[cd]inden-2-one

### i) Synthesis of 1,2-dihydro-3-methyl-1-[3-(piperidin-4-yl)propan-1-yl]-1,4,7b-triazacyclopent[cd]inden-2-one dihydrochloride

A solution prepared by dissolving 7.0 g (17.6 mM) of 1,2-dihydro-3-methyl-1-[3-(1-tert-butoxycarbonylpiperidin-4-yl)propan-1-yl]-1,4,7b-triazacyclopent[cd]inden-2-one in 50 ml of ethanol was added 40 ml (48.7 mM) of 12N hydrochloric acid and the mixture was stirred at room temperature for 1 hour. After completion of the reaction, the reaction mixture was distilled off under reduced pressure and the crystals that formed were collected by filtration and the crystals were washed with small amounts of ethanol and ether to provide 4.53 g (yield 69.3%) of the title compound as brown solid.
¹H-NMR(200MHz,DMSO-d₆)δ: 1.18-1.42(4H,m), 1.44-1.66(1H,m), 1.66-1.90(4H,m), 2.64-2.92(2H,m), 2.81(3H,s), 3.08-3.28(2H,m), 4.08(2H,t,J=6.6Hz), 7.62(1H,d,J=7.6Hz), 7.79(1H,d,J=8.4Hz), 8.19(1H,t,J=8.4Hz), 8.85-9.26(2H,m,NH).
IR(KBR): 1712, 1643, 1555 cm⁻¹.

### ii) Synthesis of 1,2-dihydro-3-methyl-1-[3-(1-trifluoroacetylpiperidin-4-yl)propan-1-yl]-1,4,7b-triazacyclopent[cd]inden-2-one

In 20 ml of acetonitrile was suspended 743 mg (2.0 mM) of 1,2-dihydro-3-methyl-1-[3-(piperidin-4-yl)propan-1-yl]-1,4,7b-triazacyclopent[cd]inden-2-one dihydrochloride followed by addition of 0.60 ml (4.0 mM) of DBU and 0.83 ml (6.0 mM) of triethylamine with stirring to provide uniform solution. The solution was added 0.34 ml (2.4 mM) of trifluoro acetic anhydride and was stirred at 0°C for 1 hour. After completion of the reaction, the reaction mixture was poured in 100 ml of iced water and the mixture was extracted with 100 ml of ethyl acetate. The organic layer was washed with 100 ml of saturated aqueous NaCl solution and dried over MgSO₄ and the solvent was distilled off under reduced pressure. The residue was purified by silica gel column chromatography (eluent: ethyl acetateethanol = 10:1) and was recrystallized from ethyl acetate to provide 559 mg (70.9%) of the title compound as white crystal.
¹H-NMR(200MHz,CDCl₃)δ: 1.05-1.50(4H,m), 1.50-1.70(1H,m), 1.75-2.00(4H,m), 2.62-2.85(1H,m), 2.83(3H,s), 3.00-3.20(1H,m), 3.90-4.10(1H,m), 4.06(2H,t,J=7.1Hz), 4.42-4.60(1H,m), 6.78(1H,d,J=7.4Hz), 7.50(1H,d,J=8.8Hz), 7.73(1H,t,J=8.0Hz).
IR(KBR): 1697, 1263, 1198 cm⁻¹.
m.p. 136.5-137.5°C

| Elemental Analysis for C₁₉H₂₁N₄O₂F₃: | | | |
|---|---|---|---|
| Calcd.: | C, 57.86; | H, 5.37; | N, 14.21. |
| Found : | C, 57.84; | H, 5.28; | N, 14.32. |

### Example 22

### 1,2-Dihydro-3-methyl-1-[3-(1-pentafluoropropionylpiperidin-4-yl)propan-1-yl]-1,4,7b-triazacyclopent[cd]inden-2-one

In 20 ml of acetonitrile was suspended 743 mg (2.0 mM) of 1,2-dihydro-3-methyl-1-[3-(piperidin-4-yl)propan-1-yl]-1,4,7b-triazacyclopent[cd]inden-2-one dihydrochloride followed by addition of 0.60 ml (4.0 mM) of DBU and 0.83 ml (6.0 mM) of triethylamine with stirring to provide uniform solution. The solution was added 0.47 ml (2.4 mM) of pentafluoropropionic anhydride at 0°C and was stirred at 0°C at 1 hour. After completion of the reaction, the reaction mixture was poured in 100 ml of iced water and the mixture was extracted with 100 ml of ethyl acetate. The organic layer was washed with 100 ml of saturated aqueous NaCl solution and dried over MgSO₄ and the solvent was distilled off under reduced pressure. The residue was purified by silica gel chromatography (eluent: ethyl acetate-ethanol = 10:1) to provide light-yellow oil. The oil was added ether and the crystals were collected by filtration and were washed with small amounts of hexane to provide 480 mg (54.0%) of teh title compound as white solid.
¹H-NMR(200MHz,CDCl₃)δ: 1.05-1.50(4H,m), 1.50-1.75(1H,m), 1.75-2.00(4H,m), 2.60-2.90(1H,m), 2.83(3H,s), 3.00-3.17(1H,m), 4.07(2H,t,J=6.9Hz), 4.00-4.20(1H,m), 4.45-4.60(1H,m), 6.78(1H,d,J=7.2Hz), 7.50(1H,d,J=8.4Hz), 7.72(1H,dd,J=7.2, 8.4Hz).
IR(KBR): 1695, 1626, 1234 cm⁻¹.
m.p. 78.0-79.0°C

| Elemental Analysis for C₂₀H₂₁N₄O₂F₅·H₂O: | | | |
|---|---|---|---|
| Calcd.: | C, 52.98; | H, 4.89; | N, 12.36. |
| Found : | C, 52.70; | H, 4.47; | N, 12.26. |

### Example 23

### 1,2-Dihydro-3-methyl-1-[3-(1-trifluoromethanesulfonylpiperidin-4-yl)propan-1-yl]-1,4,7b-triazacyclopent[cd]inden-2-one

In 10 ml of dichloromethane was suspended 371 ml (1.0 mM) of 1,2-dihydro-3-methyl-1-[3-(piperidin-4-yl)propan-1-yl]-1,4,7b-triazacyclopent[cd]inden-2-one dihydrochloride followed by addition of 0.69 ml (5.0 mM) of triethylamine and the solution was further added 0.20 ml (1.2 mM) of trifluoromethanesulfonic anhydride at 0°C and was stirred at 0°C for 1 hour. After completion of the reaction, the solvent was distilled off under reduced pressure and the residue was poured in 100 ml of iced water and the mixture was extracted with 100 ml of ethyl acetate. The organic layer was washed with 100 ml of saturated aqueous NaCl solution and dried over MgSO₄ and the solvent was distilled off under reduced pressure. The residue was purified by silica gel chromatography (eluent: ethyl acetate) to provide 168 mg (yield 39.0%) of the title compound as light-yellow oil. The oil was added ether and the crystals were collected by filtration and were washed with small amounts of ether to provide 117 mg (yield 27.2%) of the title compound as brown solid.
¹H-NMR(200MHz,CDCl₃)δ: 1.15-1.70(5H,m), 1.70-2.00(4H,m), 2.83(3H,s), 2.90-3.10(2H,m), 3.85-4.00(2H,m), 4.07(2H,t,J=7.1Hz), 6.78(1H,d,J=7.6Hz), 7.50(1H,d,J=8.6Hz), 7.72(1H,dd,J=7.6, 8.6Hz).
IR(KBR): 1699, 1383, 1192 cm⁻¹.
m.p. 127-128°C

| Elemental Analysis for C₁₈H₂₁N₄O₃SF₃·0.2H₂O: | | | |
|---|---|---|---|
| Calcd.: | C, 49.81; | H, 4.97; | N, 12.91. |
| Found : | C, 49.70; | H, 4.97; | N, 12.84. |

### Example 24

### 3-Methyl-2-[2-(1-tert-butoxycarbonylpiperidin-4-yl)ethan-1-yl]thio-1,4,7b-triazacyclopent[cd]indene

A solution prepared by dissolving 9.13 g (29.7 mM) of 2-(tert-butoxycarbonylpiperidin-4-yl)ethan-1-ylmethanesulfonate in 150 ml of acetnitrile was added 8.99 g (60.0 mM) of sodium iodide and the solution was refluxed for 1 hour. After completion of the reaction, crystals of methanesulfonic sodium that formed were collected by filtration and the filtrate was concentrated under reduced pressure. The residue was added 100 ml of purified water and was extracted with 100 ml of ethyl acetate. The layer was washed with saturated aqueous NaCl solution and dried over MgSO₄ and the solvent was distilled off under reduced pressure to provide 8.94 g (yield 88.7%) of 2-(tert-butoxycarbonylpiperidin-4-yl)ethan-1-yl iodide as brown oil. A solution prepared by dissolving 5.00 g (26.4 mM) of 1,2-dihydro-3-methyl-1,4,7b-triazacyclopent[cd]inden-2-thione and 8.94 g (26.4 mM) of 2-(tert-butoxycarbonylpiperidin-4-yl)ethan-1-yl iodide in 60 ml of DMF was added 7.32 ml (52.9 mM) of ethylamine with stirring at 80°C for 20 minutes. After completion of the reaction, the reaction mixture was poured in 100 ml of iced water the mixture was extracted with 200 ml of ethyl acetate. The organic layer was washed with 200 ml of purified water at 3 times and further with 200 ml of saturated aqueous NaCl solution and dried over MgSO₄. The solvent was distilled off under reduced pressure to provide 10.57 (yield 100%) of the title compound as brown liquid.
¹H-NMR(200MHz,CDCl₃)δ: 1.08-1.33(2H,m), 1.46(9H,s), 1.60-1.93(5H,m), 2.62-2.83(2H,m), 2.91(3H,s), 3.56(2H,t,J=7.4Hz), 4.00-4.22(2H,m), 7.67(1H,d,J=7.8Hz), 7.73(1H,d,J=7.8Hz), 7.95(1H,t,J=7.8Hz).

### Example 25

### 3-Methyl-2-[2-(1-trifluoromethanesulfonylpiperidin-4-yl)ethan-1-yl]thio-1,4,7b-triazacyclopent[cd]indene

### i) Synthesis of 3-methyl-2-[2-(piperidin-4-yl)ethane-1-yl]thio-1,4,7b-triazacyclopent[cd]indene dihydrochloride

A solution prepared by dissolving 10.57 g (26.4 mM) of 3-methyl-2-[2-(piperidin-4-yl)ethan-1-yl]thio-1,4,7b-triazacyclopent[cd]indene in 100 ml of 2-propanol was added 12.5 ml (150 mM) of 12N hydrochloric acid with stirring at room temperature for 1 hour. After completion of the reaction, the reaction mixture was concentrated under reduced pressure and the precipitate that formed was collected by filtration. The precipitate was washed with small amounts of 2-propanol and ether to provide 8.14 g (yield 82.5%) of the title compound as brown solid.
¹H-NMR(200MHz,DMSO-d₆)δ: 1.34-2.02(5H,m), 1.68-2.02(5H,m), 2.72-3.00(2H,m), 2.89(3H,s), 3.14-3.32(2H,m), 3.50-3.65(2H,m), 8.00(1H,d,J=8.0Hz), 8.02(1H,d,J=8.0Hz), 8.23(1H,t,J=8.0Hz), 8.44-9.30(2H,m,NH).

### ii) Synthesis of 3-methyl-2-[2-(1-trifluoromethanesulfonylpiperidin-4-yl)ethan-1-yl]thio-1,4,7b-triazacyclopent[cd]indene

In 20 ml of acetnitrile was suspended 1.12 g (3.0 mM) of 3-methyl-2-[2-(piperidin-4-yl)ethan-1-yl]thio-1,4,7b-triazacyclopent[cd]indene dihydrochloride followed by addition of 0.9 ml (6.0 mM) of DBU and 0.83 ml (6.0 mM) of triethylamine with stirring to provide uniform solution. The solution was added 2.14 g (6.0 mM) of N-phenyltrifluoromethanesulfonimide and was stirred at room temperature for 16 hours. After completion of the reaction, the solvent was distilled off under reduced pressure and the residue was added 100 ml of purified water and was extracted with 100 ml of ethyl acetate. The organic layer was washed with 100 ml of saturated aqueous NaCl solution and dried over MgSO₄ and the solvent was distilled off under reduced pressure. The residue was purified by silica gel column chromatography (ethyl acetate-hexane = 2:1) and the crystals were recrystallized from ethyl acetate to provide 890 mg (yield 68.6%) of the title compound as white crystals.
¹H-NMR(200MHz,CDCl₃)δ: 1.29-1.50(2H,m), 1.66-1.85(1H,m), 1.90-1.97(4H,m), 2.91(3H,s), 3.06(2H,m), 3.57(2H,t,J=7.3Hz), 3.99(2H,m), 7.68(1H,d,J=8.0Hz), 7.74(1H,d,J=8.0Hz), 7.96(1H,t,J=8.0Hz).
IR(KBR): 1491, 1393, 1184 cm⁻¹.
m.p. 125.0-126.0°C

| Elemental Analysis for C₁₇H₁₉N₄O₂S₂F₃: | | | |
|---|---|---|---|
| Calcd.: | C, 47.21; | H, 4.43; | N, 12.95. |
| Found : | C, 47.21; | H, 4.31; | N, 13.00. |

### Example 26

### 1,2-Dihydro-3-methyl-1-[2-(1-trifluoroacetylpiperidin-4-yl)-ethan-1-yl]-1,4,7b-triazacyclopent[cd]inden-2-one

To a solution prepared by dissolving 2.14 g (6.0 mM) of 1,2-dihydro-3-methyl-1-[2-(piperidin-4-yl)ethan-1-yl]-1,4,7b-triazacyclopent[cd]inden-2-one dihydrochloride and 1.8 ml (12.0 mM) of DBU in 10 ml of acetonitrile, was added 1.7 ml (12.0 mM) of triethylamine and 0.85 ml (6.0 mM) of trifluoroacetic anhydride dropwise at 0°C. After completion of the addition, the reaction solution was stirred at room temperature foran hour. After the reaction, the solvent was distilled off under reduced pressure. The resulting residue was extracted with 100 ml of ethyl acetate and the organic layer was washed with 100 ml of brine. The organic layer was dried over MgSO₄ and then the solvent was distilled off under reduced pressure. The resulting residue was purified by silica gel column chromatography (eluent: ethyl acetate-ethanol = 10:1) to provide 1.88 g (yield 82.3%) of title compound as white solid. Recrystallization of the solid from ethanol afforded analytical pure white crystals.
¹H-NMR(200MHz,CDCl₃)δ: 1.17-1.41(2H,m), 1.54-2.07(5H,m), 2.65-2.82(1H,m), 2.83(3H,s), 3.01-3.19(1H,m), 3.93-4.07(1H,m), 4.13(2H,t,J=6.6Hz), 4.47-4.62(1H,m), 6.78(1H,d,J=7.4Hz), 7.51(1H,d,J=8.6Hz), 7.72(1H,dd,J=8.6Hz,7.4Hz).
IR(KBr): 1691, 1685, 1454, 1149 cm⁻¹.
m.p. 203-204°C

| Elemental Analysis for C₁₈H₁₉N₄O₂F₃ | | | |
|---|---|---|---|
| Calcd. : | C, 56.84; | H, 5.03; | N, 14.73 |
| Found : | C, 56.88; | H, 5.10; | N, 14.88 |

### Example 27

### 1,2-Dihydro-3-methyl-1-[2-(1-pentafluoropropionylpiperidin-4-yl)ethan-1-yl]-1,4,7b-triazacyclopent[cd]inden-2-one

To a solution prepared by dissolving 2.14 g (6.0 mM) of 1,2-dihydro-3-methyl-1-[2-(piperidin-4-yl)ethan-1-yl]1,4,7b-triazacyclopent[cd]inden-2-one dihydrochloride and 1.8 ml (12.0 mM) of DBU in 10 ml of acetonitrile, was added 1.7 ml (12.0 mM) of triethylamine and 1.18 ml (6.0 mM) of pentafluoropropionic anhydride dropwise at 0°C. After completion of the addition, the reaction solution was stirred at room temperature for an hour. After the reaction, the solvent was distilled off under reduced pressure. The resulting residue was extracted with 100 ml of ethyl acetate and the organic layer was washed with 100 ml of brine. The organic layer was dried over MgSO₄ and then the solvent was distilled off under reduced pressure. The resulting residue was purified by silica gel column chromatography (eluent: ethyl acetate-ethanol = 10:1) to provide 2.21 g (yield 85.6 %) of title compound as white solid. Recrystallization of the solid from ethanol afforded analytical pure white crystals.
¹H-NMR(200MHz,CDCl₃)δ: 1.17-1.42(2H,m), 1.54-2.06(5H,m), 2.62-2.81(1H,m), 2.84(2H,s), 3.01-3.20(1H,m), 4.13(2H,t,J=6.8Hz), 4.08-4.23(1H,m), 4.48-4.63(1H,m), 6.78(1H,d,J=7.4Hz), 7.52(1H,d,J=8.6Hz), 7.72(1H,dd,J=8.6Hz,7.4Hz).
IR(KBr): 1689, 1450, 1209 cm⁻¹.
m.p.
146-147°C

| Elemental Analysis for C₁₉H₁₉N₄O₂F₅ | | | |
|---|---|---|---|
| Calcd.: | C, 53.03; | H, 4.45; | N, 13.02 |
| Found : | C, 53.11; | H, 4.51; | N, 13.09 |

### Example 28

### 1,2-Dihydro-3-methyl-1-[trans-4-(tert-butoxycarbonylamino)methyl-1-cyclohexylmethyl]1,4,7b-triazacyclopent[cd]inden-2-one

To a solution prepared by dissolving 4.87 g (20.0 mM) of trans-4-(tert-butoxycarbonylamino)methyl-1-cyclohexanemethanol and 5.5 ml(40.0 mM) of triethylamine in 50 ml of tetrahydrofuran, was added 1.9 ml (24.0 mM) of methanesulfonyl chloride dropwise at 0°C. After completion of the addition, the reaction mixture was stirred at room temperature for an hour. After the reaction, the solvent was distilled off under reduced pressure. After the reaction, the solvent was distilled off under reduced pressure. The resulting residue was extracted with 100 ml of ethyl acetate and the organic layer was washed with 100 ml of brine. The organic layer was dried over MgSO₄ and then the solvent was distilled off under reduced pressure to afford 6.29 g of trans-4-(tert-butoxycarbonylamino)methyl-1-cyclohexylmethyl methanesulfonate.

A solution prepared by dissolving 6.29 g (19.6 mM) of trans-4-(tert-butoxycarbonylamino)methyl-1-cyclohexylmethyl methanesulfonate, 3.40 g (19.6 mM) of 1,2-dihydro-3-methyl-cyclopent[cd]inden-2-one, and 3.5 ml (23.5 mM) of DBU in 50 ml of DMF was heated at 100°C for an hour. After the reaction, the reaction mixture was poured onto iced water and the precipitate appeared was collected by filtration. The precipitate was dissolved in 100 ml of dichloromethane and the organic layer was washed with brine once. The organic layer was dried over MgSO₄ and the solvent was distilled off under reduced pressure to give 6.23 g (yield 79.8%) of title compound as brown solid.
¹H-NMR(200MHz,CDCl₃)δ: 0.77-1.09(4H,m), 1.19-1.52(2H,m), 1.43(9H,s), 1.62-1.98(4H,m), 2.83(3H,s), 2.96(2H,m), 3.90(2H,d,J=6.8Hz), 4.61(1H,NH,t,J=5.4Hz), 6.78(1H,d,J=7.4Hz), 7.48(1H,d,J=7.2Hz), 7.71(1H,t,J=7.2Hz).
IR(KBr): 1705, 1525, 1261 cm⁻¹.
m.p. 148-150°C

### Example 29

### 1,2-Dihydro-3-methyl-1-[trans-4-(trifluoroacetylamino)methyl-1-cyclohexylmethyl]-1,4,7b-triazacyclopent[cd]inden-2-one

### i) Synthesis of 1,2-dihydro-3-methyl-1-[trans-4-aminomethyl-1-cyclohexylmethyl]-1,4,7b-triazacyclopent[cd]inden-2-one dihydrochloride

To a solution prepared by dissolving 6.23 g (15.6 mM) of 1,2-dihydro-3-methyl-1-[trans-4-(tert-butoxycarbonylamino)methyl-1-cyclohexylmethyl]1,4,7b-triazacyclopent[cd]inden-2-one in 50 ml of ethanol, was added 12.8 ml (156 mM) of 12N hydrochloric acid dropwise at room temperature and the reaction solution was stirred at room temperature for an hour. After the reaction, the solvent was condensed under reduced pressure. The precipitate appeared was collected by filtration. The precipitate was washed with ethanol and ether to give 4.56 g (yield 78.7%) of title compound as light brown solid.
¹H-NMR(200MHz,DMSO-d₆)δ: 0.76-1.22(4H,m), 1.44-1.68(2H,m), 1.68-1.93(4H,m), 2.55-2.70(2H,m), 2.82(3H,s), 3.95(2H,d,J=7.0Hz), 7.62(1H,d,J=7.6Hz), 7.81(1H,d,J=8.4Hz), 8.21(1H,t,J=7.8Hz)/
IR(KBr): 3385, 1732, 1591 cm⁻¹.

### ii) Synthesis of 1,2-dihydro-3-methyl-1-[trans-4-(trifluoroacetylamino)-methyl-1-cyclohexylmethyl]-1,4,7b-triazacyclopent[cd]inden-2-one]

To a solution prepared by dissolving 1.49 g (4.0 mM) of 1,2-dihydro-3-methyl-1-[trans-4-aminomethyl-1-cyclohexylmethyl]-1,4,7b-triazacyclopent[cd]inden-2-one dihydrochloride and 1.19 ml (8.0 mM) of DBU in 30 ml of acetonitrile, was added 1.1 ml (8.0mM) of triethylamine and 0.95 ml (8.0 mM) of ethyl trifluoroacetate at room temperature. The reaction solution was stirred at room temperature for an hour. After the reaction, the solvent was distilled off under reduced pressure. The residue was extracted with 100 ml of dichloromethane and the organic layer was washed with 100 ml of brine. The organic layer was dried over MgSO₄ and the solvent was distilled off under reduced pressure. The resulting residue was purified by silica gel column chromatography (eluent: ethyl acetate-ethanol = 10:1) to give white solid which was recrystallized from ethanol to afford 1.14 g (yield 72.2%) of title compound as white needles.
¹H-NMR(200MHz,CDCl₃)δ: 0.94-1.25(4H,m), 1.55-1.63(1H,m), 1.75-1.87(5H,m), 2.82(3H,s), 3.22(2H,t,J=6.6Hz), 3.51(2H,d,J=4.8Hz), 6.56(1H,NH,brs), 6.78(1H,d,J=7.2Hz), 7.49(1H,d,J=8.8Hz), 7.71(1H,dd,J=7.4Hz,8.8Hz).
IR(KBr): 3290, 1689, 1568, 1188 cm⁻¹.
m.p. 190-191°C

| Elemental Analysis for C₁₉H₂₁N₄O₇F₃: | | | |
|---|---|---|---|
| Calcd.: | C, 57.86; | H, 5.37; | N, 14.21 |
| Found : | C, 58.08; | H, 5.18; | N, 14.21 |

### Example 30

### 1,2-Dihydro-3-methyl-1-[trans-4-(pentafluoropropionylamino)-methyl-1-cyclohexylmethyl]-1,4,7b-triazacyclopent[cd]inden-2-one

To a solution prepared by dissolving 1.49 g (4.0 mM) of 1,2-dihydro-3-methyl-1-[trans-4-aminomethyl-1-cyclohexylmethyl]-1,4,7b-triazacyclopent[cd]inden-2-one dihydrochloride and 1.19 ml (8.0 mM) of DBU in 30 ml of acetonitrile, was added 1.1 ml (8.0 mM) of triethylamine and 1.18 ml (8.0 mM) of ethyl pentafluoropropionate at room temperature. The reaction solution was stirred at room temperature for an hour. After the reaction, the solvent was distilled off under reduced pressure. The residue was extracted with 100 ml of dichloromethane and the organic layer was washed with 100 ml of brine. The organic layer was dried over MgSO₄ and the solvent was distilled off under reduced pressure. The resulting residue was purified by silica gel column chromatography (eluent: ethyl acetate-ethanol = 10:1) to give colarless oil which was crystallized from ethyl acetate-ether to afford 1.30 g (yield 73.3%) of title compound as white crystals.
¹H-NMR(200MHz,CDCl₃)δ: 0.94-1.18(4H,m), 1.58(1H,m), 1.82-1.89(5H,m), 2.82(3H,s), 3.24(2H,t,J=6.4Hz), 3.90(2H,d,J=7.0Hz), 6.77(2H,d,J=7.4Hz), 7.49(1H,d,J=8.4Hz), 7.71(1H,t,J=8.0Hz)
IR(KBr): 3313, 1686, 1215, 1151 cm⁻¹.
m.p. 170.5-171.5°C

| Elemental Analysis for C₂₀H₂₁N₄O₂F₅ | | | |
|---|---|---|---|
| Calcd.: | C, 54.05; | H, 4.76; | N, 12.61 |
| Found : | C, 54.25; | H, 4.57; | N, 12.70 |

### Example 31

### 1,2-Dihydro-3-methyl-1-[trans-4-(trifluoromethanesulfonylaminomethyl-1-cyclohexylmethyl]-1,4,7b-triazacyclopent[cd]inden-2-one

To a solution prepared by dissolving 1.49 g (4.0 mM) of 1,2-dihydro-3-methyl-1-[trans-4-aminomethyl-1-cyclohexylmethyl]-1,4,7b-triazacyclopent[cd]inden-2-one dihydrochloride and 1.19 ml (8.0 mM) of DBU in 30 ml of acetonitrile, was added 1.1 ml (8.0 mM) of triethylamine and 2.14 g (8.0 mM) of N-phenyltrifluoromethanesulfonimide at room temperature. The reaction solution was stirred at room temperature for an hour. After the reaction, the solvent was distilled off under reduced pressure. The residue was extracted with 100 ml of dichloromethane and organic layer was washed with 100 ml of brine. The organic layer was dried over MgSO₄ and the solvent was distilled off under reduced pressure. The resulting residue was purified by silica gel column chromatography (eluent: ethyl acetate-ethanol = 10:1) to give white solid which was recrystallized from chloroform-ethanol to afford 1.39 g (yield 80.7%) of title compound as white crystals.
¹H-NMR(200MHz,CDCl₃)δ: 0.91-1.19(4H,m), 1.52-1.59(1H,m), 1.83-1.88(5H,m), 2.81(3H,s), 3.16(2H,d,J=6.8Hz), 3.91(2H,d,J=6.8Hz), 6.79(1H,d,J=7.6Hz), 7.50(1H,d,J=8.6Hz), 7.73(1H,dd,J=8.6Hz,7.6Hz).
IR(KBr): 3313, 1685, 1191 cm⁻¹.
m.p. 213-214°C

| Elemental Analysis for C₁₈H₂₁N₄O₃SF₃·0.2H₂O | | | |
|---|---|---|---|
| Calcd.: | C, 49.81; | H, 4.97; | N, 12.91 |
| Found : | C, 49.53; | H, 4.74; | N, 13.30 |

### Example 32

### 1,2-Dihydro-3-methyl-1-[trans-4-(4-methylphenylsulfonylamino)methyl-1-cyclohexylmethyl]-1,4,7b-triazacyclopent[cd]inden-2-one

To a solution prepared by dissolving 0.74 mg (2.0 mM) of 1,2-dihydro-3-methyl-1-[trans-4-aminomethyl-1-cyclohexylmethyl]-1,4,7b-triazacyclopent[cd]inden-2-one dihydrochloride and 0.6 ml (4.0 mM) of DBU in 20 ml of dichloromethane, was added 1.4 ml (10.0 mM) of triethylamine and 0.46 g (2.4 mM) of p-toluenesulfonyl chloride at room temperature. The reaction solution was stirred at room temperature for an hour. After the reaction, the solvent was distilled off under reduced pressure. The residue was extracted with 100 ml of dichloromethane and the organic layer was washed with 100 ml of brine. The organic layer was dried over MgSO₄ and the solvent was distilled off under reduced pressure. The resulting residue was purified by silica gel column chromatography (eluent: ethyl acetateethanol = 10:1) to give white solid which was recrystallized from ethyl acetate to afford 0.78 g (yield 86.6%) of title compound as white crystals.
¹H-NMR(200MHz,CDCl₃)δ: 0.75-1.20(4H,m), 1.30-1.50(1H,m), 1.70-2.00(5H,m), 2.42(3H,s), 2.76(2H,t,J=6.6Hz), 2.82(3H,s), 3.87(2H,d,J=7.0Hz), 4.62(1H,t,J=6.6Hz), 6.76(1H,d,J=7.4Hz), 7.29(2H,d,J=7.8Hz), 7.49(1H,d,J=8.6Hz), 7.67-7.74(3H,m).
IR(KBr): 3178, 2920, 1674 cm⁻¹.

| Elemental Analysis for C₂₄H₂₈N₄O₃S: | | | |
|---|---|---|---|
| Calcd.: | C, 63.69; | H, 6.24; | N, 12.38 |
| Found : | C, 63.49; | H, 6.28; | N, 12.11 |

### Test Example 1

Experiments of PDGF-induced proliferation in Hs68 cells Method: Human skin fibroblasts (Hs68, Institute for Fermentation, Osaka) were seeded in 48-well plates precoated with collagen type I (Falcon) (25000 cells/well), and cultured for one day. Then, the cells were cultured in Dulbecco's modified Eagle's medium (high glucose) containing 0.1% bovine serum albumin for 48 hours. Fifteen (15) minutes after the addition of the drug, the cells were stimulated with 5 ng/ml of PDGF-BB (Becton Dickinson Labware) containing ³H-thymidine (final concentration 0.4 mCi/ml) (Amersham). After 24 hours, the reaction was stopped with 7.5% trichloroacetic acid at 4°C and the plate was allowed to stand at 4°C for 30 minutes. The cells were washed with Ca²⁺- and Mg²⁺-free phosphate-buffered saline (PBS), after then 0.1% sodium dodecyl sulfate (SDS)/0.4N NaOH was added and the plate was allowed to stand at 37°C for 1 hour to lyze the cells. The whole content of each well was taken in a vial and neutralized with 1N HCl, and then toluene scintillator (Wako Pure Chemical Ind.) was added to each vial. After stirring, the amount of ³H-thymidine incorporated in the cells was determined with a liquid scintillation counter. The cellular uptake of ³H-thymidine was used as an indicator of cell proliferation. The effect of the drug on the PDGF-BB (5 ng/ml) uptake of ³H-thymidine was expressed as % inhibition. The results are shown in Table 1. From the results, it is clear that the compound of this invention shows an inhibitory effect on PDGF-induced cell proliferation.

**Table 1**

| Inhibitory effect on cell proliferation | | |
|---|---|---|
| Compound (Example No.) | Concentration (M) | % Inhibition |
| 9(ii) | 3x10⁻⁷ | 94.9 |
| 8(ii) | 1x10⁻⁶ | 74.9 |
| 11(iii) | 3x10⁻⁶ | 59.2 |
| 12(ii) | 1x10⁻⁶ | 96.6 |
| 19 | 1x10⁻⁶ | 72.8 |
| 23 | 3x10⁻⁷ | 44.6 |
| 25(ii) | 3x10⁻⁷ | 83.4 |
| 27 | 1x10⁻⁶ | 62.7 |
| 29 | 3x10⁻⁶ | 76.1 |
| 30 | 1x10⁻⁶ | 62.3 |
| 32 | 1x10⁻⁶ | 80.3 |

### Test Example 2

Experiments of urinary protein excretion in SHC rats Method: Spontaneously hypercholesterolemic rats (male, 7 weeks old) were given a drug suspended in 0.5% methylcellulose (100 cP) by oral administration once daily for 6 weeks. A control group was given 0.5% methylcellulose (100 cP) by the same route once daily. As a normal control group, Sprague-Dawley rats (SD rats) (male, 7 weeks old, CLEA Japan, Inc.) were given 0.5% methylcellulose (100 cP) orally once daily. Before the start of medication and at 2-weeks intervals after the first administration of drugs, urine was collected for twenty four hours and the total protein and albumin excreted in the urine were determined. The assay of total protein and albumin was carried out using the A/GB Test Wako (Wako Pure Chemical Ind.).

The results are shown in Table 2 and Table 3. From the results, it is clear that the compound of the invention inhibits urinary protein excretion and is useful for treating renal diseases such as chronic glomerulonephritis.

**Table 2**

| Effect on urinary total protein excretion | | | | | |
|---|---|---|---|---|---|
| Group | Dose (mg/kg/day) | Urinary total protein (mg/day) | | | |
| | | Duration of administration (weeks) | | | |
| | | 0 | 2 | 4 | 6 |
| Control | - | 39.5 | 125.7 | 235.3 | 275.2 |
| Example 9(ii) | 1 | 39.2 | 50.4 | 91.3 | 199.4 |
| SD rat | - | 19.1 | 45.4 | 53.0 | 38.4 |

**Table 3**

| Effect on urinary albumin excretion | | | | | |
|---|---|---|---|---|---|
| Group | Dose (mg/kg/day) | Urinary albumin (mg/day) | | | |
| | | Duration of administration (weeks) | | | |
| | | 0 | 2 | 4 | 6 |
| Control | - | 12.1 | 67.1 | 154.7 | 174.4 |
| Example 9(ii) | 1 | 12.0 | 10.9 | 46.2 | 129.9 |
| SD rat | - | 4.2 | 10.1 | 8.2 | 9.7 |

### Test Example 3

### Inhibition of the proliferation of mesangial cells

Glomerular mesangial cells isolated from 4-week-old male Sprague-Dawley rats (Japan Clea) were cultured in a 24-well plate (Falcon) up to the subconfluence. The cells were subsequently cultured in Dulbecco's modified Eagle MEM containing 0.2% bovine serum albumin for 24 - 48 hours before addition of a drug substance. Fifteen minutes after the drug substance was added to the culture, the cells were allowed to react with 5 ng/ml of PDGF-BB (Becton Dickinson Labwere) for stimulation, simultaneously adding ³H-Thymidine (final concentration 0.2 µ Ci/ml) (Amasham), for 24 hours. The reaction was blocked by adding 7.5 % TCA of 4°C. The mixture was allowed to stand at 4°C for 30 minutes. The cells were washed with phosphate buffered saline containing no Ca²⁺ and Mg²⁺. A 0.4N NaOH/0.1% sodium dodecyl sulfate (SDS) solution was added to the washed cells and the mixture was allowed to stand at 37°C for an hour to solubilize the cells. The whole qauntity was placed in a vial and neutralized with 1N HCl. Scintillator A (Wako Pure Chemicals) was added to this with stirring. The quantity of ³H-Thymidine incorporated in the cells was measured by the liquid scintillation counter. The increase in the quantity of the incorporated ³H-Thymidine was designated as the parameter of the cell proliferation. The inhibitory effect of a drug substance on the intake with PDGF-BB (5 ng/ml) was expressed as % inhibition (Table 1).

**Table 4**

| Cell proliferation inhibiting activity | | |
|---|---|---|
| Compound (No.) | Dose (M) | Proliferation inhibiting activity (% Inhibition) |
| 1 | 10⁻⁶ | 20.5 |
| 1 | 3 x 10⁻⁶ | 39.4 |
| 1 | 10⁻⁶ | 87.6 |
| 2 | 10⁻⁶ | 11.1 |
| 2 | 3 x 10⁻⁶ | 40.1 |
| 2 | 10⁻⁵ | 58.3 |
| 3 | 3 x 10⁻⁷ | 39.0 |
| 3 | 10⁻⁶ | 91.1 |

Compound 1, Compound 2 and Compound 3 can be synthesized by the methods disclosed in Example 26, Example 6 and Example 71 of WO 96/02542.

### Compound 1:

### 1,2-dihydro-3-methyl-1-[5-(trifluoromethanesulfonamide)pentan-1-4,7b-triazacyclopent[cd]inden-2-one hydrochloride,

### Compound 2:

### 4,5-dihydro-4-[4-(trifluoromethanesulfonamido)butan-1-yl]-3H-1,4,8b-triazaacenaphthylen-3-one hydrochloride,

### Compound 3:

### 1,2-dihydro-3-methyl-1-[2-(1-trifluoromethanesulfonylpiperidin-4-yl)ethan-1-yl]-1,4,7b-triazacyclopent[cd]inden-2-one hydrochloride

### Preparation Example 1 (as one coated tablet)

| | |
|---|---|
| (1) Compound of Example 9(ii) | 10.0 mg |
| (2) Lactose | 60.0 mg |
| (3) Corn starch | 35.0 mg |
| (4) Gelatin | 3.0 mg |
| (5) Magnesium stearate | 2.0 mg |

A mixture of 10.0 mg of the compound of Example 9(ii), 60.0 mg of lactose and 35.0 mg of corn starch was granulated through a 1 mm mesh screen, using 0.03 ml of an aqueous solution of 10 weight % gelatin (3.0 mg in terms of gelatin), which was dried at 40°C and screened again. The granules thus obtained were mixed with 2.0 mg of magnesium stearate and compressed. The core tablet thus obtained was coated with a suspension of sucrose, titanium dioxide and talc in gum arabic, which was polished with bees wax.

### Preparation Example 2 (as one tablet)

| | |
|---|---|
| (1) compound of Example 9(ii) | 10.0 mg |
| (2) Lactose | 70.0 mg |
| (3) Corn starch | 50.0 mg |
| (4) Soluble starch | 7.0 mg |
| (5) Magnesium stearate | 3.0 mg |

A mixture of 10.0 mg of the compound of Example 9(ii) and 3.0 mg of magnesium stearate was granulated with 0.07 ml of an aqueous solution of soluble starch (7.0 mg in terms of soluble starch), which was dried and, then mixed with 70.0 mg of lactose and 50.0 mg of corn starch. The mixture was compressed to obtain a tablet.

### Preparation Example 3

| | |
|---|---|
| (1) Compound of Example 9(ii) | 5.0 mg |
| (2) Common salt | 20.0 mg |
| (3) Distilled water to make the whole volume | 2 ml |

In distilled water were dissolved 5.0 mg of the compound of Example 1 and 20.0 mg of common salt. To the solution was added distilled water to make the whole volume 2.0 ml. The solution was subjected to filtration, which was filled into a 20 ml-ampoule under sterile conditions. The ampoule was sterilized and sealed to provide an injectable solution.

### Preparation Example 4 (as one coated tablet)

| | |
|---|---|
| (1) Compound 3 | 10.0 mg |
| (2) Lactose | 60.0 mg |
| (3) Corn starch | 35 mg |
| (4) Gelatin | 3.0 mg |
| (5) Magnesium stearate | 2.0 mg |

A mixture of 10.0 mg of Compound 3, 60.0 mg of lactose and 35.0 mg of corn starch was granulated through a 1 mm mesh screen, using 0.03 ml of an aqueous solution of 10 weight % gelatin (3.0 mg in terms of gelatin), which was dried at 40°C and screened again. The granules thus obtained were mixed with 2.0 mg of magnesium stearate and compressed. The core tablet thus obtained was coated with a suspension of sucrose, titanium dioxide, talc in gum arabic, which was polished with bees wax.

### Preparation Example 5 (as on tablet)

| | |
|---|---|
| (1) Compound 3 | 10.0 mg |
| (2) Lactose | 70.0 mg |
| (3) Corn starch | 50.0 mg |
| (4) Soluble starch | 7.0 mg |
| (5) Magnesium stearate | 3.0 mg |

A mixture of 10.0 mg of Compound 3 and 3.0 mg of magnesium stearate was granulated with 0.07 ml of an aqueous solution of soluble starch (7.0 mg in terms of soluble starch), which was dried and, then mixed with 70.0 mg of lactose and 50.0 mg of corn starch. The mixture was compressed to obtain a tablet.

This application is based on applications Nos. 231855/1996 and 292059/1996 filed in Japan, the content of which is incorporated hereinto by reference.

## Claims

1. A pharmaceutical composition for treating or preventing proliferative glomerulonephritis, which comprises a compound of the formula: 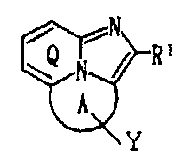
wherein ring A is a nitrogen-containing heterocyclic ring, having two nitrogen atoms as the hetero-atoms, which may be substituted with oxo or thioxo;
ring Q may be substituted;
Y is an optionally substituted hydrocarbon group, an optionally substituted hydroxy group or an optionally substituted mercapto group; and
R¹ is a hydrogen atom, a halogen atom, an optionally substituted hydrocarbon group or an acyl group, or a salt thereof, in admixture with a pharmaceutically acceptable carrier, excipient or diluent.

2. A pharmaceutical composition of claim 1, wherein the proliferative glomerulonephritis is mesangium proliferative glomerulonephritis.

3. Use of a compound of the formula: 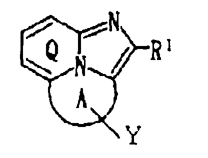
wherein ring A is a nitrogen-containing heterocyclic ring, having two nitrogen atoms as the hetero-atoms, which may be substituted with oxo or thioxo;
ring Q may be substituted;
Y is an optionally substituted hydrocarbon group, an optionally substituted hydroxyl group or an optionally substituted mercapto group; and
R¹ is a hydrogen atom, a halogen atom, an optionally substituted hydrocarbon group or an acyl group, or a salt thereof, for manufacturing a pharmaceutical composition for treating or preventing proliferative glomerulonephritis.

4. Use of claim 3, wherein the proliferative glomerulonephritis is mesangium proliferative glomerulonephritis.

5. A compound of the formula: 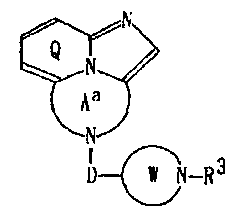
wherein ring A^{a} is a nitrogen-containing heterocyclic ring which may have one oxo group;
D is a bond or an optionally substituted divalent hydrocarbon group;
ring W is an optionally substituted nitrogen-containing heterocyclic ring;
ring Q may be substituted; and
R³ is an electron-withdrawing group, or a salt thereof.

6. A compound of claim 5, wherein the ring A^{a} is a 5- or 6-membered nitrogen-containing heterocyclic ring which may have one oxo group.

7. A compound of claim 5, wherein 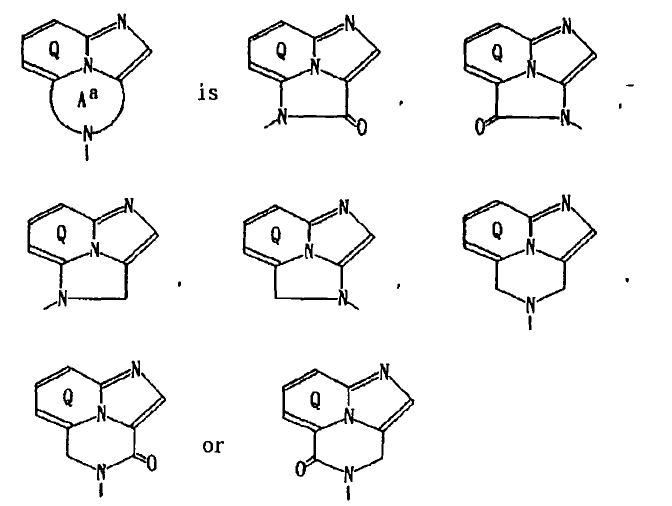 wherein ring Q is as defined in claim 5.

8. A compound of claim 5, wherein D is an optionally substituted divalent hydrocarbon group.

9. A compound of claim 5, wherein the ring W is an optionally substituted 5- or 6-membered nitrogen-containing heterocyclic ring.

10. A compound of claim 5, wherein R³ is -SO₂R⁴ (R⁴ is an optionally substituted hydrocarbon group), -COR⁵ (R⁵ is a hydrogen atom or an optionally substituted hydrocarbon group), -COOR⁶ (R⁶ is an optionally substituted hydrocarbon group) or -CON(R⁷)R⁸ (R⁷ and R⁸ respectively are a hydrogen atom or an optionally substituted hydrocarbon group, or R⁷ and R⁸ form a ring together with the adjacent nitrogen atom).

11. A compound of claim 5, wherein the ring Q is unsubstituted.

12. A compound of claim 5, wherein the ring W is

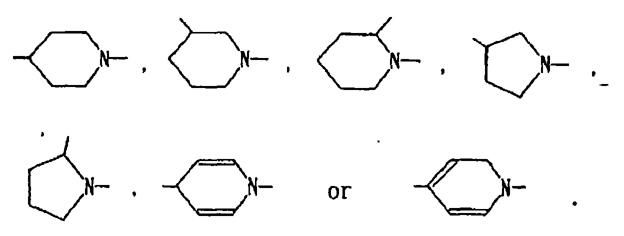

13. A compound of claim 5, wherein D is a C₁₋₆ alkylene group.

14. A compound of claim 5, which is a compound of the formula: 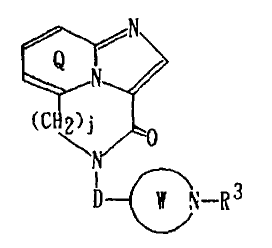 wherein j is 0 or 1, and the other symbols are as defined in claim 5, or a salt thereof.

15. A compound of claim 14, wherein R³ is -SO₂R⁴ (R⁴ is an optionally substituted hydrocarbon group), -COR⁵ (R⁵ is a hydrogen atom or an optionally substituted hydrocarbon group) or -COOR⁶ (R⁶ is an optionally substituted hydrocarbon group).

16. A compound of claim 15, wherein R⁴, R⁵ and R⁶ respectively are an optionally halogenated hydrocarbon group.

17. A compound of claim 14, wherein D is a C₁₋₆ alkylene group.

18. A compound of claim 14, wherein D is an ethylene group.

19. A compound of claim 14, wherein the ring W is

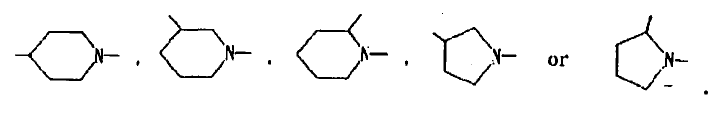

20. A compound of claim 14, wherein R³ is -SO₂R⁴ (R⁴ is an optionally substituted hydrocarbon group).

21. A compound of claim 20, wherein R⁴ is an optionally halogenated C₁₋₆ alkyl group.

22. A compound of claim 14, wherein the ring W is

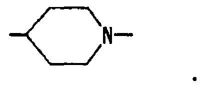

23. A compound of claim 14, wherein the groups of

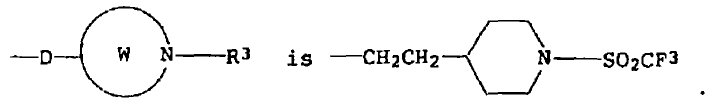

24. A compound of claim 14, wherein the ring Q is unsubstituted.

25. A compound of claim 14, wherein j is 0.

26. A compound of claim 5, which is 1,2-dihydro-1-(1-trifluoromethanesulfonylpiperidin-4-ylmethyl)-1,4,7b-triazacyclopent[cd]inden-2-one or a salt thereof.

27. A compound of claim 5, which is 1,2-dihydro-1-[2-(1-trifluoromethanesulfonylpiperidin-4-yl)ethan-1-yl]-1,4,7b-triazacyclopent[cd]inden-2-one or a salt thereof.

28. A compound of claim 5, which is 1,2-dihydro-1-[3-(trifluoromethanesulfonylpiperidin-4-yl)propan-1-yl]-1,4,7b-triazacyclopent[cd]inden-2-one or a salt thereof.

29. A compound of claim 5, which is 4,5-dihydro-4-(1-trifluoromethanesulfonylpiperidin-4-ylmethyl)-3H-1,4,8b-triazaacenaphthylene-3-one or a salt thereof.

30. A compound of claim 5, which is 4,5-dihydro-4-[2-(1-trifluoromethanesulfonylpiperidin-4-ylmethyl)-3H-1,4,8b-triazaacenaphthylene-3-one or a salt thereof.

31. A process for producing a compound of claim 5, which comprises reacting a compound of the formula: 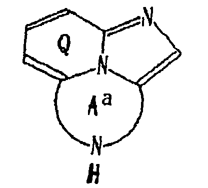 wherein the symbols are as defined in claim 5, or a salt thereof with a compound of the formula: 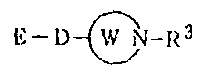 wherein E is a leaving group and the other symbols are as defined in claim 5, or a salt thereof.

32. A pharmaceutical composition which comprises a compound of claim 5, in admixture with a pharmaceutically acceptable carrier, excipient or diluent.

33. A pharmaceutical composition for inhibiting platelet-derived growth factor, which comprises a compound of claim 5, in admixture with a pharmaceutically acceptable carrier, excipient or diluent.

34. A pharmaceutical composition for treating or preventing renal diseases, which comprises a compound of claim 5, in admixture with a pharmaceutically acceptable carrier, excipient or diluent.

35. A pharmaceutical composition for treating or preventing arteriosclerosis, which comprises a compound of claim 5, in admixture with a pharmaceutically acceptable carrier, excipient or diluent.

36. A pharmaceutical composition for treating or preventing reobstruction after percutaneous transluminal coronary angioplasty, which comprises a compound of claim 5, in admixture with a pharmaceutically acceptable carrier, excipient or diluent.

37. A pharmaceutical composition for lowering lipid level, which comprises a compound of claim 5, in admixture with a pharmaceutically acceptable carrier, excipient or diluent.

38. A pharmaceutical composition for treating or preventing diabetic nephropathy, which comprises a compound of claim 5, in admixture with a pharmaceutically acceptable carrier, excipient or diluent.

39. A pharmaceutical composition for treating or preventing chronic glomerulonephritis, which comprises a compound of claim 5, in admixture with a pharmaceutically acceptable carrier, excipient or diluent.

40. Use of a compound of claim 5 for manufacturing a pharmaceutical composition.

41. Use of a compound of claim 5 for manufacturing a pharmaceutical composition for inhibiting platelet-derived growth factor.

42. Use of a compound of claim 5 for manufacturing a pharmaceutical composition for treating or preventing renal diseases.

43. Use of a compound of claim 5 for manufacturing a pharmaceutical composition for treating or preventing arteriosclerosis.

44. Use of a compound of claim 5 for manufacturing a pharmaceutical composition for treating or preventing reobstruction after percutaneous transluminal coronary angioplasty.

45. Use of a compound of claim 5 for manufacturing a pharmaceutical composition for lowering lipid level.

46. Use of a compound of claim 5 for manufacturing a pharmaceutical composition for treating or preventing diabetic nephropathy.

47. Use of a compound of claim 5 for manufacturing a pharmaceutical composition for treating or preventing chronic glomerulonephritis.
